(19) **Europäisches Patentamt**
**European Patent Office**
**Office européen des brevets**

(11) **EP 2 635 679 B1**

(12) **EUROPEAN PATENT SPECIFICATION**

(45) Date of publication and mention
of the grant of the patent:
**19.04.2017 Bulletin 2017/16**

(51) Int Cl.:
***C12N 15/10*** (2006.01)    ***C12N 15/90*** (2006.01)
***C40B 40/08*** (2006.01)    ***C40B 50/06*** (2006.01)

(21) Application number: **11802179.9**

(22) Date of filing: **07.11.2011**

(86) International application number:
**PCT/US2011/059642**

(87) International publication number:
**WO 2012/061832 (10.05.2012 Gazette 2012/19)**

(54) **LINKING SEQUENCE READS USING PAIRED CODE TAGS**

VERKNÜPFUNG VON SEQUENZSTÜCKEN MIT GEPAARTEN CODE-TAGS

LIAISON ENTRE DES LECTURES DE SÉQUENCES À L'AIDE DE CODES MARQUEURS APPARIÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.04.2011 US 201113080345
10.02.2011 US 201113025022
05.11.2010 US 410671 P**

(43) Date of publication of application:
**11.09.2013 Bulletin 2013/37**

(73) Proprietor: **Illumina, Inc.
San Diego, CA 92122 (US)**

(72) Inventors:
• **STEEMERS, Frank J.
San Diego,
California 92122 (US)**
• **GUNDERSON, Kevin
San Diego,
California 92122 (US)**
• **ROYCE, Thomas
San Diego,
California 92122 (US)**
• **PIGNATELLI, Natasha
San Diego,
California 92122 (US)**
• **GORYSHIN, Igor Yu
Madison,
Wisconsin 53713 (US)**
• **CARUCCIO, Nicholas
Madison,
Wisconsin 53713 (US)**

• **MAFFITT, Mark
Madison,
Wisconsin 53713 (US)**
• **JENDRISAK, Jerome
Madison,
Wisconsin 53713 (US)**
• **AMINI, Sasan
San Diego,
California 92122 (US)**
• **KAPER, Fiona
San Diego,
California 92122 (US)**
• **TURK, Casey
San Diego,
California 92122 (US)**
• **KALHOR, Reza
San Diego,
California 92122 (US)**

(74) Representative: **Cooley (UK) LLP
Dashwood
69 Old Broad Street
London EC2M 1QS (GB)**

(56) References cited:
**WO-A1-2010/048605      WO-A2-2005/100585
US-B1- 7 138 267**

• **SYED FRAZ ET AL: "Optimized library
preparation method for next-generation
sequencing", NATURE METHODS, vol. 6, no. 10,
October 2009 (2009-10), pages I-II, XP002672202,
ISSN: 1548-7091**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- BIMBER BENJAMIN N ET AL: "Whole-genome characterization of human and simian immunodeficiency virus intrahost diversity by ultradeep pyrosequencing.", JOURNAL OF VIROLOGY NOV 2010 LNKD- PUBMED:20844037, vol. 84, no. 22, 15 September 2010 (2010-09-15), pages 12087-12092, XP002672203, ISSN: 1098-5514
- WANG HAOYI ET AL: "Calling Cards enable multiplexed identification of the genomic targets of DNA-binding proteins.", GENOME RESEARCH MAY 2011 LNKD- PUBMED:21471402, vol. 21, no. 5, May 2011 (2011-05), pages 748-755, XP002672204, ISSN: 1549-5469
- MARINE RACHEL ET AL: "Evaluation of a transposase protocol for rapid generation of shotgun high-throughput sequencing libraries from nanogram quantities of DNA.", APPLIED AND ENVIRONMENTAL MICROBIOLOGY NOV 2011 LNKD- PUBMED:21948828, vol. 77, no. 22, 23 September 2011 (2011-09-23), pages 8071-8079, XP002672205, ISSN: 1098-5336
- POBIGAYLO N ET AL: "Construction of a large signature-tagged mini-Tn5 transposon library and its application to mutagenesis of Sinorhizobium meliloti", APPLIED AND ENVIRONMENTAL MICROBIOLOGY, AMERICAN SOCIETY FOR MICROBIOLOGY, US, vol. 72, no. 6, 1 June 2006 (2006-06-01), pages 4329-4337, XP002743587, ISSN: 0099-2240, DOI: 10.1128/AEM.03072-05
- ZHOU M ET AL: "Molecular genetic analysis of transposase-end DNA sequence recognition: cooperativity of three adjacent base-pairs in specific interaction with a mutant Tn5 transposase", JOURNAL OF MOLECULAR BIOLOGY, ACADEMIC PRESS, UNITED KINGDOM, vol. 276, no. 5, 13 March 1998 (1998-03-13), pages 913-925, XP004454012, ISSN: 0022-2836, DOI: 10.1006/JMBI.1997.1579

**Description**

FIELD OF THE INVENTION

Embodiments of the present invention relate to the fields of biology and genomics. Some embodiments of the present invention relate to methods and compositions that include certain transposon sequences. Some such methods and compositions include analyzing target nucleic acids.

BACKGROUND OF THE INVENTION

The detection of specific nucleic acid sequences present in a biological sample has been used, for example, as a method for identifying and classifying microorganisms, diagnosing infectious diseases, detecting and characterizing genetic abnormalities, identifying genetic changes associated with cancer, studying genetic susceptibility to disease, and measuring response to various types of treatment. A common technique for detecting specific nucleic acid sequences in a biological sample is nucleic acid sequencing.

Nucleic acid sequencing methodology has evolved significantly from the chemical degradation methods used by Maxam and Gilbert and the strand elongation methods used by Sanger. Today several sequencing methodologies are in use which allow for the parallel processing of nucleic acids all in a single sequencing run. As such, the information generated from a single sequencing run can be enormous. Library preparation methods for such "next-generation sequencing" has been advanced by using a transposon-based method for preparing fragmented and tagged DNA libraries (International Publication No. WO2010/048605; Syed F et al., 2009, Nat Methods, 6(10), I-II; Bimber B. et al., 2010, J Virol, 84(22), 12087-12092; Pobigaylo N. et al., 2006, Appl Environ Microb, 72(6), 4329-4337; Zhou M. et al., 1998, J Mol Biol, 276(8), 913-925).

SUMMARY OF THE INVENTION

In a first aspect, there is provided an artificial transposon comprising: a first transposase recognition site, a second transposase recognition site, a barcode disposed therebetween, wherein the barcode comprises a double-stranded nucleic acid sequence comprising a first strand barcode and a second strand barcode, and a linker disposed between the first and the second barcode sequences, wherein the linker comprises a first primer site, a second primer site and a non-amplifiable site disposed between the first and second primer sites.

Some embodiments of the present invention relate to methods and compositions that include such transposon sequences. Some such methods and compositions include analyzing target nucleic acids. Some embodiments include methods of preparing a template nucleic acid. Some such methods include: (a) providing a target nucleic acid; (b) providing a plurality of transposon sequences of the first aspect; and (c) contacting the target nucleic acid with the plurality of transposon sequences under conditions such that at least a portion of said plurality of sequences inserts into the target nucleic acid ("tagementation"), thereby preparing a template nucleic acid.

In some embodiments, the barcode of each transposon is different.

The barcode comprises a first barcode sequence, a second barcode sequence, said first and second barcode sequences being separated by a linker disposed therebetween.

The barcode comprises a double-stranded nucleic acid sequence comprising a first strand barcode and a second strand barcode. In some embodiments, the first strand barcode and second strand barcode comprise complementary sequences. In some embodiments, the first strand barcode and second strand barcode comprise non-complementary sequences.

In some embodiments, the linker comprises a nucleic acid.

The linker comprises a sequencing adapter comprising a first primer site.

In some embodiments, the linker comprises a fragmentation site. In some embodiments, the fragmentation site comprises a first nickase recognition sequence and a second nickase recognition sequence, wherein the cut site for each recognition sequence is the same site. In some embodiments, the fragmentation site comprises a restriction endonuclease recognition sequence.

In some embodiments, the linker comprises a sequencing adapter comprising a first primer site and a fragmentation site. In some embodiments, the linker comprises a sequencing adapter comprising a first primer site and a second primer site having a fragmentation site disposed therebetween.

Some embodiments include methods of preparing a library of template nucleic acids. Some such methods include: (a) providing a target nucleic acid; (b) providing a plurality of transposon sequences of the first aspect; and (c) contacting the target nucleic acid with the plurality of transposon sequences under conditions such that at least a portion of said plurality of transposon sequences inserts into the target nucleic acid; and (d) amplifying at least a portion of the target nucleic acid by hybridizing a primer to said first primer site and a primer to said second primer site, such that the amplification product comprises a first barcode sequence and a second barcode sequence, thereby preparing a library of template nucleic acids.

In some embodiments, the barcode of each transposon is different.

Some methods also include a step subsequent to (c) and prior to (d) comprising reducing the number of target nucleic acid molecules comprising inserted transposon sequences.

The sequencing adapter comprises a first primer site, a second primer site, and a non-amplifiable site therebetween. In some embodiments, the non-amplifiable site comprises a nucleic acid. In some embodiments, the non-amplifiable site comprises at least one nucleotide analogue. In some embodiments, the nucleotide analogue does not significantly base-pair with A, C, G or T.

Some embodiments include methods of preparing a library of template nucleic acids. Some such methods include: (a) providing a target nucleic acid; (b) providing a plurality of transposon sequences of the first aspect , wherein the linker comprises a fragmentation site; (c) contacting the target nucleic acid with the plurality of transposon sequences under conditions such that at least a portion of said plurality of transposon sequences inserts into the target nucleic acid; and (d) fragmenting said target nucleic acid at said fragmentation sites, such that at least a portion of the fragmented nucleic acids each comprise a first barcode sequence and a second barcode sequence, thereby preparing a library of template nucleic acids.

In some embodiments, the barcode of each transposon is different.

In some embodiments, the linker comprises a nucleic acid.

In some embodiments, the fragmentation site comprises a first nickase recognition sequence, a second nickase recognition sequence, wherein the cut site for each recognition sequence is the same site.

In some embodiments, the fragmentation site comprises a restriction endonuclease recognition sequence.

In some methods, the fragmenting comprises contacting the target nucleic acid with a polymerase. Useful polymerases include those with exonuclease activity (such as 3' to 5' activity, *e.g., E. coli* DNA polymerase III, or 5' to 3' activity, *e.g., E. coli* DNA polymerase I), non-strand-displacing (e.g. T4 DNA polymerase) and strand-displacing activities (e.g. *Bst* DNA polymerase, large fragment).

Some methods also include ligating a first primer site to a first end of at least one fragmented nucleic acid. Some methods also include ligating a second primer site to the second end of the at least one fragmented nucleic acid.

Some methods also include amplifying said at least one fragmented nucleic acid by hybridizing a primer to the first primer site and a primer to the second primer site.

In some embodiments, the linker comprises a sequencing adapter comprising a first primer site, a second primer site having the fragmentation site disposed therebetween. Some methods also include amplifying said at least a portion of the fragmented nucleic acids by hybridizing a primer to said first primer site and a primer to said second primer site.

In some embodiments, the target nucleic acid comprises genomic DNA.

In some embodiments, the first transposase recognition site comprises a mosaic element.

In some embodiments, the sequences of the first barcode and the second barcode comprise the reverse complements of each other.

Some embodiments include libraries of template nucleic acids prepared by any one of the foregoing methods.

Some embodiments include methods of preparing a transposon of the first aspect. Some such methods include: (a) providing a transposon template nucleic acid sequence comprising a first transposase recognition site, a first barcode 3' thereof, and a linker 3' thereof; and (b) extending the transposon template sequence by hair-pin extension, such that the extended transposon template sequence further comprises sequences complementary to said barcode and to said transposase recognition site, thereby preparing a transposon comprising a first transposase recognition site, a first barcode, a linker, a second barcode, and a second transposase recognition site.

In some embodiments, the barcode comprises a random sequence.

In some embodiments, the transposase recognition site comprises a mosaic element.

In some embodiments, the barcode comprises at least about five nucleotides.

In some embodiments, the linker comprises a nucleic acid.

In some embodiments, the linker comprises a fragmentation site. In some embodiments, the fragmentation site comprises a first nickase recognition sequence, a second nickase recognition sequence, wherein the cut site for each recognition sequence is the same site. In some embodiments, the fragmentation site comprises a restriction endonuclease recognition sequence.

The linker comprises a sequencing adapter.

The sequencing adapter comprises a first primer site and second primer site. In some embodiments, the sequencing adapter comprises a first primer site and a second primer site having a fragmentation site disposed therebetween.

The sequencing adapter comprises a first primer site and a second primer site, having a non-amplifiable site disposed therebetween.

In some embodiments, the non-amplifiable site comprises a nucleic acid. In some embodiments, the non-amplifiable site comprises at least one nucleotide analogue. In some embodiments, the nucleotide analogue does not significantly base-pair with A, C, G or T.

Some embodiments include methods of preparing a plurality of transposon sequences. Some such method include repeating steps (a) and (b) of any one of the foregoing methods of preparing a transposon sequence, wherein the barcode of each transposon template nucleic acid is different.

Some embodiments include transposons prepared by the method of any one of the foregoing methods of preparing a transposon sequence.

Some embodiments include methods of analyzing a target nucleic acid. Some such methods include:

(a) providing a template nucleic acid, wherein the template nucleic acid comprises the target nucleic acid and a plurality of transposons of the first aspect inserted therein;

(b) obtaining sequence data from said template nucleic acid; and (c) assembling a representation of at least a portion of said target nucleic acid from said sequence data.

In some embodiments, the barcode of each marker is different.

The barcode comprises a double-stranded nucleic acid sequence comprising a first strand barcode and a second strand barcode. In some embodiments, the first strand barcode and second strand barcode comprise complementary sequences. In some embodiments, the first strand barcode and second strand barcode comprise non-complementary sequences.

In some embodiments, obtaining sequence data comprises hybridizing a primer to said first primer site, and extending said primer.

In some embodiments, obtaining sequence data comprises hybridizing a primer to the second primer site and extending said primer. In some embodiments, the primers hybridize to the sites in opposite orientations.

In some embodiments, the sequences of the first barcode sequence and the second barcode sequence comprise the reverse complements of each other.

In some embodiments, the assembling step comprises identifying more than one sequencing read comprising the first barcode sequence or second barcode sequence of a barcode.

In some embodiments, the presence of a first barcode sequence or second barcode sequence of a barcode in more than one sequencing read is indicative of the more than one sequencing reads representing sequences adjacent to each other in the target nucleic acid.

In some embodiments, each marker sequence comprises a first host tag and second host tag having the barcode disposed therebetween. In some embodiments, the first and second host tag of a marker comprises the same sequence. In some embodiments, the assembling step further comprises identifying more than one sequencing read comprising the same host tags.

In some embodiments, the target nucleic acid comprises genomic DNA.

The artificial transposon sequences of the first aspect include:

transposon sequences include: a first transposase recognition site and a second transposase recognition site having a barcode disposed therebetween, wherein the barcode comprises a first barcode sequence and a second barcode sequence, said first and second barcode sequences being separated by a linker.

The barcode comprises a double-stranded nucleic acid sequence comprising a first strand barcode and a second strand barcode. In some embodiments, the first strand barcode and second strand barcode comprise complementary sequences. In some embodiments, the first strand barcode and second strand barcode comprise non-complementary sequences.

In some embodiments, the first transposon recognition site comprises a mosaic element.

In some embodiments, the linker comprises a nucleic acid.

In some embodiments, the linker comprises a fragmentation site. In some embodiments, the fragmentation site comprises a first nickase recognition sequence and a second nickase recognition sequence, wherein the cut site for each recognition sequence is the same site. In some embodiments, the fragmentation site comprises a restriction endonuclease recognition sequence.

The linker comprises a sequencing adapter comprising a first primer site and a second primer site, having a non-amplifiable site disposed therebetween. In some embodiments, the non-amplifiable site comprises a nucleic acid. In some embodiments, the non-amplifiable site comprises at least one nucleotide analogue. In some embodiments, the nucleotide analogue does not significantly base-pair with A, C, G or T.

In some embodiments, the linker comprises a sequencing adapter comprising a first primer site and a second primer site, having a fragmentation site disposed therebetween.

In some embodiments, a sequencing primer is hybridized to said first primer site.

In some embodiments, a sequencing primer is hybridized to said first transposase recognition site.

Some embodiments include populations of artificial transposon sequences comprising a plurality of any one of the foregoing artificial transposon sequences.

Some embodiments include target nucleic acids or copies thereof having a population of the artificial transposon sequences integrated therein, wherein the artificial transposon sequences comprise any one of the artificial transposon sequences described herein.

Some embodiments include populations of genomic DNA fragments or copies thereof having a population of the artificial transposon sequences integrated therein, wherein the artificial transposon sequences comprise any one of the artificial transposon sequences described herein.

Some embodiments include genomes having a population of artificial transposon sequences integrated therein, wherein the artificial transposon sequences comprise any one of the artificial transposon sequences described herein.

Such isolated template nucleic acids include at least a portion of a target nucleic acid or copy thereof and at least two of the artificial transposon sequences inserted therein.

In these embodiments, the barcode of each marker is different.

The barcode comprises a double-stranded nucleic acid sequence comprising a first strand barcode and a second strand barcode. In some embodiments, the first strand barcode and second strand barcode comprise complementary sequences. In some embodiments, the first strand barcode and second strand barcode comprise non-complementary sequences.

Some embodiments also include a host tag. Some embodiments also include a first host tag and a second host tag, having the at least a portion of a target nucleic acid or copy thereof disposed therebetween. Some embodiments also include a first host tag and a second host tag, having a transposase recognition site disposed therebetween, wherein the transposase recognition site comprises a first transposase recognition site and a second transposase recognition site, having the at least a portion of a target nucleic acid or copy thereof disposed therebetween.

In some embodiments, the host tag comprises nine nucleotides.

In some embodiments, the target nucleic acid comprises genomic DNA.

Some embodiments include a plurality of template nucleic acids comprising the template nucleic acid of any one of the isolated template nucleic acids described herein, wherein a first template nucleic acid of said plurality comprises a first barcode, and a second template nucleic acid comprises a second barcode, wherein the first and second barcodes are indicative of template nucleic acid sequences being adjacent to one another in a sequence representation of the target nucleic acid. In some embodiments, the first barcode comprises the reverse complement sequence of the second barcode.

Some embodiments include the use of substrates comprising nucleotide sequences attached thereto, at least one of said nucleotide sequences comprising any one of the template nucleic acids described herein, or any one of the plurality of template nucleic acids described herein. In some embodiments, the substrate can include a solid support selected from the group consisting of spheres, microparticles, beads, membranes, slides, plates, micromachined chips, tubes, microwells, microfluidic devices, channels, and filters.

BRIEF DESCRIPTION OF THE DRAWINGS

**FIG.1** depicts a schematic of a transposon sequence. M indicates a first transposon recognition sequence. C1 indicates a first barcode sequence. The linker sequence is indicated by a rectangle with a double border. C1' indicates a second barcode sequence. M' indicates a second transposase recognition sequence. Exemplary linker sequences are also shown. For example, linker sequences can have fragmentation sites, such as those with nickase recognition sites (e.g. N1, N2) or a restriction endonuclease recognition sequence (indicated by RE). Linkers can contain one or more

primer sites (e.g. A, B), corresponding to complementary primers A' and B'. A non-amplifiable site is indicated by nA.

**FIG. 2** depicts a double-stranded transposon sequence comprising transposon recognition sites (TRS and TRS'), a fragmentation site, and a barcode comprising four barcode sequences (CODE 1, CODE 2, CODE 3, and CODE 4). In the example shown, CODE 1 and CODE 3, and CODE 2 and CODE 4 are non-complementary sequences.

**FIG. 3** illustrates single-stranded transposons having bicodes or unicodes.

**FIG. 4** illustrates preparation of transposome having different transposase recognition sequences.

**FIG. 5** depicts a transposome complex that has one transposon sequence and two transposases.

**FIG. 6** illustrates gap fill-in and ligation reactions.

**FIG. 7** depicts "looped" complexes.

**FIG. 8** depicts a complex with one transposon and two transposase subunits ensures that a known combination of codes is inserted into target DNA, without fragmenting the target DNA. This configuration allows linking DNA fragments after fragmentation using the code N-Code N' combination.

**FIG. 9** illustrates strand-displacement synthesis to copy identical flanking genomic DNA sequences (g-codes).

**FIG. 10** illustrates nicking followed by 3'-exo digestion to create gaps in genomic DNA.

**FIG. 11** illustrates transposons with engineered bicodes and random bi-codes.

**FIG. 12.** Transposon-transposase complex. Complexes are formed from two half-transposons, each containing one ME region and a random or specific code region. Upon insertion, the transposase enzyme binds to both transposons preventing DNA fragmentation. The ends of the transposons have "sticky ends" allowing ligation of the two ends. A specific endonuclease fragmentation system nicks each strand. Denaturation creates DNA fragments in which each DNA fragment contains a linked code with the neighboring fragment.

**FIG. 13.** Transposons with amplifiable and non-amplifiable linkers between the ME sequences.

**FIG. 14** depicts use of bi-code junction-tagging.

**FIG. 15** depicts a library preparation method that involves ligation of a forked adapter sequence. Steps include: library preparation; extension and A-tailing; adapter ligation; and PCR amplify, cluster, and sequence products.

**FIG. 16** depicts an exemplary scheme to prepare a transposon sequence with reverse complements

**FIG. 17** depicts a method of preparing a transposon sequence which includes the use of tailed-oligonucleotides comprising mosaic elements (ME). The transposon sequence includes primer sites P3 and P4, a barcode comprising Code X and Code Y, and a fragmentation site.

**FIG. 18** depicts the integration of an exemplary Tn5 transposon comprising transposase recognition sequences (indicated by mosaic elements ME) into a target nucleic acid. The insertion results in duplication of the integration site, shown as single-stranded A'B'C'D'E'F'G'H'I' and single-stranded ABCDEFGHI. The figure also shows an optional repair step to fill in the single-stranded region. In some embodiments, the filled-in region can be used as a host tag.

**FIG. 19** shows a tagmentation method using transposomes that have symmetrical transposable end sequences.

**FIG. 20** depicts an exemplary embodiment of a contacting a target nucleic acid with a library of transposons, which integrates into the target nucleic acid.

**FIG. 21** depicts an optional step of fragmenting a linker into two parts, or an optional step of virtually fragmenting the linker by amplification using a primer A' and/or a primer B'. Fragmentation of the linker generates a library of template nucleic acids. In this figure, the filled-in region is indicated by a thick line.

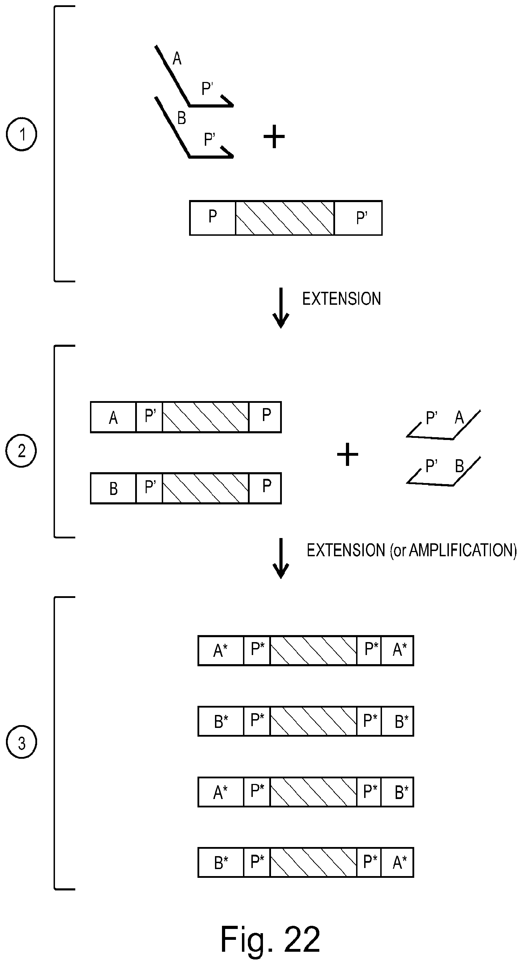

**FIG. 22** depicts an examplary embodiment using tailed-oligonucleotides to obtain a population of template nucleic acids comprising a first universal primer site (A) and a second universal primer site (B). In step 1, tailed-oligonucleotides comprising universal primer site sequences anneal to a template nucleic acid comprising primer site sequences (P and P') and are extended. In step 2, the tailed oligonucleotides of step 1 may anneal to the products of step 1 and be extended. Alternatively, step 2 can include an amplification step with the inclusion of oligonucleotides (*e.g.*, oligonucleotides comprising A' and B' sequences). The products of step 2 are depicted in step 3.

**FIG. 23** illustrates an embodiment for code-tagging individual molecules with unique tags, allowing normalization of the original molecule representation, even after amplification.

**FIG. 24** depicts how linked transposon ends result in fragmented DNA, simultaneously linking the top and bottom strand of the target DNA.

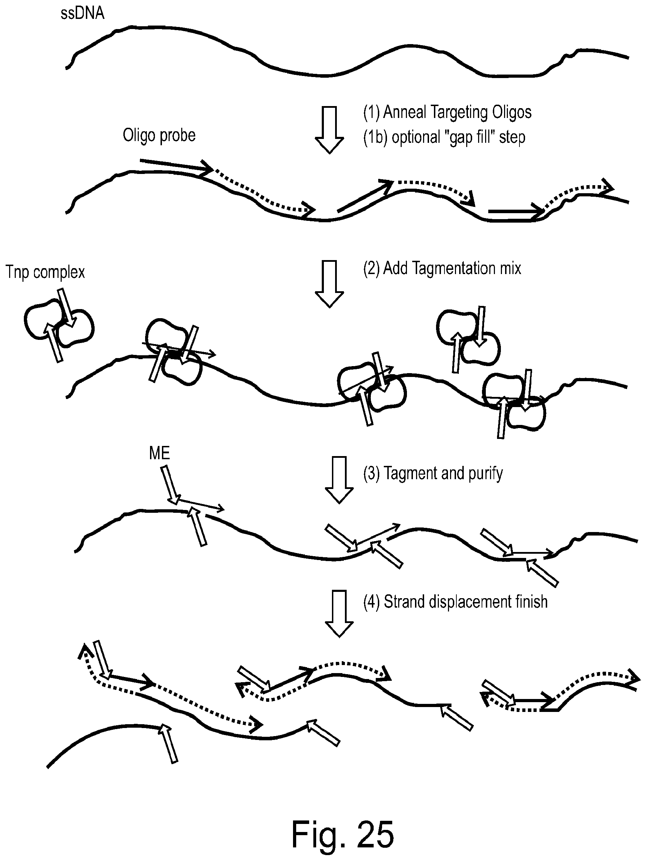

**FIG. 25.** Targeted Tagmentation using ssDNA. (1) Locus-specific oligo probes are annealed to regions of interest in the denatured DNA sample. An optional "gap-fill" extension (polymerase, ligase, nucleotides, and buffer) can be employed to create dsDNA between sites of probe annealing. (2) Tagmentation mix (i.e. Tn5-mosaic end transposon complexes and appropriate reaction buffer) is added to the annealed DNA. (3) Tagmentation only occurs at regions of dsDNA leading to "targeted" tagmentation (and nicking) at sites where the oligo probes have annealed. (4) Strand-displacement synthesis resolves the nicks into amplifiable library elements flanked by the universal ME primer. This ME primer can be used in a subsequent PCR step to attach P5-Seq1 and P7-Seq2 sequencing tails to the tagments.

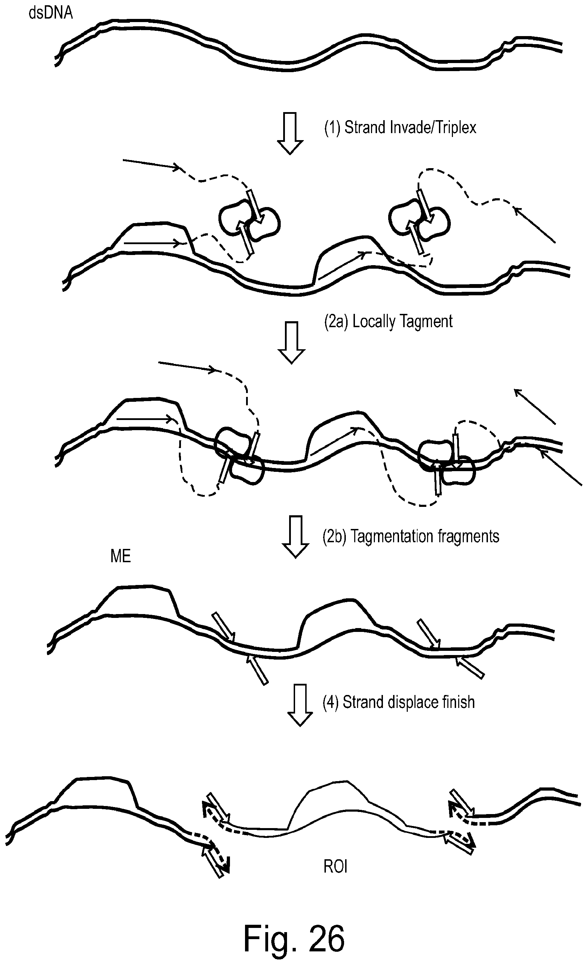

**FIG. 26.** Targeted Tagmentation using dsDNA and Strand Invasion/Triplex formation. D-loop formation via strand invasion using recA coated oligonucleotides or LNA probes can be used to create D-loops and target transposase complexes to loci or regions of interest (ROI) in dsDNA. Targeted transposition can also be accomplished by triplex formation using pyrimidine-rich oligonucleotides. The targeting probe or oligonucleotide is conjugated to the mosaic elements (ME) on the transposase-transposon complex. (1) Strand invasion opens a D-loop in the dsDNA bringing the transposase complex into the vicinity of the targeted region. Ideally two such probes will be used to generate libraries spanning the region between the probes. (2) Localization of the transposon complex leads to insertion in the vicinity of the D-loop or triplex structure. (3) Strand-displacement is use to finish the library element flanked by universal ME primers. PCR can be used append sequencing primers using the ME ends.

**FIG. 27** depicts tethering of a circularized intermediate to the surface of a particle.

**FIG. 28** depicts a schematic representation of using paired bar codes (indicated by Code 1, Code 2, Code 5) for code-pair sequencing of template contig sequences to assemble the sequence of an original target nucleic acid.

**FIG. 29** depicts an exemplary embodiment of a method including fragmentation.

**FIG. 30** depicts an exemplary embodiment of a method that includes optional steps of amplifying a target nucleic acid by PCR or whole genome amplification (WGA). In this figure, the filled-in region is indicated by $N_1$ and $N_2$.

**FIG. 31** depicts an exemplary embodiment of a method for assembling short sequencing reads using a linked read strategy. Optional steps of sub-sampling, code alignment, read assembly, and genome assembly are also illustrated.

**FIG. 32** illustrates the preparation of nested sequencing libraries.

**FIG. 33** illustrates a two-step tagging method using different transposomes.

**FIG. 34** illustrates single cell/DNA tagmentation in microwells.

**FIG. 35** illustrates single cell/DNA tagmentation in droplets.

**FIG. 36** depicts a graph showing that the proportion of template nucleic acids that are useful can decrease as the average distance between sites of integration increases.

**FIG. 37** depicts an embodiment using restriction endonucleases to generate randomer sticky ends.

DETAILED DESCRIPTION

Some embodiments of the present invention relate to methods and compositions that include certain transposon sequences. Some such methods and compositions include analyzing target nucleic acids. Generally, methods of analyzing nucleic acids include preparing a library of template nucleic acids of a target nucleic acid, obtaining sequence data from the library of template nucleic acids, and assembling a sequence representation of the target nucleic acid from such sequence data. However, assembly of a sequence representation using traditional methods is met with several challenges. For example, sequencing data is obtained using short reads and thus there is difficulty assembling contiguous reads through repetitive sequences of a target nucleic acid. In addition, many contigs are required to be constructed to represent a genome such as the human genome. Methods such as paired-end sequencing can be used to mitigate the difficulties of assembling sequence data from many short reads. However, the paired-end sequencing methods require longer template nucleic acids to span repetitive sequences in a target nucleic acid; preparation of template nucleic acids is inefficient and thus requires larger amounts of DNA; the number of different paired-ends is limited; and a reference genome is required to verify any resulting sequence representation.

Some nucleic acids of interest, such as genomic DNAs, comprise long molecules with ordered sequence. Methods to sequence such molecules tend to be highly parallelized and include sequencing short libraries in order to create high throughput. Consequently, such methods require assembly of the shorter reads to obtain ordering information, *i.e.*, a sequence representation of the target nucleic acid. Shotgun sequencing approaches uses DNA fragmentation; however, order information is lost during fragmentation. Advantageously, some methods and compositions provided herein can be used to obtain shorter reads of template nucleic acids in which the order information is preserved. Thus, assembly of shorter reads can be performed without the requirement of a reference genome.

In an exemplary embodiment, a library of template nucleic acids is prepared from a target nucleic acid. The library is prepared by inserting a plurality of unique barcodes throughout the target nucleic acid.

As will be understood, although a barcode is frequently depicted in the figures as a double-stranded, annealed structure, a useful barcode can be a double-stranded region where the two strands are not complementary or not annealed. For example, two noncomplementary regions can be linked informatically, even though they are not complementary in a conventional Watson-Crick base-pairing.

Each barcode includes a first barcode sequence and a second barcode sequence, having a fragmentation site disposed therebetween. The first barcode sequence and second barcode sequences can be identified or designated to be paired with one another. The pairing can be informatic so that a first barcode is associated with a second barcode. The pairing can also be physically associated to form a junction between the two barcodes. For example, the first barcode sequence and second barcode sequences can be the tandem or reverse complements of each other. The target nucleic acid can be fragmented at the fragmentation sites, and a library of template nucleic acids can be prepared from the fragments. Sequencing information can be obtained from the library of template nucleic acids. Advantageously, the paired barcode sequences can be used to assemble sequencing data from the library of template nucleic acids. For example, identifying a first template nucleic acid comprising a first barcode sequence and a second template nucleic acid comprising a second barcode sequence that is paired with the first indicates that the first and second template nucleic acids represent sequences adjacent to one another in a sequence representation of the target nucleic acid. In essence, two sequences originally adjacent in the target nucleic acid may be separated from each other, introducing one barcode of a barcode pair at their points of separation, so that even when sequenced separately, the detection of the paired barcodes indicates the original proximity of the two sequences. Despite having been separated physically, they can be reunited bioinformatically, much as each divided half sought its other half in Plato's *Symposium*. Thus, a sequence representation of the target nucleic acid can be assembled by identifying further sequencing reads comprising paired barcode sequences. Such methods can be used to assemble a sequence representation of a target nucleic acid *de novo*, without the requirement of a reference genome. Moreover, such methods are also useful to sequence target nucleic acids comprising highly repetitive sequences. The methods can also be used to reassemble alternative isoforms and splice junctions in cDNAs, as well as reassemble single-molecule haplotypes.

Because the methods herein involve addition of one or more tags (such as an identifier code) at the location that previously existed between two adjacent sequences, these methods can be referred to as "junction tagging". More generally, the addition of an identifier tag to an individual nucleic acid can be termed "code tagging".

**Definitions**

As used herein the term "nucleic acid" and/or "oligonucleotide" and/or grammatical equivalents thereof can refer to at least two nucleotide monomers linked together. A nucleic acid can generally contain phosphodiester bonds; however, in some embodiments, nucleic acid analogs may have other types of backbones, comprising, for example, phosphoramide (Beaucage, et al., Tetrahedron, 49:1925 (1993); Letsinger, J. Org. Chem., 35:3800 (1970); Sprinzl, et al., Eur. J. Biochem., 81:579 (1977); Letsinger, et al., Nucl. Acids Res., 14:3487 (1986); Sawai, et al., Chem. Lett., 805 (1984), Letsinger, et al., J. Am. Chem. Soc., 110:4470 (1988); and Pauwels, et al., Chemica Scripta, 26:141 (1986)), phosphorothioate (Mag, et al., Nucleic Acids Res., 19:1437 (1991); and U.S. Pat. No. 5,644,048), phosphorodithioate (Briu, et al., J. Am. Chem. Soc., 111:2321 (1989), O-methylphosphoroamidite linkages (see Eckstein, Oligonucleotides and Analogues: A Practical Approach, Oxford University Press), and peptide nucleic acid backbones and linkages (see Egholm, J. Am. Chem. Soc., 114:1895 (1992); Meier, et al., Chem. Int. Ed. Engl., 31:1008 (1992); Nielsen, Nature, 365:566 (1993); Carlsson, et al., Nature, 380:207 (1996)).

Other analog nucleic acids include those with positive backbones (Denpcy, et al., Proc. Natl. Acad. Sci. USA, 92:6097 (1995)); non-ionic backbones (U.S. Pat. Nos. 5,386,023; 5,637,684; 5,602,240; 5,216,141; and 4,469,863; Kiedrowshi, et al., Angew. Chem. Intl. Ed. English, 30:423 (1991); Letsinger, et al., J. Am. Chem. Soc., 110:4470 (1988); Letsinger, et al., Nucleosides & Nucleotides, 13:1597 (1994); Chapters 2 and 3, ASC Symposium Series 580, "Carbohydrate Modifications in Antisense Research", Ed. Y. S. Sanghui and P. Dan Cook; Mesmaeker, et al., Bioorganic & Medicinal Chem. Lett., 4:395 (1994); Jeffs, et al., J. Biomolecular NMR, 34:17 (1994); Tetrahedron Lett., 37:743 (1996)) and non-ribose (U.S. Patent No. 5,235,033 and No. 5,034,506, and Chapters 6 and 7, ASC Symposium Series 580, "Carbohydrate Modifications in Antisense Research", Ed. Y. S. Sanghui and P. Dan Coo). Nucleic acids may also contain one or more carbocyclic sugars (see Jenkins, et al., Chem. Soc. Rev., (1995) pp. 169 176).

Modifications of the ribose-phosphate backbone may be done to facilitate the addition of additional moieties such as labels, or to increase the stability of such molecules under certain conditions. In addition, mixtures of naturally occurring nucleic acids and analogs can be made. Alternatively, mixtures of different nucleic acid analogs, and mixtures of naturally occurring nucleic acids and analogs may be made. The nucleic acids may be single stranded or double stranded, as specified, or contain portions of both double stranded or single stranded sequence. The nucleic acid may be DNA, for example, genomic or cDNA, RNA or a hybrid, from single cells, multiple cells, or from multiple species, as with metagenomic samples, such as from environmental samples. A nucleic acid can contain any combination of deoxyribo- and ribo-nucleotides, and any combination of bases, including uracil, adenine, thymine, cytosine, guanine, inosine, xanthanine, hypoxanthanine, isocytosine, isoguanine, and base analogs such as nitropyrrole (including 3-nitropyrrole) and nitroindole (including 5-nitroindole), etc.

In some embodiments, a nucleic acid can include at least one promiscuous base. Promiscuous bases can base-pair with more than one different type of base. In some embodiments, a promiscuous base can base-pair with at least two different types of bases and no more than three different types of bases. An example of a promiscuous base includes inosine that may pair with adenine, thymine, or cytosine. Other examples include hypoxanthine, 5-nitroindole, acylic 5-nitroindole, 4-nitropyrazole, 4-nitroimidazole and 3-nitropyrrole (Loakes et al., Nucleic Acid Res. 22:4039 (1994); Van Aerschot et al., Nucleic Acid Res. 23:4363 (1995); Nichols et al., Nature 369:492 (1994); Bergstrom et al., Nucleic Acid Res. 25:1935 (1997); Loakes et al., Nucleic Acid Res. 23:2361 (1995); Loakes et al., J. Mol. Biol. 270:426 (1997); and Fotin et al., Nucleic Acid Res. 26:1515 (1998)). Promiscuous bases that can base-pair with at least three, four or more types of bases can also be used.

As used herein, the term "nucleotide analog" and/or grammatical equivalents thereof can refer to synthetic analogs having modified nucleotide base portions, modified pentose portions, and/or modified phosphate portions, and, in the case of polynucleotides, modified internucleotide linkages, as generally described elsewhere (*e.g.*, Scheit, Nucleotide Analogs, John Wiley, New York, 1980; Englisch, Angew. Chem. Int. Ed. Engl. 30:613-29, 1991; Agarwal, Protocols for Polynucleotides and Analogs, Humana Press, 1994; and S. Verma and F. Eckstein, Ann. Rev. Biochem. 67:99-134, 1998). Generally, modified phosphate portions comprise analogs of phosphate wherein the phosphorous atom is in the +5 oxidation state and one or more of the oxygen atoms is replaced with a non-oxygen moiety, *e.g.*, sulfur. Exemplary phosphate analogs include but are not limited to phosphorothioate, phosphorodithioate, phosphoroselenoate, phosphorodiselenoate, phosphoroanilothioate, phosphoranilidate, phosphoramidate, boronophosphates, including associated counterions, *e.g.*, H$^+$, NH$_4$$^+$, Na$^+$, if such counterions are present. Example modified nucleotide base portions include but are not limited to 5-methylcytosine (5mC); C-5-propynyl analogs, including but not limited to, C-5 propynyl-C and C-5 propynyl-U; 2,6-diaminopurine, also known as 2-amino adenine or 2-amino-dA); hypoxanthine, pseudouridine, 2-thiopyrimidine, isocytosine (isoC), 5-methyl isoC, and isoguanine (isoG; *see, e.g.*, U.S. Pat. No. 5,432,272). Exemplary modified pentose portions include but are not limited to, locked nucleic acid (LNA) analogs including without limitation Bz-A-LNA, 5-Me-Bz-C-LNA, dmf-G-LNA, and T-LNA (*see, e.g.,* The Glen Report, 16(2):5, 2003; Koshkin et al., Tetrahedron 54:3607-30, 1998), and 2'- or 3'-modifications where the 2'- or 3'-position is hydrogen, hydroxy, alkoxy (*e.g.*, methoxy, ethoxy, allyloxy, isopropoxy, butoxy, isobutoxy and phenoxy), azido, amino, alkylamino, fluoro, chloro, or bromo. Modified internucleotide linkages include phosphate analogs, analogs having achiral and uncharged intersubunit linkages (*e.g.*, Sterchak, E. P. et al., Organic Chem., 52:4202, 1987), and uncharged morpholino-based polymers having achiral intersubunit linkages (*see, e.g.*, U.S. Pat. No. 5,034,506). Some internucleotide linkage analogs include morpholidate, acetal, and polyamide-linked heterocycles. In one class of nucleotide analogs, known as peptide nucleic acids, including pseudocomplementary peptide nucleic acids ("PNA"), a conventional sugar and internucleotide linkage has been replaced with a 2-aminoethylglycine amide backbone polymer (*see, e.g.*, Nielsen et al., Science, 254:1497-1500, 1991; Egholm et al., J. Am. Chem. Soc., 114: 1895-1897 1992; Demidov et al., Proc. Natl. Acad. Sci. 99:5953-58, 2002; Peptide Nucleic Acids: Protocols and Applications, Nielsen, ed., Horizon Bioscience, 2004).

As used herein, the term "sequencing read" and/or grammatical equivalents thereof can refer to a repetitive process of physical or chemical steps that is carried out to obtain signals indicative of the order of monomers in a polymer. The signals can be indicative of an order of monomers at single monomer resolution or lower resolution. In particular embodiments, the steps can be initiated on a nucleic acid target and carried out to obtain signals indicative of the order of bases in the nucleic acid target. The process can be carried out to its typical completion, which is usually defined by the point at which signals from the process can no longer distinguish bases of the target with a reasonable level of certainty. If desired, completion can occur earlier, for example, once a desired amount of sequence information has been obtained. A sequencing read can be carried out on a single target nucleic acid molecule or simultaneously on a population of target nucleic acid molecules having the same sequence, or simultaneously on a population of target nucleic acids having different sequences. In some embodiments, a sequencing read is terminated when signals are no longer obtained from one or more target nucleic acid molecules from which signal acquisition was initiated. For example, a sequencing read can be initiated for one or more target nucleic acid molecules that are present on a solid phase substrate and terminated upon removal of the one or more target nucleic acid molecules from the substrate. Sequencing can be terminated by otherwise ceasing detection of the target nucleic acids that were present on the substrate when the sequencing run was initiated.

As used herein, the term "sequencing representation" and/or grammatical equivalents thereof can refer to information that signifies the order and type of monomeric units in the polymer. For example, the information can indicate the order and type of nucleotides in a nucleic acid. The information can be in any of a variety of formats including, for example, a depiction, image, electronic medium, series of symbols, series of numbers, series of letters, series of colors, etc. The information can be at single monomer resolution or at lower resolution, as set forth in further detail below. An exemplary polymer is a nucleic acid, such as DNA or RNA, having nucleotide units. A series of "A," "T," "G," and "C" letters is a well known sequence representation for DNA that can be correlated, at single nucleotide resolution, with the actual sequence of a DNA molecule. Other exemplary polymers are proteins having amino acid units and polysaccharides having saccharide units.

As used herein the term "at least a portion" and/or grammatical equivalents thereof can refer to any fraction of a whole amount. For example, "at least a portion" can refer to at least about 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 99%, 99.9% or 100% of a whole amount.

**Transposomes**

A "transposome" is comprised of at least a transposase enzyme and a transposase recognition site. In some such systems, termed "transposomes", the transposase can form a functional complex with a transposon recognition site that is capable of catalyzing a transposition reaction. The transposase or integrase may bind to the transposase recognition site and insert the transposase recognition site into a target nucleic acid in a process sometimes termed "tagmentation". In some such insertion events, one strand of the transposase recognition site may be transferred into the target nucleic acid.

Some embodiments can include the use of a hyperactive Tn5 transposase and a Tn5-type transposase recognition site (Goryshin and Reznikoff, J. Biol. Chem., 273:7367 (1998)), or MuA transposase and a Mu transposase recognition site comprising R1 and R2 end sequences (Mizuuchi, K., Cell, 35: 785, 1983; Savilahti, H, et al., EMBO J., 14: 4893, 1995). An exemplary transposase recognition site that forms a complex with a hyperactive Tn5 transposase (*e.g.*, EZ-Tn5™ Transposase, Epicentre Biotechnologies, Madison, Wisconsin) comprises the following 19b transferred strand (sometimes "M" or "ME") and non-transferred strands: 5' AGATGTGTATAAGAGACAG 3', (SEQ ID NO:1), 5' CTGTCT CTTATACACATCT 3' (SEQ ID NO:2), respectively.

More examples of transposition systems that can be used with certain embodiments provided herein include *Staphylococcus aureus* Tn552 (Colegio et al., J. Bacteriol., 183: 2384-8, 2001; Kirby C et al., Mol. Microbiol., 43: 173-86, 2002), Tyl (Devine & Boeke, Nucleic Acids Res., 22: 3765-72, 1994 and International Publication WO 95/23875), Transposon Tn7 (Craig, N L, Science. 271: 1512, 1996; Craig, N L, Review in: Curr Top Microbiol Immunol., 204:27-48, 1996), Tn/O and IS10 (Kleckner N, et al., Curr Top Microbiol Immunol., 204:49-82, 1996), Mariner transposase (Lampe D J, et al., EMBO J., 15: 5470-9, 1996), Tc1 (Plasterk R H, Curr. Topics Microbiol. Immunol., 204: 125-43, 1996), P Element (Gloor, G B, Methods Mol. Biol., 260: 97-114, 2004), Tn3 (Ichikawa & Ohtsubo, J Biol. Chem. 265:18829-32, 1990), bacterial insertion sequences (Ohtsubo & Sekine, Curr. Top. Microbiol. Immunol. 204: 1-26, 1996), retroviruses (Brown, et al., Proc Natl Acad Sci USA, 86:2525-9, 1989), and retrotransposon of yeast (Boeke & Corces, Annu Rev Microbiol. 43:403-34, 1989). More examples include IS5, Tn10, Tn903, IS911, and engineered versions of transposase family enzymes (Zhang et al. (2009) PLoS Genet. 5:e1000689. Epub 2009 Oct 16; Wilson C. et al (2007) J. Microbiol. Methods 71:332-5).

**Transposon sequences**

The embodiments provided herein include transposon sequences.

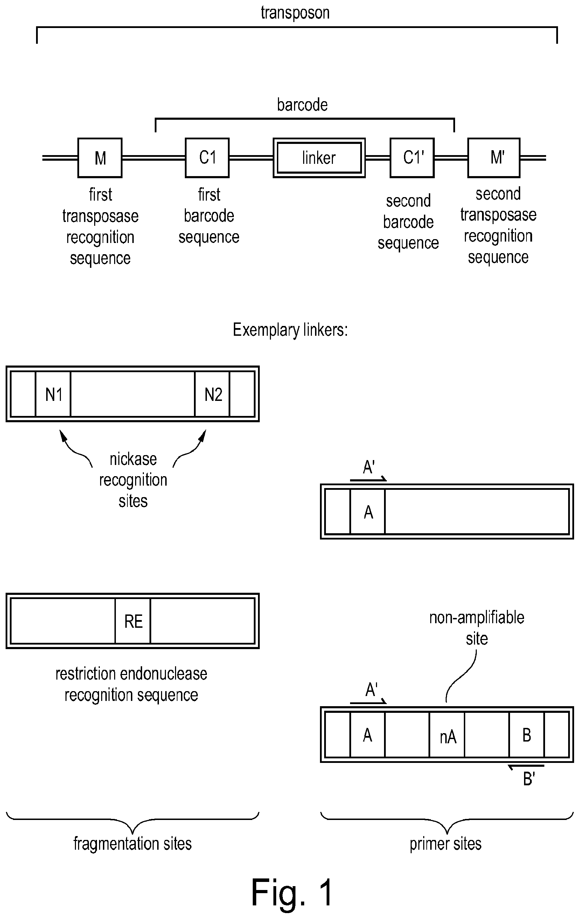

A transposon sequence includes at least one transposase recognition site and at least one barcode. In particular, a transposon sequence includes a first transposase recognition site, a second transposase recognition site, and a barcode disposed therebetween. **FIG. 1** depicts a schematic of a transposon sequence.

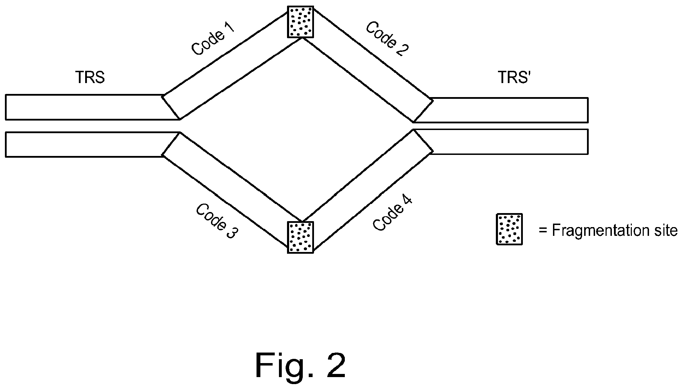

A transposase recognition site can include two complementary nucleic acid sequences, *e.g.*, a double-stranded nucleic acid or a hairpin nucleic acid, that comprise a substrate for a transposase or integrase. An exemplary embodiment of a transposon sequence comprising a double-stranded nucleic acid comprising non-complementary strands is depicted in **FIG. 2**. In some embodiments, a transposon sequence can include at least a portion comprising at least a portion comprising non-complementary sequences.

**- single-stranded sequences**

In other embodiments, the transposon sequence contains one or more single-stranded portions. Transposons with single-stranded internal sequences can be useful for avoiding the formation of hairpin structures, or when it is undesirable for transposomes to insert into previously transposome-inserted sequences ("self-insertion"), since many transposases will not insert into single-stranded DNA.

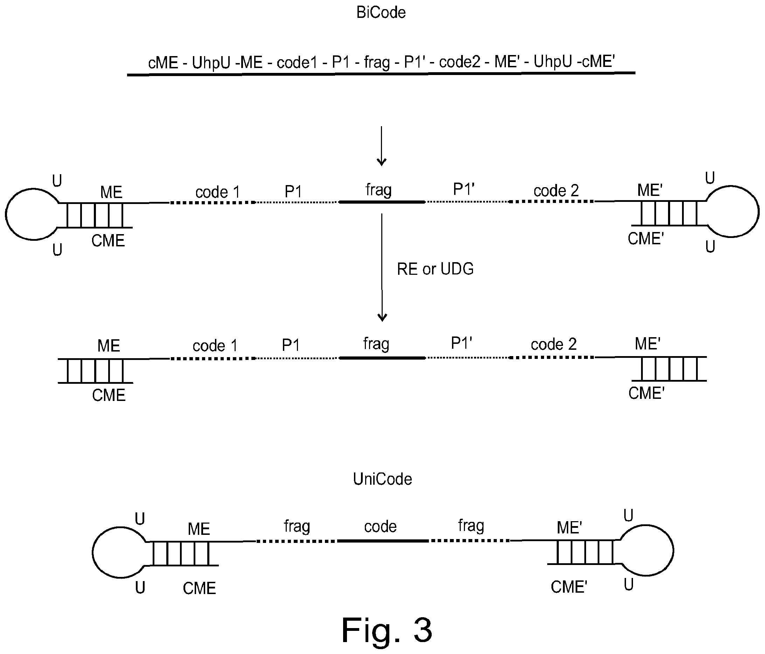

Single-stranded transposons with codes can also be prepared by making single-stranded transposons with hair-pinned ends and removing the hair-pin loops by cutting the DNA with endonucleases, such as restriction endonucleases, or uracil DNA deglycosylase (UDG), as shown in **FIG. 3**, upper part. This approach avoids sequence constraints of internal nicking recognition sites. The use of biotin-labeled primers can allow post-amplification return to single-stranded templates.

To avoid hairpins, synthesizing single-stranded transposons can be synthesized with each end forming a hairpin, as shown in **FIG. 3**, lower part. This hairpin prevents opposite-facing M-sites from forming hairpins. These hairpin structures are later processed to create active transposons with double-stranded M-sites and 5' phosphate and 3' OH groups.

**- different first and second transposase recognition sequences**

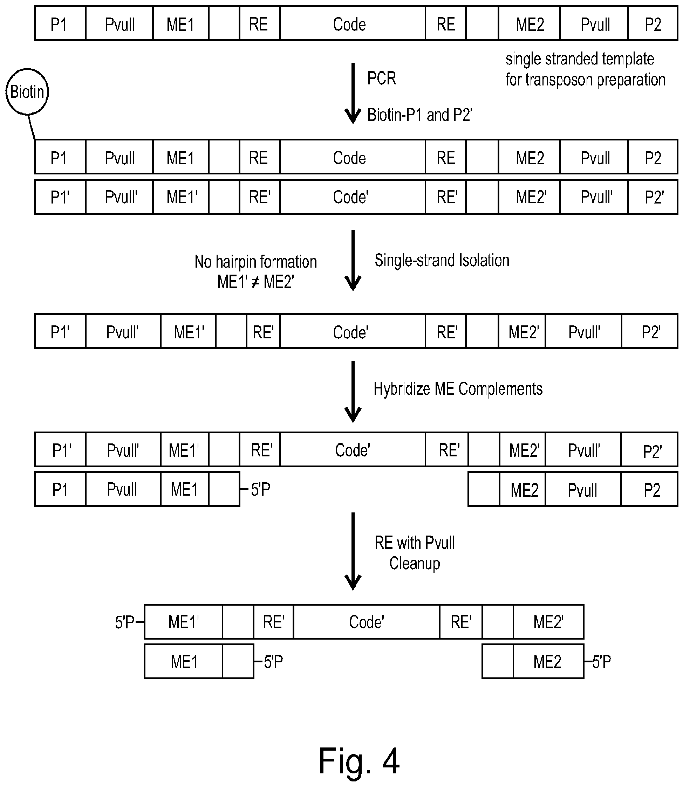

A particularly useful transposon sequence has different transposase recognition sequences (M and M') to reduce the subsequent formation of hairpin structures. For example, instead of having the same M-ends, a transposon can utilize an ME end (CTGTCTCTTATTACACATCT or ME1) with an IE (inner end) end (CTGTCTCTTGATCAGATCT or ME2). An OE (outer ends) can also be combined with an IE. Preparation of the transposon is shown in **FIG. 4**. The degree of difference can be determined by screening for how much variation can be present in the ends to allow efficient transposition. A combination of two different ends that yield high transposition efficiency can be chosen from this screening.

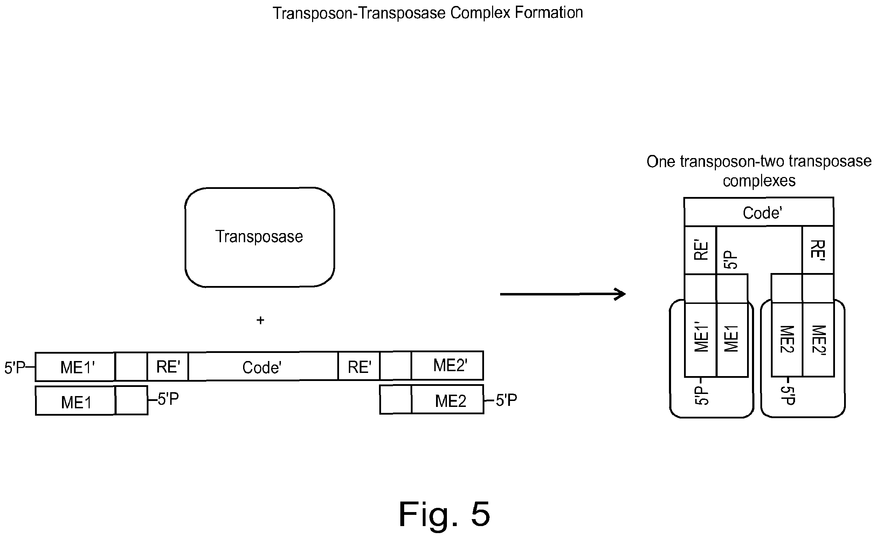

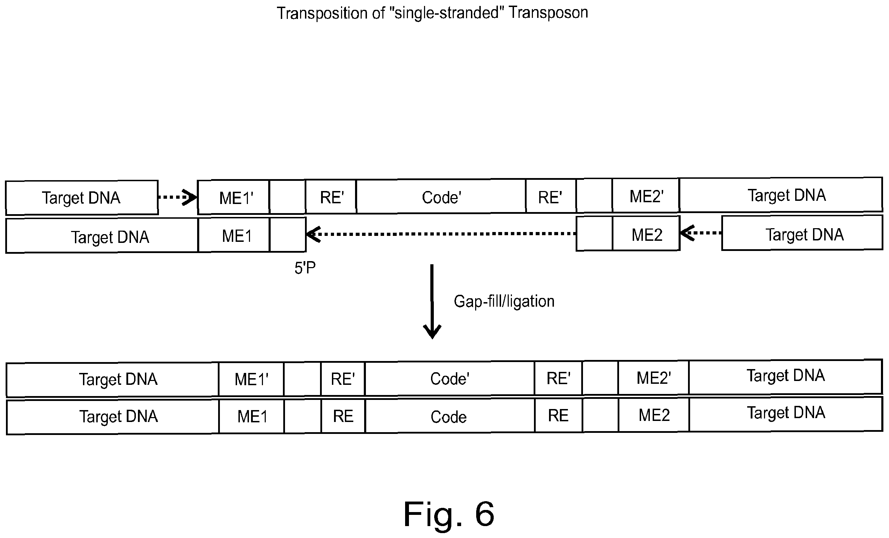

The result is a transposome complex that has one transposon sequence and two transposases, as in **FIG. 5**. As shown, the transposase does not insert into single-stranded DNA such as the internal transposon sequence. After tagmentation, the single-stranded gap can be filled and ligated, as shown in **FIG. 6**. In this figure, there is no transposition into the transposon, and no ME-ME' hairpin is formed, since ME1 and ME2 are different.

**- looped structures**

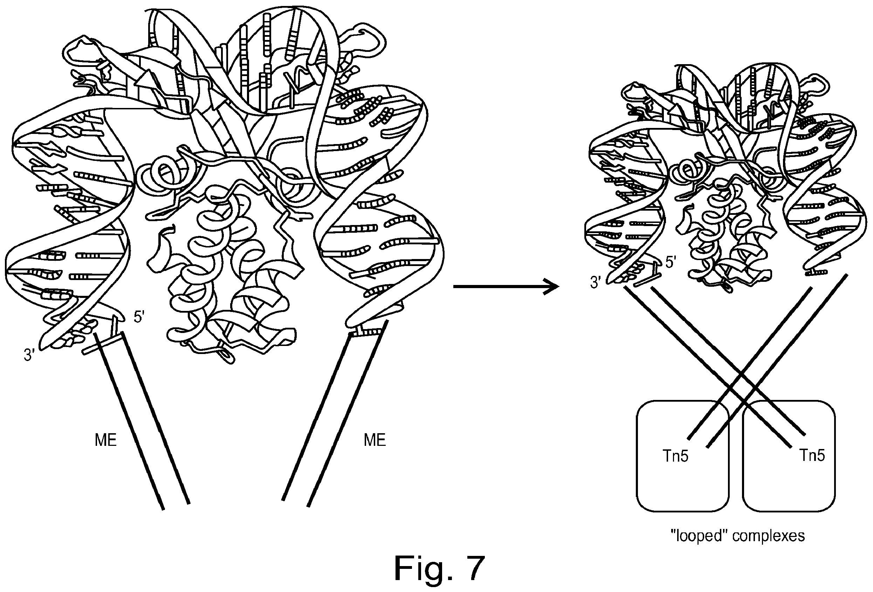

A useful transposome has one transposon that binds two transposase subunits forming a "looped" complex (**FIG. 7**). This configuration ensures that transposons are inserted into target DNA while maintaining ordering information of the original target DNA and without fragmenting the target DNA.

The transposon can be "pre-bent" in a configuration that favors the "looped" complex configuration, where transposon DNA ends have to come together and bend to derive the "looped" configuration. Without being tied to a particular mechanism, it is believed that the transposase subunits bind the ME-ends first, followed by dimerization of the subunits to form an active complex. It would be favorable for the two ends of the transposon to already be in a configuration that allows efficient intramolecular dimerization of the two bound transposase units, and can improve the yield of "looped" complex formation and stabilize the final complex. Such a configuration would also minimize undesired intermolecular dimerization products. To facilitate this bending process, the ends are positioned in close proximity, combining bending of transposon DNA with transposase complex formation.

Methods for bending DNA include surface binding, ion induction, chemical modification of DNA, protein-like bending with proteins such as Cro, histones, integration host factor (IHF), Fos, Yeast MCM1p, Jun, HU, dendrimer binding, and use of specific DNA sequences. For example, an IHF protein-DNA complex can be used to pre-bend because IHF recognizes and bends DNA at a specific sequence. Other proteins or other methods do not require a specific sequence. The introduction of one or more bends allows the creation of a specific 2- or 3-dimensional shape of the transposon that position the ME-ends in a desired position for "looped" complex formation. The agents responsible for bending can also prevent transposition into transposon-transposase complexes, and therefore favor insertion into target DNA.

Another approach favoring "looped" transposon-transposase complex formation is to relieve torsional stress, increasing rotational freedom in the transposon in order to allow the two ME-ends adapt a favorable orientation for "looped" complex formation. DNA molecules of certain sizes can be rigid ordinarily, so that nicks, gaps, single-stranded regions within the transposon can make the transposon flexible and adaptive. For example, formation of transposomes is enhanced when formed with transposons with a ~13bp region that is single-stranded, compared to its double-stranded version.

**- pre-cleaved transposomes**

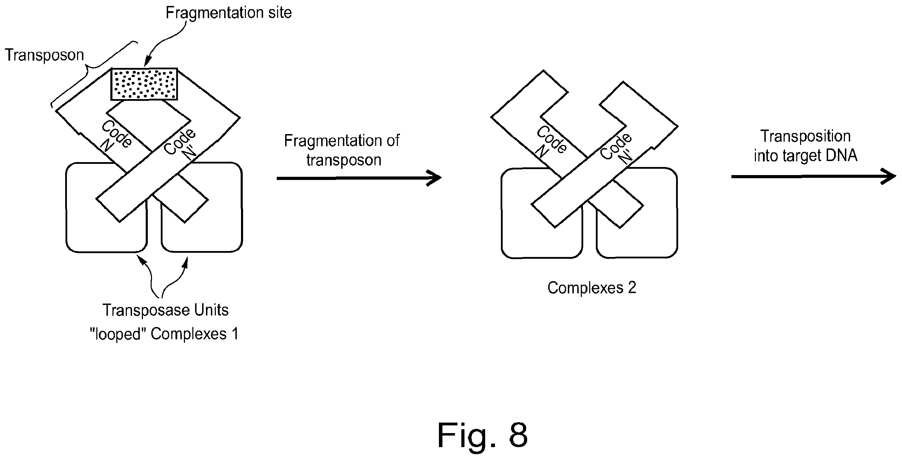

In another alternative, pre-cleaved transposomes are prepared with a looped configuration (**FIG. 8**), ensuring that neighboring DNA fragments receive code combinations that can be unambiguously assembled at a later stage of the assay. As shown, the fragmentation maintains the code combination of N and N'. Subsequent fragmentation between the code-pair combination results in transposon-transposase complexes in which the transposon-ends are not linked to each other (*i.e.* a linear configuration). However, the complexes maintain the code-combinations as they were introduced in the looped configuration. Upon insertion of the fragmented transposon into the target, DNA fragments are created with codes on the ends, allowing code-mediated assembly of the sequenced DNA fragments/libraries.

The preparation of complexes with the looped configuration can be favored by various techniques. In one technique, the complexes are prepared in a dilute environment with excess transposase to favor formation of looped complexes. A subsequent concentration step prepares the complexes at a concentration desired for insertion. In another technique, the slow addition of transposons to transposases creates an environment with low amounts of transposon with excess transposase. This reaction condition ensures that each transposon will bind to two transposase units, forming the intra-molecular complex.

**Nick-based methods**

The methods herein are not limited to using the transposomes described above. Another embodiment can involve nicking of target DNA, which exploits the genomic DNA to provide the junction tags. Nicking methods can also be used in combination with transposon methods, such as preceding or following tagmentation. Any method that nicks a single strand of the target DNA may be used, preferably those that create random gaps in DNA. When random, the nicking methods can avoid any insertional bias that transposons may have. Alternatively, sequence-specific nicking enzymes can be used, or enzymes or chemical methods to remove a base or nucleotide from a single strand. Sequence-specific nicking enzymes include Nt.CviPII, Nb.BsmI, Nb.BbvCI, Nb.Bsr.DI, Nb.BtsI, Nt.BsmAI, Nt.BbvCI, Nt.BspQI, Nt.AlwI, and Nt.BstNBI (New England Biolabs).

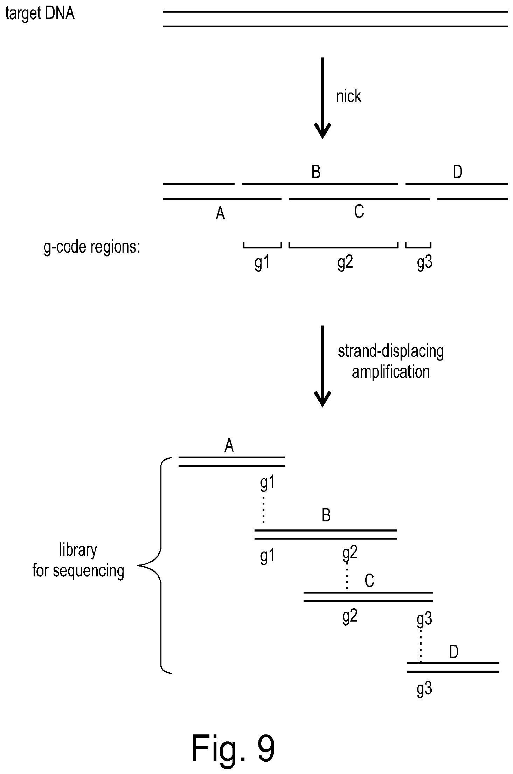

As shown in **FIG. 9**, strand-displacement synthesis is used to copy identical flanking genomic DNA sequences (g-codes) at the end of formerly juxtaposed DNA fragments. The g-code is endogenous and therefore all the sequenced bases belong to the target DNA and not to any exogenously introduced code. After DNA fragments have obtained two identical g-codes, adapters or unique adapters (adapters with unique barcode) can be ligated to allow sequencing. The g-code sequences then serve as junction tags to assemble the individual fragments.

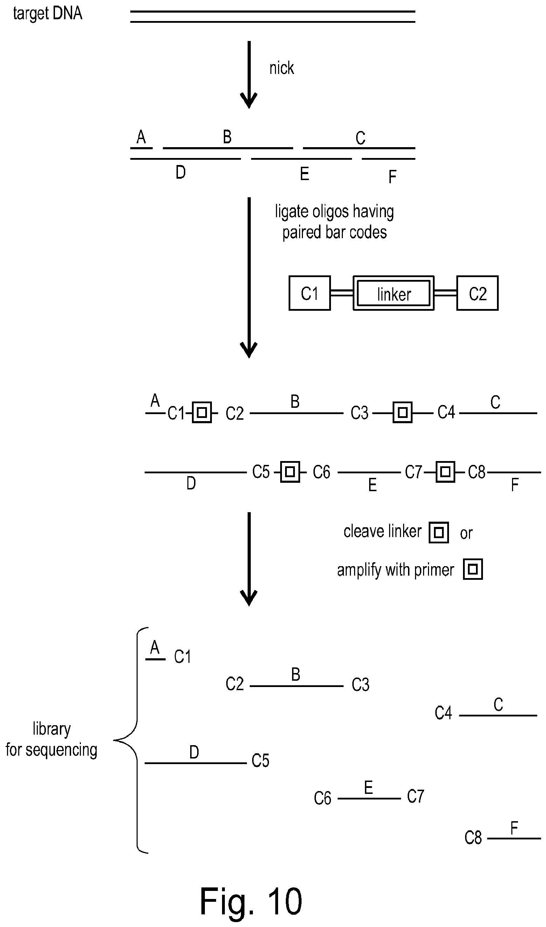

Alternatively, nicking followed by 3'-exo digestion can create gaps in genomic DNA without fragmenting the entire DNA sample, as shown in **FIG. 10**. The gaps can then be filled with code-containing oligonucleotides. For example, the oligonucleotides can be ligated to the two ends that were exposed by the nicking. Depending on the ligation method used (*e.g.* chemical or enzymatic), ligation may also be facilitated by phosphorylation of the oligo or the nicked end.

**Barcodes**

Generally, a barcode can include one or more nucleotide sequences that can be used to identify one or more particular nucleic acids. The barcode can be an artificial sequence, or can be a naturally occurring sequence, such as a g-code, described herein. A barcode can comprise at least about 1, 2, 3, 4, 5, 6, 7, 8, 9, 10, 11, 12, 13, 14, 15, 16, 17, 18, 19, 20 or more consecutive nucleotides. In some embodiments, a barcode comprises at least about 10, 20, 30, 40, 50, 60, 70 80, 90, 100 or more consecutive nucleotides. In some embodiments, at least a portion of the barcodes in a population of nucleic acids comprising barcodes is different. In some embodiments, at least about 10%, 20%, 30%, 40%, 50%, 60%, 70%, 80%, 90%, 95%, 99% of the barcodes are different. In more such embodiments, all of the barcodes are different. The diversity of different barcodes in a population of nucleic acids comprising barcodes can be randomly generated or non-randomly generated.

In the invention described herein, a transposon sequence comprises at least one barcode. In some embodiments, a transposon sequence comprises a barcode comprising a first barcode sequence and a second barcode sequence. In some such embodiments, the first barcode sequence can be identified or designated to be paired with the second barcode sequence. For example, a known first barcode sequence can be known to be paired with a known second barcode sequence using a reference table comprising a plurality of first and second bar code sequences known to be paired to one another.

In another example, the first barcode sequence can comprise the same sequence as the second barcode sequence. In another example, the first barcode sequence can comprise the reverse complement of the second barcode sequence. In some embodiments, the first barcode sequence and the second barcode sequence are different ("bi-codes").

**- bi-codes**

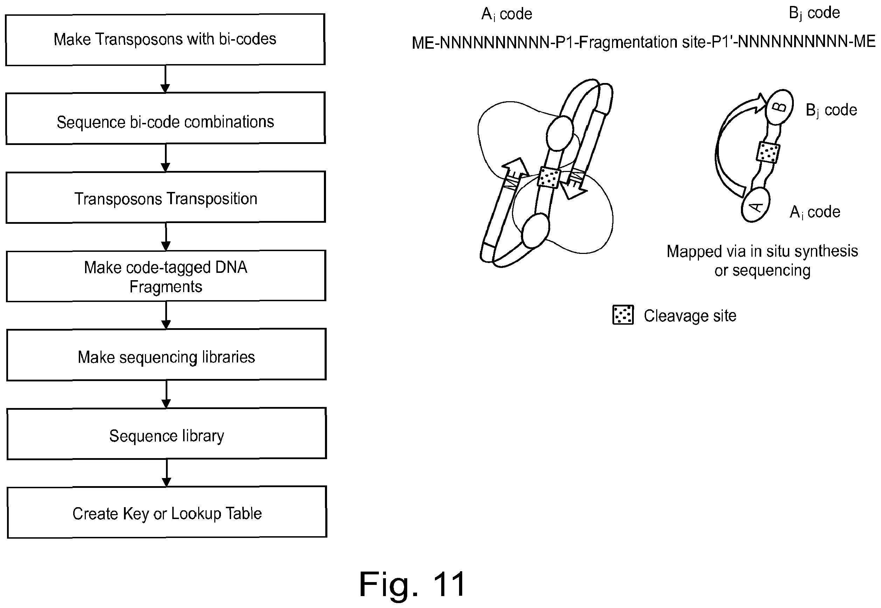

As depicted in **FIG. 11**, transposons can be generated with either engineered bi-codes or random bi-codes. In the case of random bi-codes, the code pool may be down-selected (sub-sampled) to create unique code-code pairs. These unique bi-code combinations can then be identified by sequencing the transposons. The particular bi-code combinations ($A_i$ and $B_j$) present in the final pool are determined and allow creation of a look-up table, which can be used to reassemble reads as described in the methods below.

An advantage of this approach is that not every code combination needs to be prepared individually. For example, *in situ* array technology can synthesize transposons or precursors with pre-designed code combinations in spatially separated locations, generating greater than $10^{18}$ different transposons or precursors in a single synthesis. To create unique bi-code maps, the codes are preferably down-selected so that a single first code is paired with only a single second code.

A method for reducing the presence of non-unique code-combinations (*e.g.*, Code1-Code2 and Code1-Code3 both present) is the use of "bottle-necking" or sub-sampling approaches. In these approaches, a large pool of many code combinations is generated. For example, two 15bp random sequences ($4^{15}$) can result in more than $10^{18}$ different transposons. Taking a fraction of this high-complexity pool results in unique code-combinations for the sub-sampled fraction.

In another embodiment, a transposon sequence comprising a double-strand nucleic acid, each strand can comprise a different barcode sequence. As will be understood, such a transposon sequence can be useful to inhibit insertion of other sequences into the transposon sequence by transposition. Such transposon sequences can also be used to tag particular strands of a double-stranded target nucleic acid, for example in methods that include haplotypes sequencing. An exemplary transposon sequence comprising a plurality of barcode sequences is depicted in **FIG. 2**.

**- half-transposons**

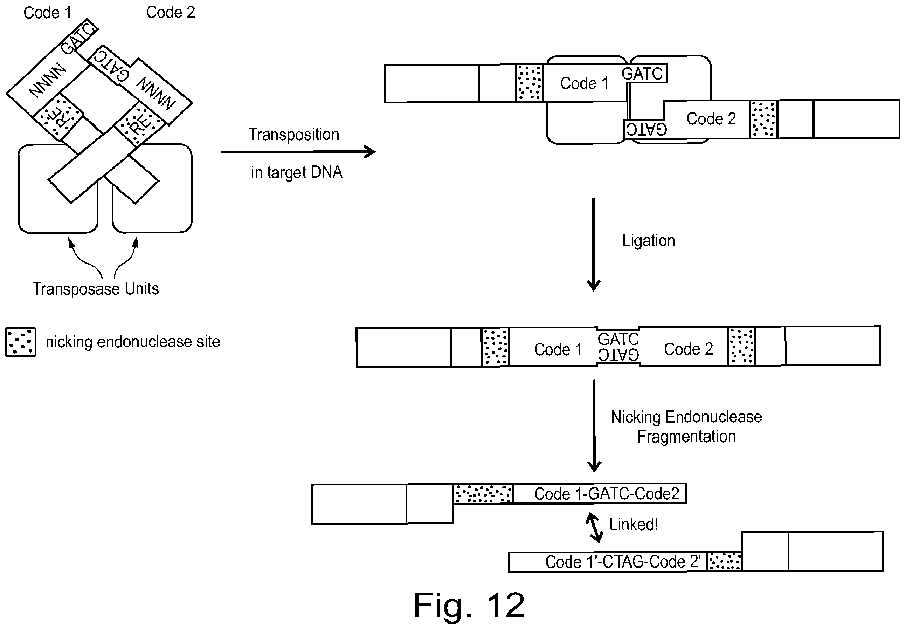

In another embodiment, code combinations are introduced after formation of the transposon-transposase complex. Complexes with non-linked ME regions can generate linked code information after transposon insertion and subsequent downstream assay steps. Complexes can be prepared as illustrated in **FIG. 12**, in which transposon ends are not linked. For example, complexes can be formed from two "half-transposons", each containing one ME region and a random or specific code region. This generates transposase complexes with randomly inserted transposons ends; no prior knowledge of pairing between the two half-codes is required. Each transposon contains a ME sequence for transposase recognition, a random or specific code, and an endonuclease fragmentation system. Upon insertion, the transposase enzyme can prevent target DNA fragmentation by binding both transposon ends. The ends of transposons can now be ligated to each other via the sticky ends. Upon nicking each strand (*e.g.*, by a sequence-specific fragmentation system, such as a restriction endonuclease), sticky code-tagged ends can be created that link one DNA fragment to the other. The codes generated at each junction are reverse complements to one another. Denaturation creates DNA fragments in which each DNA fragment contains a linked code with the neighboring fragment. An advantage of this embodiment is that transposon-transposase complexes with specific or random codes can readily be prepared, without the need for making complexes with looped configurations. Furthermore in this embodiment, the code combination does not have to be introduced at the transposase complex level, but can be generated at a later stage in the assay.

As will be described further herein, paired first and second barcode sequences can be used to identify different nucleic acids comprising barcodes linked with one another.

**Linkers**

The invention described herein includes transposon sequences comprising a first barcode sequence and a second barcode sequence having a linker disposed therebetween.

The linker can comprise, for example, one or more of a nucleotide, a nucleic acid, a non-nucleotide chemical moiety, a nucleotide analogue, amino acid, peptide, polypeptide, or protein. In preferred embodiments, a linker comprises a nucleic acid. The linker can comprise at least about 1, 2, 3, 4, 5, 6, 7, 8, 9, 10, 11, 12, 13, 14, 15, 16, 17, 18, 19, 20, or more nucleotides. In some embodiments, a linker can comprise at least about 10, 20, 30, 40, 50, 60, 70, 80, 90, 100, or more nucleotides.

**- nonamplifiable linkers**

The linkers used in the invention include a non-amplifiable site.

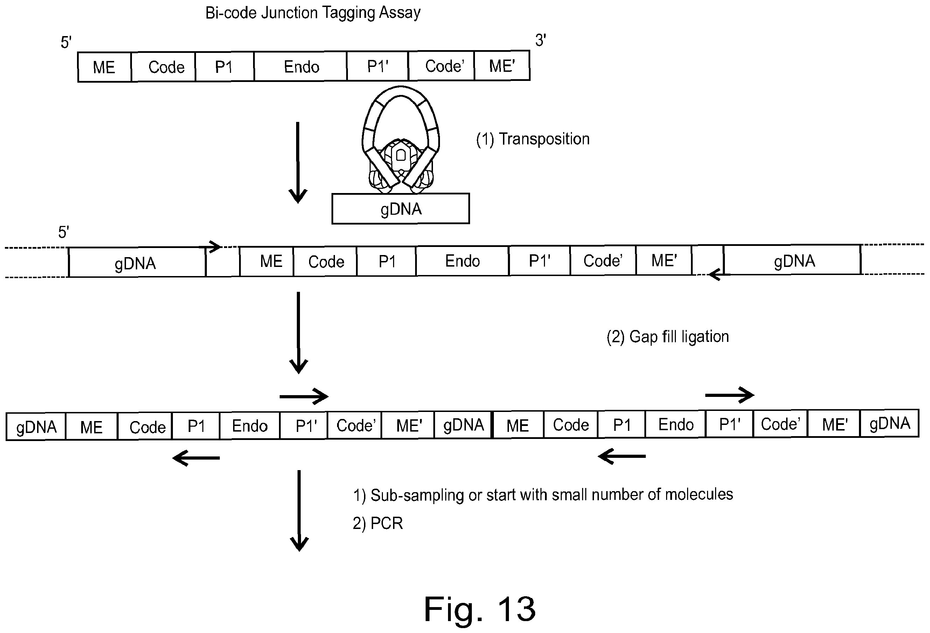

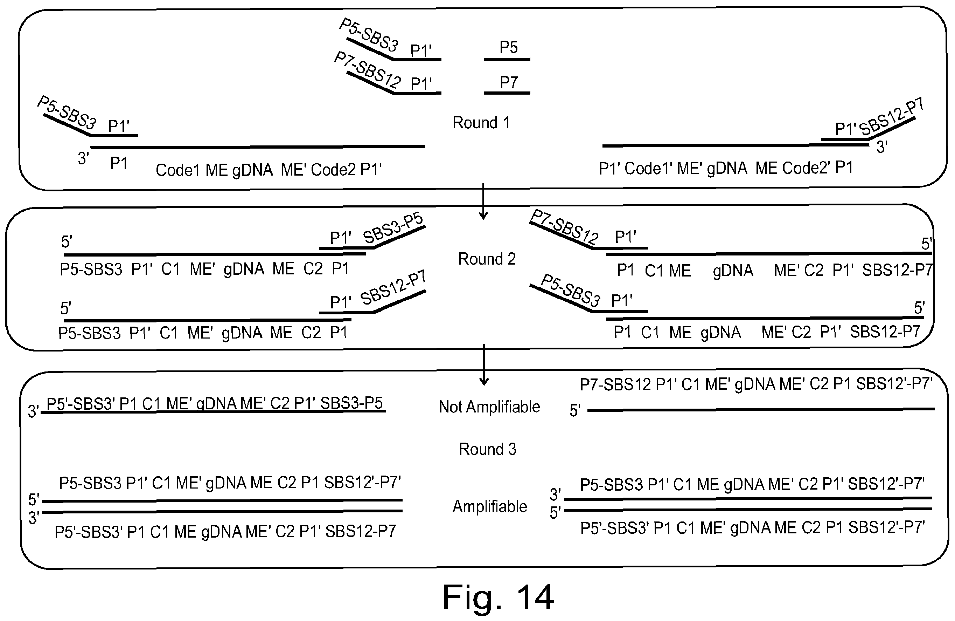

In an embodiment, non-amplifiable linkers can be introduced between ME regions (**FIG. 13**), which can be useful in methods using bi-codes, as in **FIG. 14**. Prior to Round 1 in the figure, it is preferable to perform an amplification with P1 primers to increase the number of copies, and to ensure that all templates in the population are sequenceable. Examples of non-amplifiable linkers include organic chemistry linkers such as alkyl, propyl, PEG; non-natural bases such as IsoC, isoG; or any group that does not amplify in DNA-based amplification schemes. For example, transposons containing isoC, isoG pairs can be amplified with dNTPs mixtures lacking a complementary isoG and isoC, ensuring that no amplification occurs across the inserted transposons.

In this method, transposons are inserted into target DNA with the following configuration: ME-Code*N*-P1-linker-P1'-Code*N*'-ME so that code*N* and Code*N*' generate the code combination together. P1 is a primer and P1' is its complement. PCR amplification with P1 or P1' (*e.g.*, generates DNA fragments that are code-tagged. Fragmentation at the linker site or a non-amplifiable linker can reduce undesirable amplification across transposons, which may reduce the yield of the libraries or generate undesired products.

**Fragmentation sites**

In some embodiments, the linker can comprise a fragmentation site. A fragmentation site can be used to cleave the physical, but not the informational association between a first barcode sequence and a second barcode sequence. Cleavage may be by biochemical, chemical or other means. In some embodiments, a fragmentation site can include a nucleotide or nucleotide sequence that may be fragmented by various means. For example, a fragmentation site may be a substrate for an enzyme, such as a nuclease, that will cleave the physical association between a first barcode sequence and a second barcode sequence. For example, the fragmentation site comprises a restriction endonuclease site and may be cleaved with an appropriate restriction endonuclease. In another example, a fragmentation site can comprise at least one ribonucleotide in a nucleic acid that may otherwise comprise deoxyribonucleotides and may be cleaved with an RNAse. Chemical cleavage agents capable of selectively cleaving the phosphodiester bond between a deoxyribonucleotide and a ribonucleotide include metal ions, for example rare-earth metal ions (*e.g.*, La$^{3+}$, particularly Tm$^{3+}$, Yb$^{3+}$ or Lu$^{3+}$ (Chen et al. Biotechniques. 2002, 32: 518-520; Komiyama et al. Chem. Commun. 1999, 1443-1451)), Fe(3) or Cu(3), or exposure to elevated pH, *e.g.*, treatment with a base such as sodium hydroxide. As used herein, selective cleavage of the phosphodiester bond between a deoxyribonucleotide and a ribonucleotide can refer to the chemical cleavage agent is not capable of cleaving the phosphodiester bond between two deoxyribonucleotides under the same conditions.

In another example, the fragmentation site can comprise one or more recognition sequences for a nickase, that is, a nicking endonuclease that breaks one strand of a double-stranded nucleic acid. Thus, the fragmentation site can comprise a first nickase recognition sequence, a second nickase recognition sequence. The cut site for each recognition sequence can be the same site or different site.

In another example, a fragmentation site can include one or more nucleotide analogues that comprise an abasic site and permits cleavage at the fragmentation site in the presence of certain chemical agents, such as polyamine, N,N'-dimethylethylenediamine (DMED) (U.S. Patent Publication No. 2010/0022403). In some embodiments, an abasic site may be created within a fragmentation site by first providing a fragmentation site comprising a deoxyuridine (U) of a double stranded nucleic acid. The enzyme uracil DNA glycosylase (UDG) may then be used to remove the uracil base, generating an abasic site on one strand. The polynucleotide strand including the abasic site may then be cleaved at the abasic site by treatment with endonuclease (e.g. Endo IV endonuclease, AP lyase, FPG glycosylase/AP lyase, Endo VIII glycosylase/AP lyase), heat or alkali. Abasic sites may also be generated at nucleotide analogues other than deoxyuridine and cleaved in an analogous manner by treatment with endonuclease, heat or alkali. For example, 8-oxo-guanine can be converted to an abasic site by exposure to FPG glycosylase. Deoxyinosine can be converted to an abasic site by exposure to AlkA glycosylase. The abasic sites thus generated may then be cleaved, typically by treatment with a suitable endonuclease (e.g. Endo IV, AP lyase). (U.S. Patent Publication No. 2011/0014657).

In another example, a fragmentation site may include a diol linkage which permits cleavage by treatment with periodate (*e.g.*, sodium periodate). In another example, a fragmentation site may include a disulphide group which permits cleavage with a chemical reducing agent, e.g. Tris (2-carboxyethyl)-phosphate hydrochloride (TCEP).

In some embodiments, a fragmentation site may include a cleavable moiety that may be subject to photochemical cleavage. Photochemical cleavage encompasses any method which utilizes light energy in order to achieve cleavage of nucleic acids, for example, one or both strands of a double-stranded nucleic acid molecule. A site for photochemical cleavage can be provided by a non-nucleotide chemical moiety in a nucleic acid, such as phosphoramidite [4-(4,4'-dimethoxytrityloxy)butyramidomethyl)-1-(2-nitrophenyl)-ethyl]-2-cyanoethyl-(N,N-diisopropyl)-phosphoramidite)  (Glen Research, Sterling, Va., USA, Cat No. 10-4913-XX).

In some embodiments, a fragmentation site can include a peptide, for example, conjugate structure in which a peptide molecule is linked to a nucleic acid. The peptide molecule can subsequently be cleaved by a peptidase enzyme of the appropriate specificity, or any other suitable means of non-enzymatic chemical or photochemical cleavage. In some embodiments, a conjugate between peptide and nucleic acid will be formed by covalently linking a peptide to a nucleic acid, *e.g.*, a strand of a double-stranded nucleic acid. Conjugates between a peptide and nucleic acid can be prepared using techniques generally known in the art. In one such technique the peptide and nucleic acid components of the desired amino acid and nucleotide sequence can be synthesized separately, e.g. by standard automated chemical synthesis techniques, and then conjugated in aqueous/organic solution. By way of example, the OPeC™ system commercially available from Glen Research is based on the native ligation of an N-terminal thioester-functionalized peptide to a 5'-cysteinyl oligonucleotide.

## Primer sites

A linker as used in the invention can be said to be a "sequencing adaptor" or "sequencing adaptor site", that is to say a region that comprises one or more sites that can hybridize to a primer. In the invention, a linker comprises at least a first primer site and a second primer site. The orientation of the primer sites can be such that a primer hybridizing to the first primer site and a primer hybridizing to the second primer site are in the same orientation, or in different orientations. In one embodiment, the primer sequence in the linker can be complementary to a primer used for amplification. In another embodiment, the primer sequence is complementary to a primer used for sequencing.

In the invention, a linker includes a first primer site, a second primer site having a non-amplifiable site disposed therebetween. The non-amplifiable site is useful to block extension of a polynucleotide strand between the first and second primer sites, wherein the polynucleotide strand hybridizes to one of the primer sites. The non-amplifiable site can also be useful to prevent concatamers. Examples of non-amplifiable sites include a nucleotide analogue, non-nucleotide chemical moiety, amino-acid, peptide, and polypeptide. In some embodiments, a non-amplifiable site comprises a nucleotide analogue that does not significantly base-pair with A, C, G or T.

Some embodiments include a linker comprising a first primer site, a second primer site having a fragmentation site disposed therebetween.

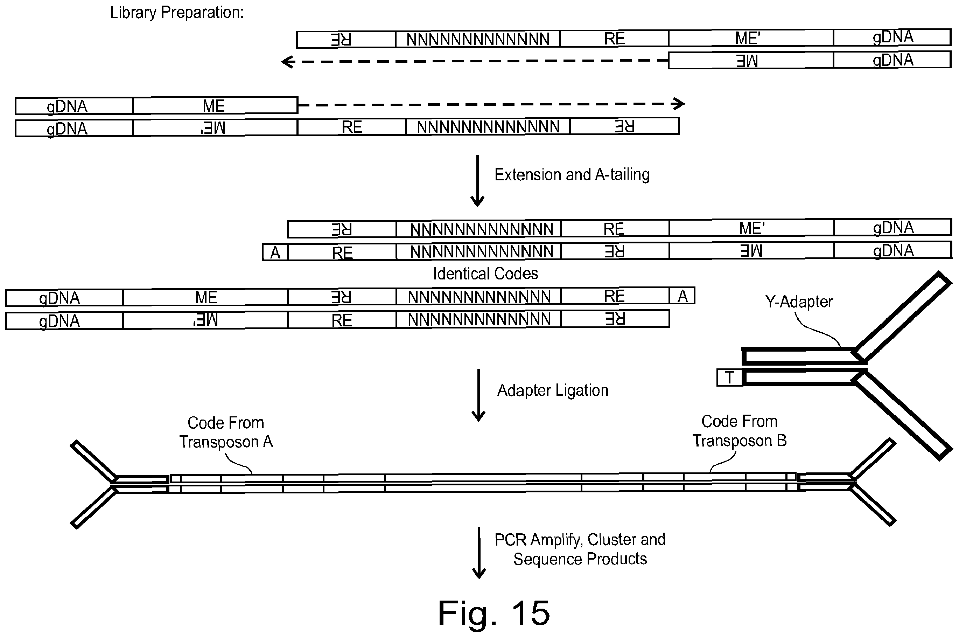

Other embodiments can use a forked or Y-shaped adapter design useful for directional sequencing, as described in U.S. Patent No. 7,741,463. An example is shown in **FIG. 15**.

## Affinity tags

In some embodiments, a linker can comprise an affinity tag. Affinity tags can be useful for the bulk separation of target nucleic acids hybridized to hybridization tags. As used herein, the term "affinity tag" and grammatical equivalents can refer to a component of a multi-component complex, wherein the components of the multi-component complex specifically interact with or bind to each other. For example an affinity tag can include biotin or His that can bind streptavidin or nickel, respectively. Other examples of multiple-component affinity tag complexes include, ligands and their receptors, for example, avidin-biotin, streptavidin-biotin, and derivatives of biotin, streptavidin, or avidin, including, but not limited to, 2-iminobiotin, desthiobiotin, NeutrAvidin (Molecular Probes, Eugene, Oreg.), CaptAvidin (Molecular Probes), and the like; binding proteins/peptides, including maltose-maltose binding protein (MBP), calcium-calcium binding protein/peptide (CBP); antigen-antibody, including epitope tags, including c-MYC (*e.g.*, EQKLISEEDL (SEQ ID NO: 3)), HA (*e.g.*, YPYDVPDYA (SEQ ID NO: 4)), VSV-G (*e.g.*, YTDIEMNRLGK (SEQ ID NO: 5)), HSV (*e.g.*, QPELAPEDPED (SEQ ID NO: 6)), V5 (*e.g.*, GKPIPNPLLGLDST (SEQ ID NO: 7)), and FLAG Tag™, (*e.g.*, DYKDDDDKG (SEQ ID NO: 8)), and their corresponding anti-epitope antibodies; haptens, for example, dinitrophenyl and digoxigenin, and their corresponding antibodies; aptamers and their corresponding targets; poly-His tags (*e.g.*, penta-His and hexa-His) and their binding partners including corresponding immobilized metal ion affinity chromatography (IMAC) materials and anti-poly-His antibodies; fluorophores and anti-fluorophore antibodies; and the like.

## Reporter moieties

In some embodiments, a linker can comprise a reporter moiety. As used herein, the term "reporter moiety" and grammatical equivalents can refer to any identifiable tag, label, or group. The skilled artisan will appreciate that many different species of reporter moieties can be used with the methods and compositions described herein, either individually or in combination with one or more different reporter moieties. In certain embodiments, a reporter moiety can emit a signal. Examples of signals fluorescent, a chemiluminescent, a bioluminescent, a phosphorescent, a radioactive, a calorimetric, or an electrochemiluminescent signals. Example reporter moieties include fluorophores, radioisotopes, chromogens, enzymes, antigens including epitope tags, semiconductor nanocrystals such as quantum dots, heavy metals, dyes, phosphorescence groups, chemiluminescent groups, electrochemical detection moieties, binding proteins, phosphors, rare earth chelates, transition metal chelates, near-infrared dyes, electrochemiluminescence labels, and mass spectrometer compatible reporter moieties, such as mass tags, charge tags, and isotopes. More reporter moieties that may be used with the methods and compositions described herein include spectral labels such as fluorescent dyes (*e.g.*, fluorescein isothiocyanate, Texas red, rhodamine, and the like), radiolabels (*e.g.*, $^{3}$H, $^{125}$I, $^{35}$S, $^{14}$C, $^{32}$P, $^{33}$P, etc.), enzymes (*e.g.*, horseradish peroxidase, alkaline phosphatase etc.) spectral calorimetric labels such as colloidal gold or colored glass or plastic (e.g. polystyrene, polypropylene, latex, etc.) beads; magnetic, electrical, thermal labels; and mass tags. Reporter moieties can also include enzymes (horseradish peroxidase, etc.) and magnetic particles. More reporter moieties include chromophores, phosphors and fluorescent moieties, for example, Texas red, dixogenin, biotin, 1- and 2-aminonaphthalene, p,p'-diaminostilbenes, pyrenes, quaternary phenanthridine salts, 9-aminoacridines, p,p'-diaminobenzophenone imines, anthracenes, oxacarbocyanine, merocyanine, 3-aminoequilenin, perylene, bis-benzoxazole, bis-p-oxazolyl benzene, 1,2-benzophenazin, retinol, bis-3-aminopyridinium salts, hellebrigenin, tetracycline, sterophenol, benzimidazolylphenylamine, 2-oxo-3-chromen, indole, xanthen, 7-hydroxycoumarin, phenoxazine, calicylate, strophanthidin, porphyrins, triarylmethanes and flavin. Individual fluorescent compounds which have functionalities for linking to an element desirably detected in an apparatus or assay provided herein, or which can be modified to incorporate such functionalities include, *e.g.*, dansyl chloride; fluoresceins such as 3,6-dihydroxy-9-phenylxanthydrol; rhodamineisothiocyanate; N-phenyl 1-amino-8-sulfonatonaphthalene; N-phenyl 2-amino-6-sulfonatonaphthalene; 4-acetamido-4-isothiocyanato-stilbene-2,2'-disulfonic acid; pyrene-3-sulfonic acid; 2-toluidinonaphthalene-6-sulfonate; N-phenyl-N-methyl-2-aminoaphthalene-6-sulfonate; ethidium bromide; stebrine; auromine-0,2-(9'-anthroyl)palmitate; dansyl phosphatidylethanolamine; N,N'-dioctadecyl oxacarbocyanine: N,N'-dihexyl oxacarbocyanine; merocyanine, 4-(3'-pyrenyl)stearate; d-3-aminodesoxy-equilenin; 12-(9'-anthroyl)stearate; 2-methylanthracene; 9-vinylanthracene; 2.,2'(vinylene-p-phenylene)bisbenzoxazole; p-bis(2- -methyl-5-phenyl-oxazolyl))benzene; 6-dimethylamino-1,2-benzophenazin; retinol; bis(3'-aminopyridinium) 1,10-decandiyl diiodidc; sulfonaphthylhydrazone of hellibrienin; chlorotetracycline; N-(7-dimethylamino-4-methyl-2-oxo-3-chromenyl)maleimide; N-(p-(2benzimidazolyl)-phenyl)maleimide; N-(4-fluoranthyl)maleimide; bis(homovanillic acid); resazarin; 4-chloro7-nitro-2,1,3-benzooxadiazole; merocyanine 540; resorufin; rose bengal; 2,4-diphenyl-3(2H)-furanone, fluorescent lanthanide complexes, including those of Europium and Terbium, fluorescein, rhodamine, tetramethylrhodamine, eosin, erythrosin, coumarin, methyl-coumarins, quantum dots (also referred to as "nanocrystals": see U.S. Patent No. 6,544,732), pyrene, Malachite green, stilbene, Lucifer Yellow, Cascade Blue™, Texas Red, Cy dyes (Cy3, Cy5, etc.), Alexa Fluor® dyes, phycoerythin, bodipy, and others described in the 6th Edition of the Molecular Probes Handbook by Richard P. Haugland,.

## Certain methods of making transposon sequences

The transposon sequences provided herein can be prepared by a variety of methods. Example methods include direct synthesis and hairpin extension methods. In the case of direct synthesis, for example, a transposon sequence comprising a nucleic acid may be prepared by methods comprising chemical synthesis.

Such methods are well known in the art, *e.g.*, solid phase synthesis using phophoramidite precursors such as those derived from protected 2'-deoxynucleosides, ribonucleosides, or nucleoside analogues.

A transposon sequence can be prepared by hairpin extension. In one example, a portion of a transposon sequence may be prepared by chemical synthesis and extended by hairpin extension. In an example , a precursor transposon sequence comprising a polynucleotide may include a first transposase recognition site, and a first barcode sequence. The precursor transposon sequence may be extended using an appropriate nucleic acid polymerase by hairpin extension, thereby preparing a hairpin structure comprising a first transposase recognition site, a first barcode sequence, a second barcode sequence, and a second transposase recognition site. In such a transposon sequence, the first transposase recognition site and first barcode sequence can have the reverse complement sequence of, and the second transposase recognition site and the second barcode sequence, respectively.

Some methods of preparing transposons sequences can include preparing barcode sequences. Barcode sequences can be generated randomly and non-randomly. Some barcode sequences may or may not include sequences likely to be found in a target nucleic acid. Some barcode sequences may or may not include restriction sites.

At least a portion of a barcode sequence can be generated randomly.

A barcode sequence can be generated using combinatorial methods. In some such methods, barcode sequences can comprise one or more subunits comprising one or more consecutive nucleotides. As will be understood, in some embodiments, a subunit can comprise a nucleotide analogue and/or a nucleotide comprising a label. At least a portion of a barcode sequence can comprise at least one subunit. Barcode sequences comprising at least one subunit can be generated randomly or non-randomly.

A t least a portion of a barcode sequences comprising at least one subunit can be generated randomly or non-randomly. As will be understood, in some embodiments, at least a portion of a barcode sequence is known.

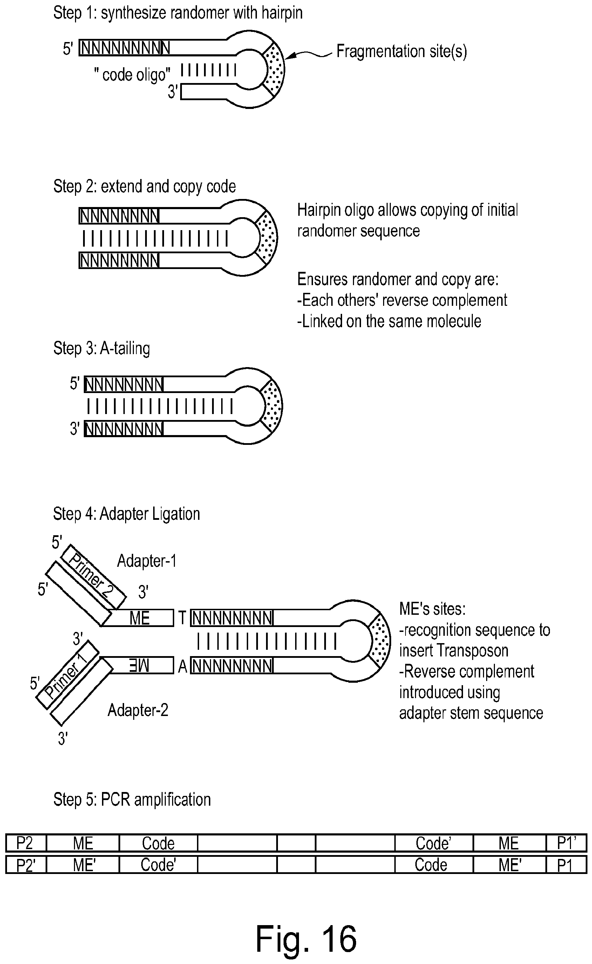

Primer sites may be ligated to the ends of the hairpin structure in order to generate a complementary strand to the single-strand of the hairpin structure. An exemplary method of making a transposon sequence is depicted in **FIG. 16**. It will be understood that the primer sites introduced to generate the complementary strand may be removed by a transposase during a transposition reaction.

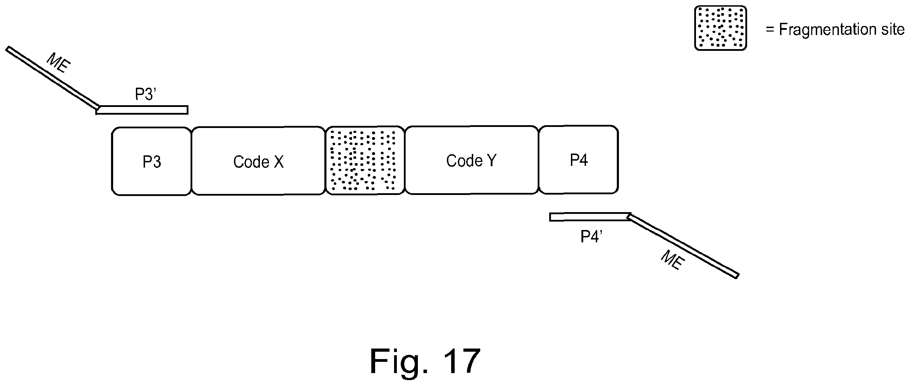

Methods of making transposon sequences can include incorporating sequences using amplification methods. Templates for amplification that include barcode sequences can be prepared by a variety of systems, for example, using *in situ* oligonucleotide arrays. Some methods of making transposon sequences include the use of tailed-oligonucleotides to incorporate sequences into a transposon sequence. An example is depicted in **FIG. 17**, where tailed-oligonucleotides comprising mosaic elements (ME) are used to prepare a transposon sequence comprising a barcode comprising Code X and Code Y, and a linker comprising a fragmentation site.

**Target nucleic acids**

A target nucleic acid can include any nucleic acid of interest. Target nucleic acids can include DNA, RNA, peptide nucleic acid, morpholino nucleic acid, locked nucleic acid, glycol nucleic acid, threose nucleic acid, mixtures thereof, and hybrids thereof. In a preferred embodiment, genomic DNA fragments or amplified copies thereof are used as the target nucleic acid. In another preferred embodiment, mitochondrial or chloroplast DNA is used.

A target nucleic acid can comprise any nucleotide sequence. In some embodiments, the target nucleic acid comprises homopolymer sequences. A target nucleic acid can also include repeat sequences. Repeat sequences can be any of a variety of lengths including, for example, 2, 5, 10, 20, 30, 40, 50, 100, 250, 500, 1000 nucleotides or more. Repeat sequences can be repeated, either contiguously or non-contiguously, any of a variety of times including, for example, 2, 3, 4, 5, 6, 7, 8, 9, 10, 15, 20 times or more.

Some embodiments described herein can utilize a single target nucleic acid. Other embodiments can utilize a plurality of target nucleic acids. In such embodiments, a plurality of target nucleic acids can include a plurality of the same target nucleic acids, a plurality of different target nucleic acids where some target nucleic acids are the same, or a plurality of target nucleic acids where all target nucleic acids are different. Embodiments that utilize a plurality of target nucleic acids can be carried out in multiplex formats so that reagents are delivered simultaneously to the target nucleic acids, for example, in one or more chambers or on an array surface. In some embodiments, the plurality of target nucleic acids can include substantially all of a particular organism's genome. The plurality of target nucleic acids can include at least a portion of a particular organism's genome including, for example, at least about 1%, 5%, 10%, 25%, 50%, 75%, 80%, 85%, 90%, 95%, or 99% of the genome. In particular embodiments the portion can have an upper limit that is at most about 1%, 5%, 10%, 25%, 50%, 75%, 80%, 85%, 90%, 95%, or 99% of the genome

Target nucleic acids can be obtained from any source. For example, target nucleic acids may be prepared from nucleic acid molecules obtained from a single organism or from populations of nucleic acid molecules obtained from natural sources that include one or more organisms. Sources of nucleic acid molecules include, but are not limited to, organelles, cells, tissues, organs, or organisms. Cells that may be used as sources of target nucleic acid molecules may be prokaryotic (bacterial cells, for example, *Escherichia, Bacillus, Serratia, Salmonella, Staphylococcus, Streptococcus,*

*Clostridium, Chlamydia, Neisseria, Treponema, Mycoplasma, Borrelia, Legionella, Pseudomonas, Mycobacterium, Helicobacter Erwinia, Agrobacterium, Rhizobium*, and *Streptomyces* genera); archeaon, such as crenarchaeota, nanoarchaeota or euryarchaeotia; or eukaryotic such as fungi, (for example, yeasts), plants, protozoans and other parasites, and animals (including insects (for example, *Drosophila* spp.), nematodes (*e.g., Caenorhabditis elegans*), and mammals (for example, rat, mouse, monkey, non-human primate and human)).

**Methods of preparing template nucleic acids**

Some embodiments include methods of preparing template nucleic acids. As used herein, the term "template nucleic acid" can refer to a target nucleic acid, a fragment thereof, or any copy thereof comprising at least one transposon sequence, a fragment thereof, or any copy thereof. Accordingly, some methods of preparing template nucleic acids include inserting a transposon sequence into a target nucleic acid, thereby preparing a template nucleic acid. Some methods of insertion include contacting a transposon sequence provided herein with a target nucleic acid in the presence of an enzyme, such as a transposase or integrase, under conditions sufficient for the integration of the transposon sequence into the target nucleic acid.

In some embodiments, insertion of transposon sequences into a target nucleic acid can be non-random. In some embodiments, transposon sequences can be contacted with target nucleic acids comprising proteins that inhibit integration at certain sites. For example, transposon sequences can be inhibited from integrating into genomic DNA comprising proteins, genomic DNA comprising chromatin, genomic DNA comprising nucleosomes, or genomic DNA comprising histones. In some embodiments, transposon sequences can be associated with affinity tags in order to integrate the transposon sequence at a particular sequence in a target nucleic acid. For example, a transposon sequence may be associated with a protein that targets specific nucleic acid sequences, *e.g.*, histones, chromatin-binding proteins, transcription factors, initiation factors, etc., and antibodies or antibody fragments that bind to particular sequence-specific nucleic-acid-binding proteins. In an exemplary embodiment, a transposon sequence is associated with an affinity tag, such as biotin; the affinity tag can be associated with a nucleic-acid-binding protein.

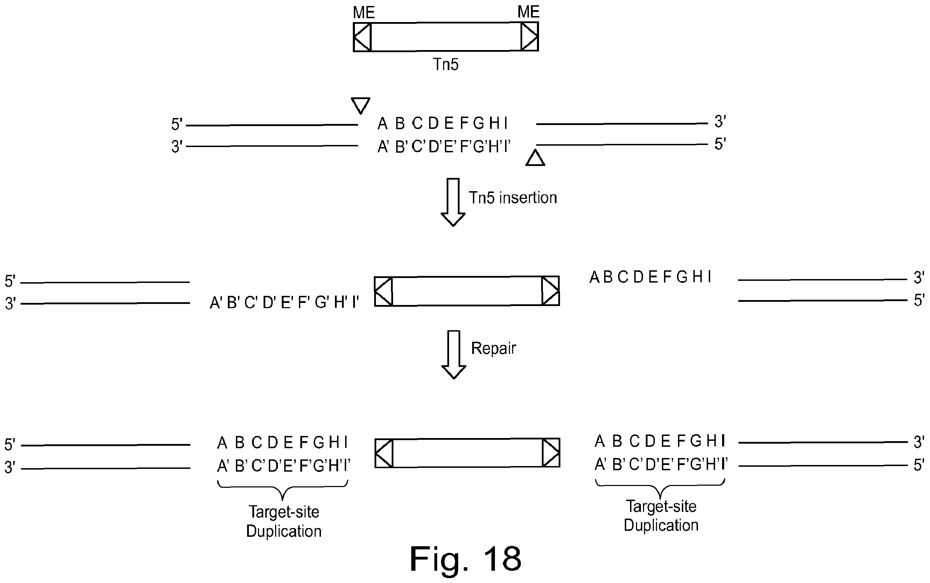

It will be understood that during integration of some transposon sequences into a target nucleic acid, several consecutive nucleotides of the target nucleic acid at the integration site are duplicated in the integrated product. Thus the integrated product can include a duplicated sequence at each end of the integrated sequence in the target nucleic acid. An example of such a duplication event is depicted in **FIG. 18**. As used herein, the term "host tag" can refer to a target nucleic acid sequence that is duplicated at each end of an integrated transposon sequence. Single-stranded portions of nucleic acids that may be generated by the insertion of transposon sequences can be repaired by a variety of methods well known in the art, for example by using ligases, oligonucleotides and/or polymerases.

In some embodiments, a plurality of the transposon sequences provided herein is inserted into a target nucleic acid. Some embodiments include selecting conditions sufficient to achieve integration of a plurality of transposon sequences into a target nucleic acid such that the average distance between each integrated transposon sequence comprises a certain number of consecutive nucleotides in the target nucleic acid.

In some embodiments, conditions for insertion of transposon sequences are sufficient to reduce the likelihood of forming concatameric complexes comprising a transposase associated with more than one transposon sequence. In one example, complexes comprising a transposase and a transposon sequence can be formed under dilute conditions; subsequent steps of inserting the transposon sequences into a target nucleic acid may be carried out at higher concentrations of transposase/transposon sequence complex. In another example, transposase/transposon sequence complexes can be prepared by contacting a circular transposon sequence with a transposase. As will be understood, the transposon sequence may be linearized during formation of the transposase/transposon sequence complex. In another example, a transposase/transposon sequence complex may be prepared by preparing complexes comprising partial transposon sequences comprising a transposon recognition site, and contacting the partial sequences with transposase monomers. Two partial transposon sequences may be ligated to one another to prepare a whole transposon sequence associated with a transposase comprising a dimer.

Some embodiments include selecting conditions sufficient to achieve insertion of a transposon sequence into a target nucleic acid, but not into another transposon sequence. A variety of methods can be used to reduce the likelihood that a transposon sequence inserts into another transposon sequence. For example, transposon sequences can comprise thiophosphate-modified nucleic acids. In another example, a transposon sequence can comprise a DNA/RNA hybrid, such as an RNA transposon sequence comprising DNA transposase recognition sites. In another example, a transposon sequence comprises a single-stranded sequence, further comprising double-stranded transposase recognition sites. It will be appreciated that more methods can include transposon sequences comprising single-stranded nucleic acids to inhibit insertion into the transposon sequence; transposon sequences comprising RNA to inhibit insertion into the transposon sequence; and transposon sequences associated with nucleic acid binding proteins to inhibit insertion into the transposon sequence.

In some embodiments, conditions may be selected so that the average distance in a target nucleic acid between integrated transposon sequences is at least about 5, 10, 20, 30, 40, 50, 60, 70, 80, 90, 100, or more consecutive nucleotides. In some embodiments, the average distance in a target nucleic acid between integrated transposon sequences is at least about 100, 200, 300, 400, 500, 600, 700, 800, 900, 1000, or more consecutive nucleotides. In some embodiments, the average distance in a target nucleic acid between integrated transposon sequences is at least about 1 kb, 2 kb, 3 kb, 4 kb, 5 kb, 6 kb, 7 kb, 8 kb, 90 kb, 100 kb, or more consecutive nucleotides. In some embodiments, the average distance in a target nucleic acid between integrated transposon sequences is at least about 100 kb, 200 kb, 300 kb, 400 kb, 500 kb, 600 kb, 700 kb, 800 kb, 900 kb, 1000 kb, or more consecutive nucleotides. As will be understood, some conditions that may be selected include contacting a target nucleic acid with a certain number of transposon sequences.

Some embodiments include selecting conditions sufficient to achieve at least a portion of transposon sequences integrated into a target nucleic acid are different. In preferred embodiments, each transposon sequence integrated into a target nucleic acid is different. Some conditions that may be selected to achieve a certain portion of transposon sequences integrated into a target sequences that are different include selecting the degree of diversity of the population of transposon sequences. As will be understood, the diversity of transposon sequences arises in part due to the diversity of the barcodes of such transposon sequences. Accordingly, some embodiments include providing a population of transposon sequences in which at least a portion of the barcodes are different. In some embodiments, at least about 10%, 20%, 30%, 40%, 50%, 60%, 70%, 80%, 90%, 95%, 98%, 99%, or 100% of barcodes in a population of transposon sequences are different.

Some embodiments of preparing a template nucleic acid can include copying the sequences comprising the target nucleic acid. For example, some embodiments include hybridizing a primer to a primer site of a transposon sequence integrated into the target nucleic acid. In some such embodiments, the primer can be hybridized to the primer site and extended. The copied sequences can include at least one barcode sequence and at least a portion of the target nucleic acid. In some embodiments, the copied sequences can include a first barcode sequence, a second barcode sequence, and at least a portion of a target nucleic acid disposed therebetween. In some embodiments, at least one copied nucleic acid can include at least a first barcode sequence of a first copied nucleic acid that can be identified or designated to be paired with a second barcode sequence of a second copied nucleic acid. In some embodiments, the primer can include a sequencing primer. In some embodiments sequencing data is obtained using the sequencing primer.

Some embodiments of preparing a template nucleic acid can include amplifying sequences comprising at least a portion of one or more transposon sequences and at least a portion of a target nucleic acid. In some embodiments, at least a portion of a target nucleic acid can be amplified using primers that hybridize to primer sites of integrated transposon sequences integrated into a target nucleic acid. In some such embodiments, an amplified nucleic acid can include a first barcode sequence, and second barcode sequence having at least a portion of the target nucleic acid disposed therebetween. In some embodiments, at least one amplified nucleic acid can include at least a first barcode sequence of a first amplified nucleic acid that can be identified to be paired with a second barcode sequence of a second amplified sequence.

### - symmetrical transposomes, adding sequences by amplification

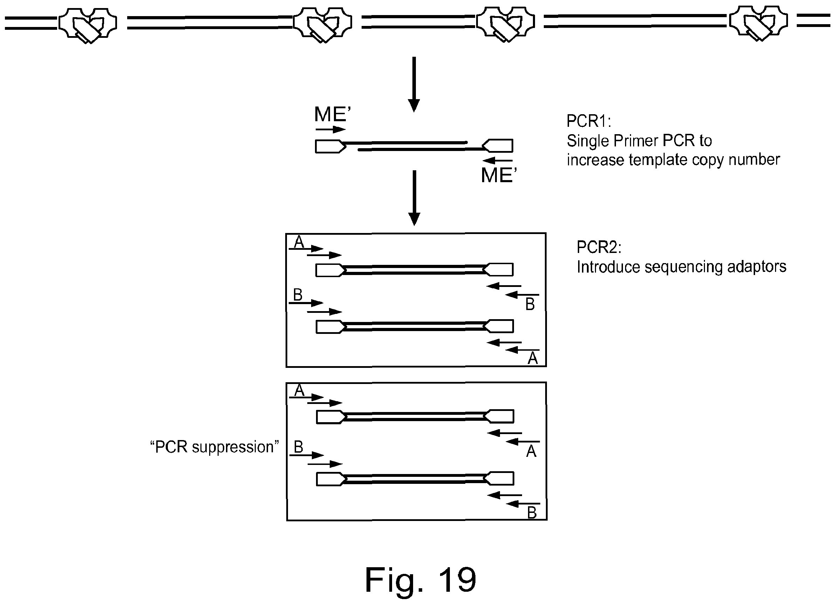

In an embodiment, transposomes are used that have symmetrical transposable end sequences, as exemplified in **FIG. 19**. Each tagmented fragment therefore contains identical ends, lacking directionality. A single primer PCR, using the transposon end sequences, can then be employed to amplify the template copy number from $2n$ to $2n*2^x$ where $x$ corresponds to the number of PCR cycles. In a subsequent step, PCR with primers can add additional sequences, such as sequencing adapter sequences.

Some embodiments of preparing a template nucleic acid can include fragmenting a target nucleic acid comprising transposon sequences. Methods of fragmenting nucleic acids are well known in the art. In some embodiments, a nucleic acid comprising transposon sequences can be fragmented at random positions along the length of the nucleic acid. In some embodiments, a target nucleic acid comprising transposon sequences can be fragmented at the fragmentation sites of the transposon sequences. In some embodiments, insertion of a transposon sequence can include the duplication of the insertion site so that the inserted transposon sequence is disposed between duplicated single-stranded sequences (*See*, *e.g.*, **FIG. 18**). In some embodiments, a polymerase may be used to cleave the fragmentation site. Examples of such polymerases include strand-displacing nucleic acid polymerases.

Further embodiments of preparing a template nucleic acid that include fragmenting a target nucleic acid comprising transposon sequences can also include amplifying the fragmented nucleic acids. In some embodiments, the fragmented nucleic acids can be amplified using primers that hybridize to primer sites of transposon sequences. In more embodiments, primer sites can be ligated to the ends of the fragmented nucleic acids. In some such embodiments, the fragmented nucleic acids with ligated primer sites can be amplified from such primer sites.

Some embodiments include reducing the complexity of a library of template nucleic acids. A complexity-reduction step can be performed before or after the fragmentation step in the method. For example, the target nucleic acid comprising the transposon sequences can be diluted so that a small number or a single molecule represents the target diluted before performing subsequent steps.

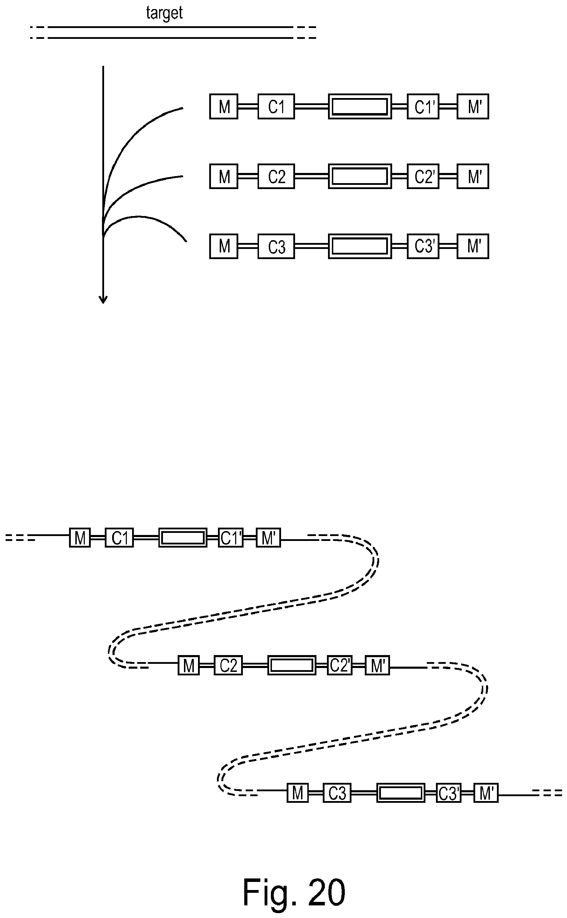

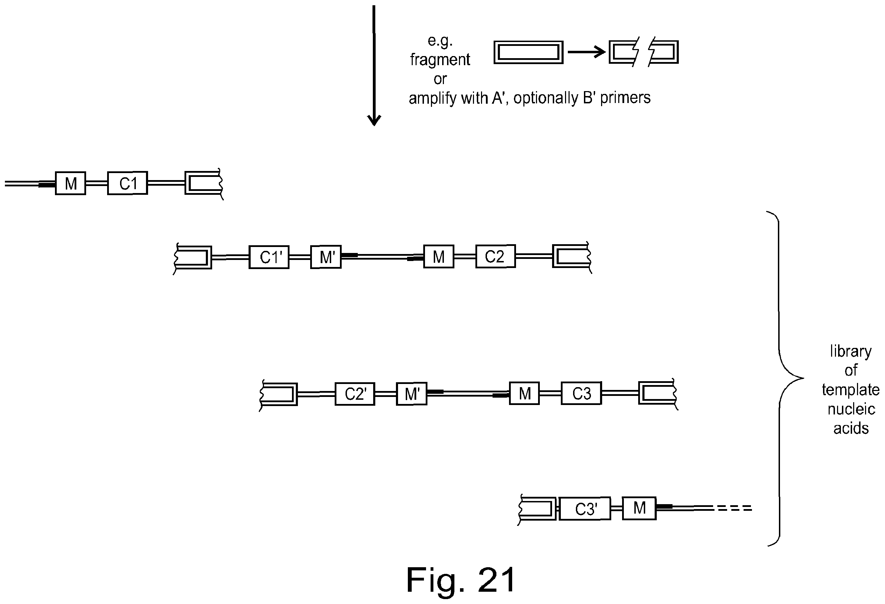

**FIG. 20** depicts an exemplary embodiment of a contacting a target nucleic acid with a library of transposons, which integrates into the target nucleic acid. **FIG. 21** depicts an optional step of fragmenting a linker into two parts, or an optional step of virtually fragmenting the linker by amplification using a primer A' and/or a primer B'. Fragmentation of the linker generates a library of template nucleic acids. In this figure, the filled-in region is indicated by a thick line.

In some embodiments, it can be advantageous for each template nucleic acid to incorporate at least one universal primer site. For example, a template nucleic acid can include first end sequences that comprise a first universal primer site, and second end sequences that comprise a second universal primer site. Universal primer sites can have various applications, such as amplifying, sequencing, and/or identifying one or more template nucleic acids. The first and second universal primer sites can be the same, substantially similar, similar, or different. In some embodiments, in order to prepare a template nucleic acid comprising a first universal primer site and a second universal primer site, a transposon sequence is prepared that includes a first transposase recognition site, a second transposase recognition site, a barcode disposed therebetween, wherein the barcode comprises a first barcode sequence and a second barcode sequence, separated by a linker. The linker includes a first primer site and a second primer site with a fragmentation site therebetween. The first primer site can comprise sequences that are the reverse complement of sequences within the second primer. In some embodiments, the first primer site comprises sequences having dyad symmetry to sequences within the second primer. In some embodiments, the first primer site comprises sequences having $C_2$ symmetry to sequences within the second primer. A plurality of transposons may be inserted into a target nucleic acid by transposition in the presence of a transposase. The incorporated sequences may be cleaved to yield a plurality of target nucleic acids, each comprising the first primer site and second primer site. In some embodiments, a first universal primer site and a second universal primer site can be incorporated into each template nucleic acid by a variety of methods. For example, template nucleic acids can be amplified using the first primer site and second primer site using tailed-oligonucleotides. As is understood in the art, a tailed-oligonucleotide can include sequences complementary to a primer site and additional sequences. In an example embodiment, a first tailed-oligonucleotide comprises sequences complementary to a first primer site and sequences for a first universal primer site, and a second tailed-oligonucleotide comprises sequences complementary to a second primer site and sequences for a second universal primer site. **FIG. 22** depicts an example embodiment series of rounds of extending template using tailed-oligonucleotides to obtain a population of template nucleic acids comprising template nucleic acids that include a first universal primer site and a second universal primer site. With respect to, in step 1, oligonucleotides comprising either a first universal primer sequence (A) or a second universal primer sequence (B) anneal to a primer site (P) of a template nucleic acid, and are extended. In step 2, the extension products provide templates for a further extension step. Alternatively, the extension products can be amplified with the use of additional oligonucleotides. The products of step 2 are depicted in step 3 and include nucleic acid sequences comprising first universal primer sites only, second universal primer sites only, or first and second universal primer sites. As will be understood, nucleic acid sequences comprising a first universal primer site and second universal primer site may be used in further sequencing methods.

It will be understood that in some embodiments, the vast number of available barcodes permits each template nucleic acid molecule to comprise a unique identification. Unique identification of each molecule in a mixture of template nucleic acids can be used in several applications to identify individual nucleic acid molecules, in samples having multiple chromosomes, genomes, cells, cell types, cell disease states, and species, for example in haplotype sequencing, parental allele discrimination, metagenomic sequencing, and sample sequencing of a genome.


**Tagging molecules individually**

The present invention also relates to methods for tagging molecules uniquely so individual molecules can be tracked and identified, so that bulk data can be deconvoluted back to the individual molecule. The ability to distinguish individual molecules and relate the information back to the molecule of origin is especially important when processes from original molecule to final product change the (stoichiometric) representation of the original population. For example, amplification leads to duplication (*e.g.*, PCR duplicates or biased amplification) that can skew the original representation. This can alter the methylation state call, copy number, allelic ratio due to non-uniform amplification and/or amplification bias. By identifying individual molecules, code-tagging distinguishes between identical molecules after processing. As such, duplications, and amplification bias can be filtered out, allowing accurate determination of the original representation of a molecule or population of molecules.

An advantage to this method is molecules that are otherwise identical in the original pool become uniquely identified by virtue of their tagging. In further downstream analysis these molecules can now be distinguished. This technique can be exploited in assay schemes in which amplification is employed. For example, amplification is known to distort the original representation of a mixed population of molecules. If unique tagging were not employed, the original representation (such as copy number or allelic ratio) would need to account for the biases (known or unknown) for each molecule in the representation. With unique tagging, the representation can accurately be determined by removing duplicates and counting the original representation of molecules, each having a unique tag. Thus, the cDNAs can be amplified and sequenced, without fear of bias, followed by filtering the data so that only authentic sequences or sequences of interest are selected for further analysis.

It is preferred to tag the original population in the early stages of the assay, although tagging can occur at later stages if the earlier steps do not introduce bias or are not important. In any of these applications, the complexity of the barcode sequences should be larger than the number of individual molecules to be tagged. This ensures that different target molecules receive different and unique tags. As such, a pool of random oligonucleotides of a certain length (5, 10, 20, 30, 40, 50, 100, 200) is desirable. Random pool of tags represents a large complexity of tags with code space $4^n$ where $n$ is the number of nucleotides. Additional codes (whether designed or random) can be incorporated at different stages to serve as a further check, such as a parity check for error correction.

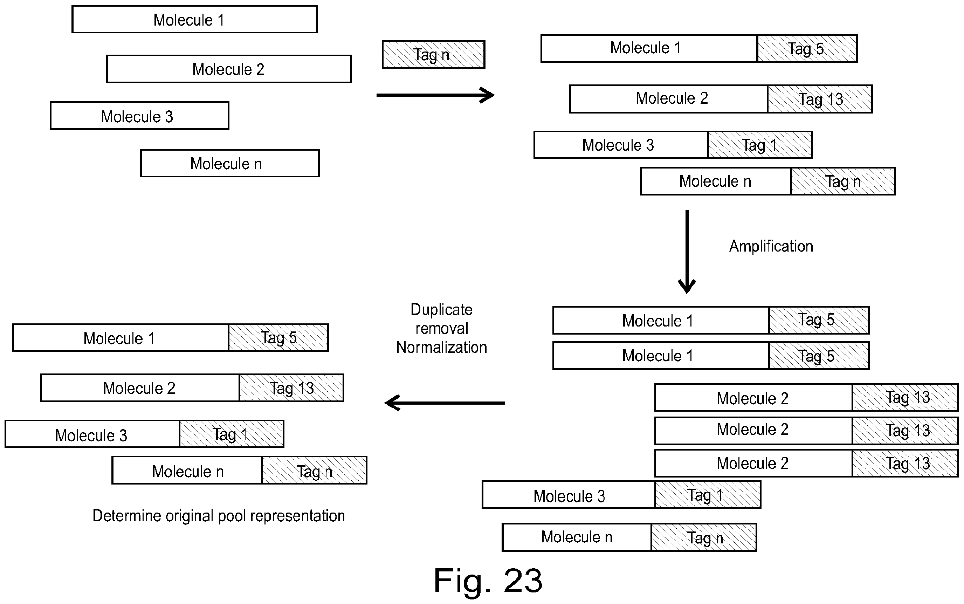

In one example:, individual molecules (such as target DNA) are attached to unique labels, such as unique oligo sequences, barcodes, as shown in **FIG. 23**. Attachment can occur through ligation, coupling chemistry, adsorption, etc. Other means include amplification (such as by PCR), copying (such as addition by a polymerase), and non-covalent interactions.

Specific methods include including barcodes (*e.g.*, designed or random sequences) to PCR primers so that the each template will receive an individual code within the code space, yielding unique amplicons that can be discriminated from other amplicons. This concept can be applied to any method that uses polymerase amplification, such as GoldenGate assays as disclosed in U.S. Patent No. 7,582,420, No. 7,955,794, and No. 8,003,354. Code-tagged target sequences can be circularized and amplified by methods such as rolling-circle amplification to yield code-tagged amplicons. Similarly, the code can also be added to RNA

**Methods for making DNA circles**

The present invention also relates to methods and compositions for making DNA circles by fragmenting and circularizing DNA at the same time by insertion of "hairpin-like" transposons. Previous methods of circularizing DNA are based on ligating "sticky" or blunt end ends of DNA fragments together or by the use of ligases or related enzymes to ligate 5' and 3' DNA fragments from the same template molecule.

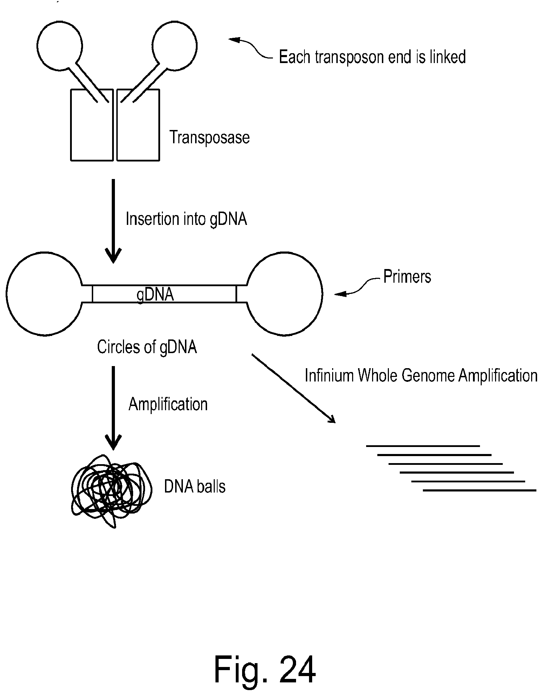

As described herein, special transposons are prepared in which DNA recognition sites for the transposase are double-stranded, with the overall transposon forming a hairpin. Two transposons are loaded into a transposon-transposase complex allowing the insertion of the hairpin transposon. Since the transposon ends are linked (as in **FIG. 24**, for example), insertion will fragment the DNA and simultaneously link the top and bottom strand of the target DNA. This process can occur throughout the genome, leading to the creation of many circles of the original starting DNA. A linker that links the top and bottom strand of the transposon together and can be any standard chemical linkers or a nucleic acid linker, such as single stranded DNA or RNA. Single-stranded DNA linkers are particularly useful since any DNA sequence can be designed as a linker, allowing pre-designed DNA to be incorporated into the DNA circles. The single-stranded linker can contain primer sites for amplification purposes.

Amplification of circularized DNA can be useful for sample preparation schemes for sequencing, genotyping, genotyping of FFPE samples. The method can be used for whole genome amplification with specific primers (not randomers) of genomic DNA. For example, primers can be designed into the single-stranded linker of the transposons allowing MDA or whole genome amplification of genomic DNA. One or more primers can be designed to accomplish exponential whole genome amplification. Alternatively, the method can be used to amplify a subset of genomic DNA. The circular genomic DNA can be amplified to prepare DNA balls. Whole genome amplification can be used for sample preparation for whole genome amplification, such as for the Infinium® assay workflow (Illumina, Inc.). In a particular example, the method can be used to amplify FFPE DNA for whole genome genotyping. Short FFPE (Formalin-Fixed Paraffin-Embedded) DNA does not amplify efficiently in whole genome amplification schemes (Klenow/random primers or MD-like approaches). This method can generate circular DNA and these circular templates are efficient substrates for amplification. The amplification can be combined with lesion-proofpolymerases to start the amplification.

A further advantage is that pre-designed primers can be used for amplification purposes. As such, whole genome amplification can be performed with one or few primers instead of the standard randomers. This method can lead to higher uniformity of amplification of a DNA sample with less locus dropout.

**Targeted insertion**

The method is particularly useful when tagmentation is target-specific, so that a desired subset of the genome is made into a sequencing library.

In one implementation, dsDNA is denatured into ssDNA and annealed with oligonucleotide probes (20-200 bases). These probes create sites of dsDNA that can be efficiently tagmented with the transposase-transposon complex.

This approach takes advantage of the fact that transposition into dsDNA is much more efficient compared to ssDNA targets. Furthermore, the transposase complex is covalently linked to the targeting oligonucleotides so that annealing brings the transposon complex into the vicinity of the targeted region of interest. At least two targeting oligos can be employed to generate library elements (tagments) that span the region between the targeting oligos (see **FIG. 25**).

The repeat regions may quickly re-hybridize, creating tagmentation sites that are non-specific and not desirable. To prevent this from occurring, a genomic representation using non-complementary bases can be prepared. For example, the DNA circles discussed above can be prepared that are uniformly amplified with specific primers, allowing the incorporation of non-complementary or pseudo-complementary bases. The end result is a genomic DNA representation that does not interact with itself or other amplified genomic DNA molecules. Specific annealing of standard oligonucleotides to these pseudo-complementary, single-stranded, genomic DNA can create specific tagmentation sites.

In another implementation (see **FIG. 26**), dsDNA is targeted directly using oligonucleotides (or LNA) probes coupled to the transposase complex. dsDNA can be targeted using a number of approaches including D-loop formation with recA-coated oligo probes, triplex formation, and other methods described in the literature. As above, two probes should flank the region of interest to generate a final library element spanning the region between the probes. Depending on the length of the linker between the targeting oligo and the transposase complex, the complex will insert a variable distance upstream or downstream of the targeted complex.

In yet another implementation regions of interest in dsDNA can be targeted using sequence-specific DNA binding proteins such as zinc-finger complexes, and other affinity ligands to specific DNA regions. Tagmentation on either end of a targeted region creates DNA fragments that have primers on the 5' and 3' end. These primers can be used for the amplification of a specific region.

**Methods for generating mate-pair libraries**

The present invention also relates to an improved method for preparing mate-pair libraries. Mate-pair libraries offer powerful means of mapping inserts and deletions in genomes as well as genome finishing and contig assembly. Current mate-pair library preparation methods can involve sequentially: a first fragmentation, size selection, end-repair, adapter ligation, circularization, a secondary fragmentation, junction fragment enrichment, and PCR. Though random fragmentation is desirable in general, the Poisson distribution of fragment sizes in the second fragmentation can yield numerous mate-pair arms that are too small to be of bioinformatic use. Such mate pairs must be discarded and this reduces the size of the mate-pair library.

The methods described herein can involve tethering the circularized intermediate to the surface of a particle to use the steric hindrance of the particle to reduce access of transposomes to the DNA, reducing transposase cleavage immediately adjacent to the site of immobilization.

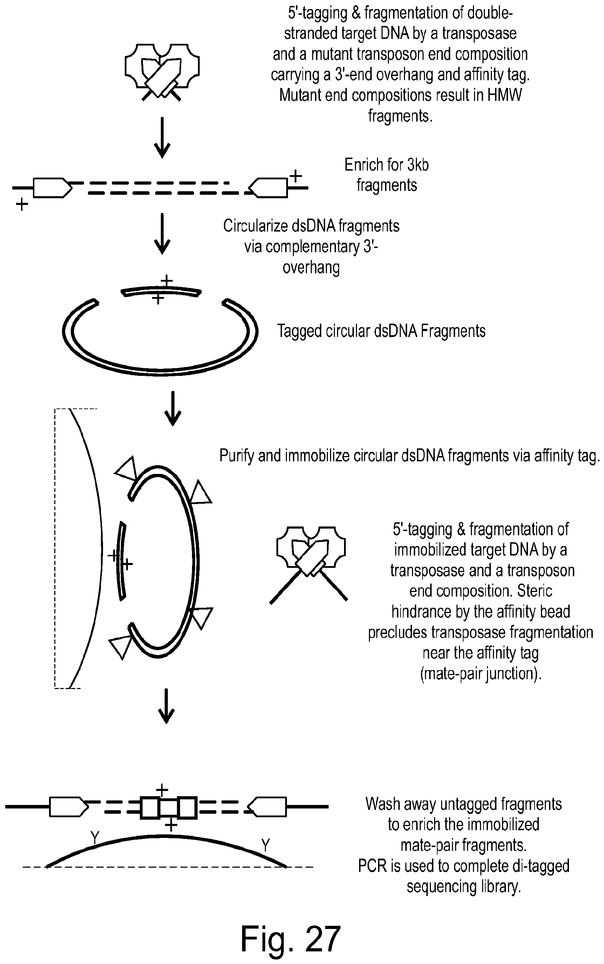

In the example shown in **FIG. 27**, the process begins by cleaving genomic DNA with transposomes and selecting fragments of the desired size by gel purification or other fragment selection methodology. The 3'-ends of the cleavage products are then melted away and replaced with an excess quantity of a new sequence that has been amended to contain a 3'-palindrome (for subsequent circularization) and a binding residue. An exemplary binding residue is a biotin residue for enrichment on streptavidin-paramagnetic particles (SA-PMPs). In this example, the newly modified fragments retain the 9-bp gaps left by the Tn5 transposase (or other enzyme that is used) and may be repaired in a gap-filling reaction driven by a non-strand displacing DNA polymerase that also lacks 3'-5' exonuclease activity, and a DNA ligase. The DNA ligase used in the gap-filling reaction can also serve to generate DNA circles. Following exonuclease-mediated removal of residual linear molecules, the circular DNAs can be captured onto the surface of a binding surface (*e.g.*, SA-PMPs) and subjected to a second tagmentation reaction. This tagmentation reaction introduces priming sites compatible with the next-generation sequencing platform of choice. In further examples, sequencing is performed.

Since the protective effect diminishes as the distance from a tether increases, the steric hindrance reduces the number of less-desirable small mate-pair arms results in mate-pair libraries of greater complexity due to a process that precludes losses from occurring during the second cleavage reaction.

**Methods of analyzing template nucleic acids**

Some embodiments include methods of analyzing template nucleic acids. Sequencing information can be obtained from a template nucleic acids and a sequence representation of the target nucleic acid can be obtained from such sequencing data.

In some embodiments, a linked read strategy may be used. A linked read strategy can include identifying sequencing data that links at least two sequencing reads. For example, a first sequencing read may contain a first marker, and a second sequencing read may contain a second marker. The first and second markers can identify the sequencing data from each sequencing read to be adjacent in a sequence representation of the target nucleic acid. In some embodiments, markers can comprise a first barcode sequence and a second barcode sequence in which the first barcode sequence can be paired with the second barcode sequence. In more embodiments, markers can comprise a first host tag and a second host tag. In more embodiments, markers can comprise a first barcode sequence with a first host tag, and a second barcode sequence with a second host tag.

An exemplary embodiment of a method for sequencing a template nucleic acid can comprise the following steps. First, sequence the first barcode sequence using a primer hybridizing to the first primer site as the sequencing primer; second, sequence the second barcode sequence using a primer hybridizing to the second primer site as the sequencing primer. The result is two sequence reads that help link the read to its genomic neighbors. Given long enough reads, and short enough library fragments, these two reads can be merged informatically to make one long read that covers the entire fragment. Using the barcode sequence reads and the 9 nucleotide duplicated sequence present from the insertion, reads can now be linked to their genomic neighbors to form much longer "linked reads" *in silico*. As will be understood, a library comprising template nucleic acids can include duplicate nucleic acid fragments. Sequencing duplicate nucleic acid fragments is advantageous in methods that include creating a consensus sequence for duplicate fragments. Such methods can increase the accuracy for providing a consensus sequence for a template nucleic acid and/or library of template nucleic acids.

In some embodiments, sequence analysis is performed in real time, for example, sequence data can be obtained and simultaneously analyzed. In some embodiments, a sequencing process to obtain sequencing data can be terminated at various points, including after at least a portion of a target nucleic acid sequence data is obtained or before the entire nucleic acid read is sequenced. Exemplary methods, systems, and further embodiments are provided in International Patent Publication WO 2010/062913.

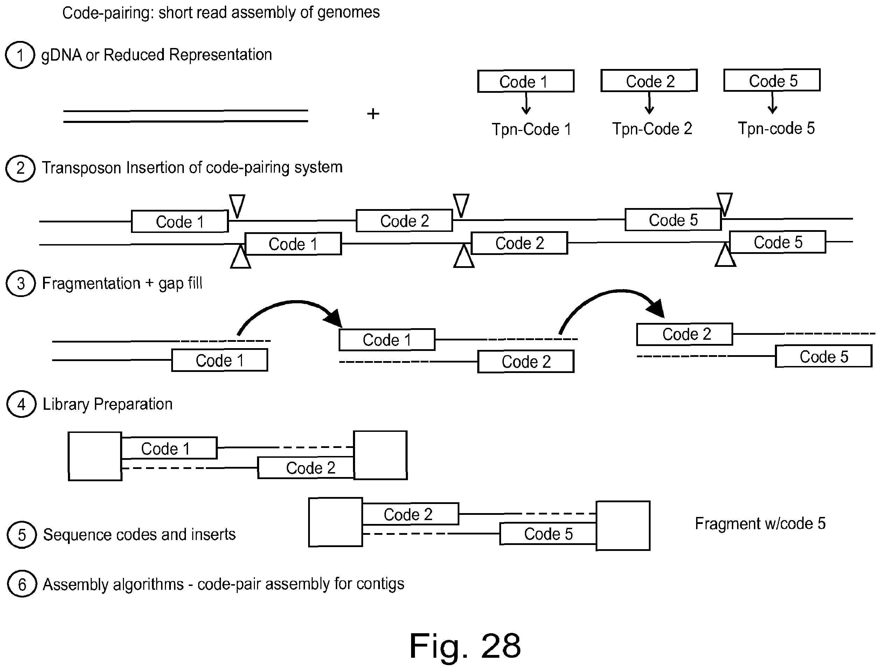

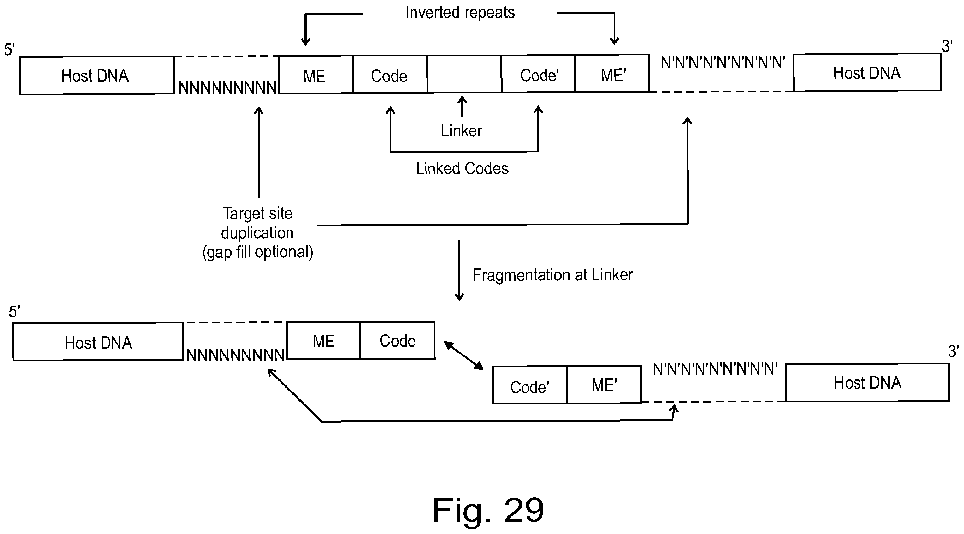

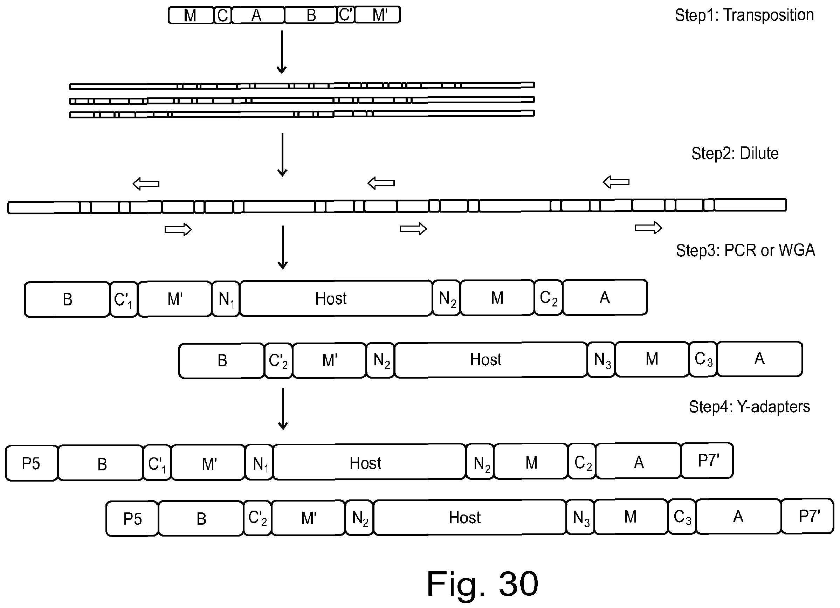

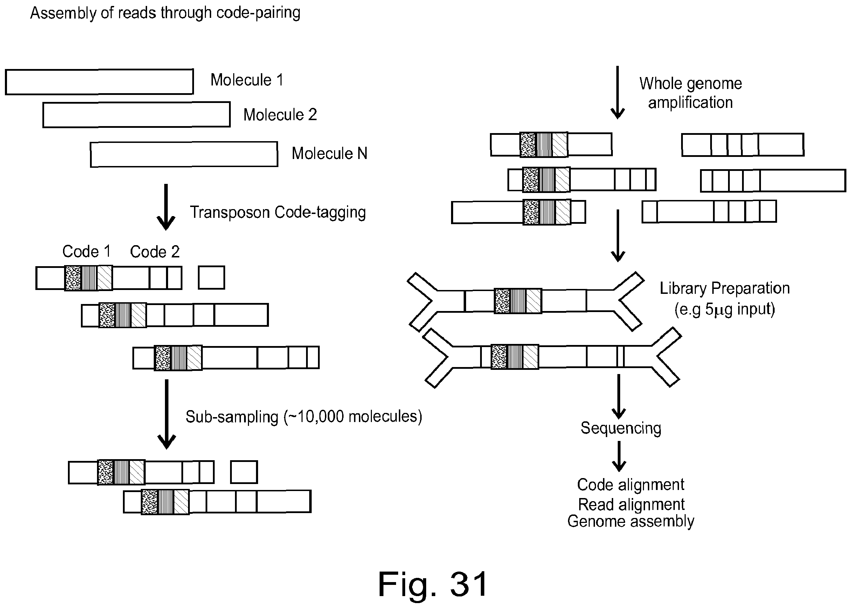

FIG. 28 depicts an exemplary embodiment of a method for assembling short sequencing reads using a linked read strategy. In such an exemplary embodiment, transposon sequences comprising barcodes are inserted into genomic DNA, a library is prepared and sequencing data obtained for the library of template nucleic acids. Blocks of templates are assembled by identifying paired barcodes and larger contigs are then assembled. FIG. 29, FIG. 30 and FIG. 31 depict exemplary embodiments of methods of assembling sequencing reads using a linked ready strategy.

Some embodiments include error detection and correction. Examples of errors can include errors in base calls during a sequencing process, and errors in assembling fragments into larger contigs. As would be understood, error detection can include detecting the presence or likelihood of errors in a data set, and as such detecting the location of an error or number of errors may not be required. For error correction, information regarding the location of an error and/or the number of errors in a data set is useful. Methods for error correction are well known in the art. Examples include the use of hamming distances, and the use of a checksum algorithm (*See, e.g.*, U.S. Publication 2010/0323348; U.S. Patent No. 7,574,305; U.S. Patent No. 6,654,696).

**- nested libraries**

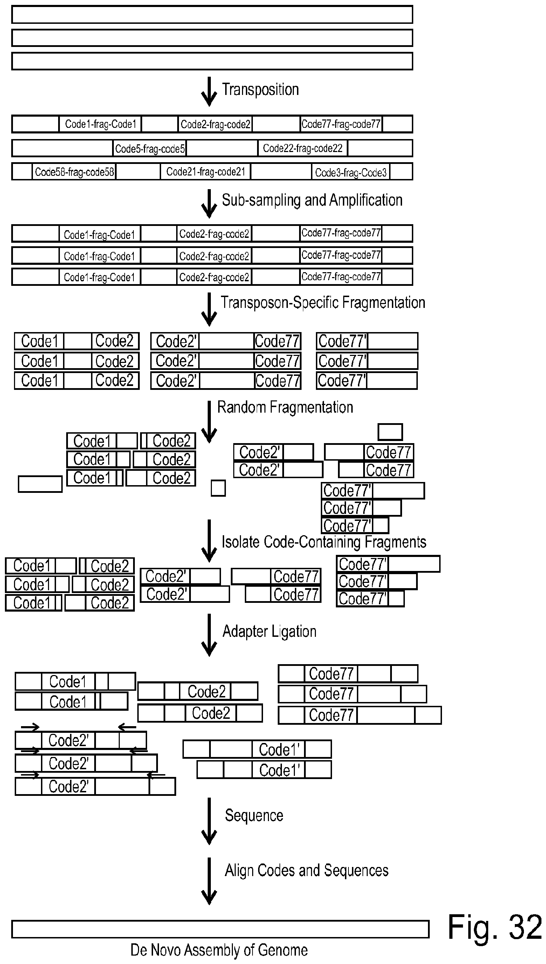

An alternative method involves the junction tagging methods above and preparation of nested sequencing libraries, as illustrated in FIG. 32. The nested sub-libraries are created from code-tagged DNA fragments. This can allow less frequent transposon tagging across the genome. It can also create a larger diversity of (nested) sequencing reads. These factors can lead to improved coverage and accuracy.

Sub-sampling and whole genome amplification can create many copies of a certain population of starting molecules. DNA fragments are then generated by transposon-specific fragmentation, where each fragment receives a code that allows one to link the fragment back to the original neighbor having a matching code (whether identical, complementary or otherwise informatically linked). The tagged fragments are fragmented at least a second time by methods random or sequence-specific methods such as enzymatic digestion, random shearing, transposon-based shearing or other methods, thereby creating sub-libraries of the code-tagged DNA fragments. In a useful variation, code-tagged fragments can be preferentially isolated by using transposons that contain a biotin or other affinity functionality for downstream enrichment purposes. Subsequently, library preparation converts the nested DNA fragments into sequencing templates. Paired-end sequencing results in sequencing the of the code-tag of the DNA fragments and of the target DNA. Since nested libraries for the same code-tag are created, long DNA fragments can be sequenced with short reads.

**- two-step sub-assembly**

In another embodiment, a two-step tagging method is provided using one or more transposition reactions with different transposomes. The transposomes can be of different types (e.g. Mu versus Tn5) or be two sets of Tn5 transposomes with different sequences, for example.

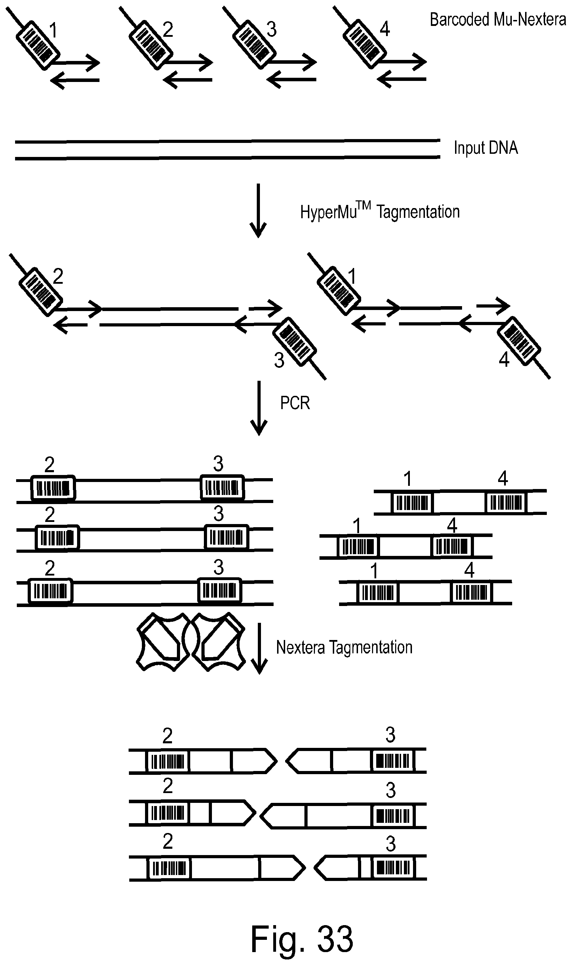

As exemplified in FIG. 33, a first transposome can use a Mu-type transposase, such as HyperMu™ (Epicentre). The DNA section of this complex is barcoded at the individual molecule level and presence of a molecular barcode at both ends of each fragment ("end-tags"). Each DNA fragment receives a unique tag (code-tagging) for the purpose of making each DNA fragment unique. The library is then amplified via PCR, so that every fragment is represented multiple times in the amplified reaction. The amplified library is next subjected to a second round of tagmentation with a different transposome, such as Tn5. If desired, the concentration of enzyme can be limited so every molecule is minimally targeted with the second transposome to preserve maximum continuity information. In addition, complexity reduction schemes at various stages throughout the assay can be utilized to ensure that the majority of libraries sequenced cover a significant fraction of the target DNA.

**- compartmentalization of single molecules**

Some embodiments include the use of the compositions and methods provided herein in single cell applications. In some embodiments, a transposon sequence can be associated with a bead via a cleavable linker. A bead comprising the transposon sequence and transposase is provided in a droplet. The cell is lysed and the transposon sequence cleaved from the bead. The transposition reaction is initiated. Such methods can be used to provide a library of template nucleic acids for each cell in population. Methods particularly useful for single-cell applications include clonal transposon beads, transposase mix, and single cell/single DNA molecule suspensions.

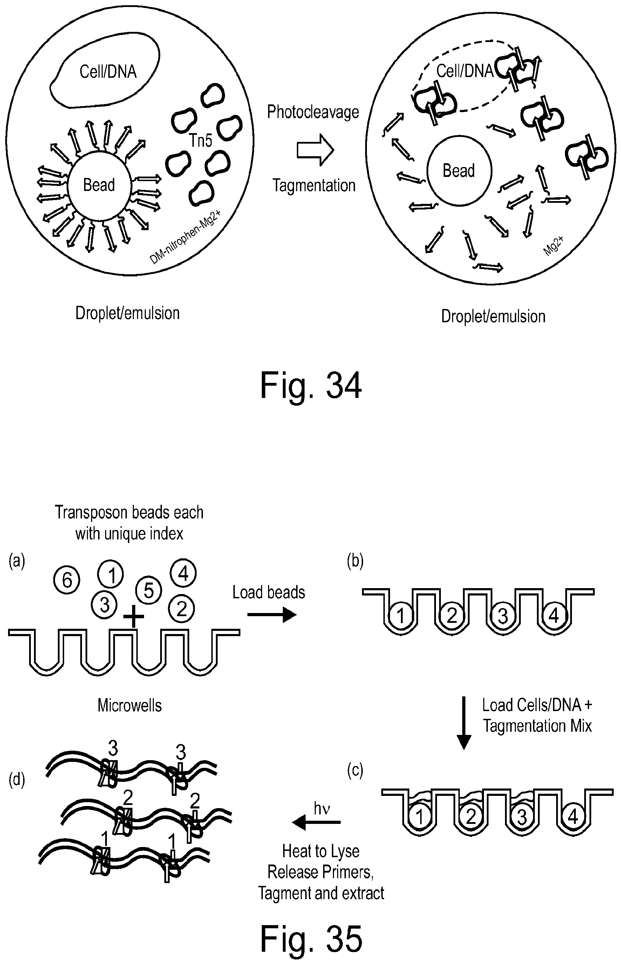

In an embodiment illustrated in **FIG. 34**, droplets or microwells are used to compartmentalize a single transposon bead with a single cell/DNA molecule in the presence of tagmentation reaction mix. The transposon is immobilized to the bead through a cleavable linker, such as a photocleavable linker. Each bead has a unique transposon index sequence for the appropriate barcoding of a particular cell. Additionally, photo-caged $Mg^{++}$ can be used to activate the tagmentation reaction at the same time the oligos are cleaved from the bead. Upon exposure to an appropriate wavelength of light (*e.g.*, 365 nm for many photoreactive compounds), the transposon is cleaved from the bead and assembles with the transposase to create an active complex. In the presence of $Mg^{++}$, this complex will insert into the available target DNA and fragment the sample. The DNA sample now contains a unique index along with adapter sequences from the transposon. All the compartmentalized reactions are simultaneously lysed/extracted into a single eluant. This eluant can be amplified with adapter-primers to create final sequencing library. For methylation analysis, the eluant can be treated with bisulfite first, and then amplified to enable bisulfite sequence analysis.

In an embodiment exemplified in **FIG. 35**, beads with uniquely indexed transposons (attached or hybridized via identifier sequences) are loaded into a microwell to create a transposon bead array. Accordingly, the present invention provides a transposon bead array. The transposon beads can be created in a number of ways. One way is clonal amplification of single-transposon seed molecules using bridge amplification of emulsion PCR. Another way is split-and-pool oligonucleotide synthesis in which the index portion of the transposon on the bead is generated during a split-and-pool oligonucleotide bead synthesis. A further way is to hybridize indexed transposons with an address sequence to beads containing the cognate address sequence.

Alternatively, the transposon bead array can be preloaded with beads having index sequences and then hybridizing indexed transposons. The transposon bead array is loaded with tagmentation mix and cells/DNA so that each well has less than one cell on average. The transposon bead array can be overlaid with oil and heated to lyse the cells. The transposons are released from the bead via photocleavage. Alternatively, photo-caged $Mg^{++}$ can be released to activate the tagmentation reaction. After tagmentation, the wells are extracted and the eluate is pooled. The eluate can be amplified (such as by PCR) to yield a cell-indexed sequencing library.

**Sequencing methods**

The methods described herein can be used in conjunction with a variety of sequencing techniques. In some embodiments, the process to determine the nucleotide sequence of a target nucleic acid can be an automated process.

Some embodiments include pyrosequencing techniques. Pyrosequencing detects the release of inorganic pyrophosphate ($PP_i$) as particular nucleotides are incorporated into the nascent strand (Ronaghi et al., "Real-time DNA sequencing using detection of pyrophosphate release." Analytical Biochemistry 242(1): 84-9 (1996); Ronaghi, M. "Pyrosequencing sheds light on DNA sequencing" Genome Res. 11(1):3-11 (2001); Ronaghi et al., "A sequencing method based on real-time pyrophosphate." Science 281(5375):363 (1998); U.S. Patent No. 6,210,891; U.S. Patent No. 6,258,568 and U.S. Patent No. 6,274,320). In pyrosequencing, released $PP_i$ can be detected by being immediately converted to adenosine triphosphate (ATP) by ATP sulfurylase, and the level of ATP generated is detected via luciferase-produced photons.

In another example type of SBS, cycle sequencing is accomplished by stepwise addition of reversible terminator nucleotides containing, for example, a cleavable or photobleachable dye label as described, for example, in U.S. Patent No. 7,427,67, U.S. Patent No. 7,414,1163 and U.S. Patent No. 7,057,026. This approach is being commercialized by Solexa (now Illumina Inc.), and is also described in WO 91/06678 and WO 07/123744 (filed in the United States patent and trademark Office as US 12/295,337). The availability of fluorescently-labeled terminators in which both the termination can be reversed and the fluorescent label cleaved facilitates efficient cyclic reversible termination (CRT) sequencing.

Polymerases can also be co-engineered to efficiently incorporate and extend from these modified nucleotides.

Additional example SBS systems and methods which can be utilized with the methods and systems described herein are described in U.S. Patent Application Publication No. 2007/0166705, U.S. Patent Application Publication No. 2006/0188901, U.S. Patent No. 7057026, U.S. Patent Application Publication No. 2006/0240439, U.S. Patent Application Publication No. 2006/0281109, PCT Publication No. WO 05/065814, U.S. Patent Application Publication No. 2005/0100900, PCT Publication No. WO 06/064199 and PCT Publication No. WO 07/010251.

Some embodiments can utilize sequencing by ligation techniques. Such techniques utilize DNA ligase to incorporate nucleotides and identify the incorporation of such nucleotides. Example SBS systems and methods which can be utilized with the methods and systems described herein are described in U.S. Patent No 6,969,488, U.S. Patent No. 6,172,218, and U.S. Patent No. 6,306,597.

Some embodiments can include techniques such as next-next technologies. One example can include nanopore sequencing techniques (Deamer, D.W. & Akeson, M. "Nanopores and nucleic acids: prospects for ultrarapid sequencing." Trends Biotechnol. 18, 147-151 (2000); Deamer, D. and D. Branton, "Characterization of nucleic acids by nanopore analysis". Acc. Chem. Res. 35:817-825 (2002); Li et al., "DNA molecules and configurations in a solid-state nanopore microscope" Nat. Mater. 2:611-615 (2003)). In such embodiments, the target nucleic acid passes through a nanopore. The nanopore can be a synthetic pore or biological membrane protein, such as $\alpha$-hemolysin. As the target nucleic acid passes through the nanopore, each base-pair can be identified by measuring fluctuations in the electrical conductance of the pore. (U.S. Patent No. 7,001,792; Soni & Meller, "A. Progress toward ultrafast DNA sequencing using solid-state nanopores." Clin. Chem. 53, 1996-2001 (2007); Healy, K. "Nanopore-based single-molecule DNA analysis." Nanomed. 2:459-481 (2007); Cockroft et al., "A single-molecule nanopore device detects DNA polymerase activity with single-nucleotide resolution." J. Am. Chem. Soc. 130:818-820 (2008)). In some such embodiments, nanopore sequencing techniques can be useful to confirm sequence information generated by the methods described herein.

Some embodiments can utilize methods involving the real-time monitoring of DNA polymerase activity. Nucleotide incorporations can be detected through fluorescence resonance energy transfer (FRET) interactions between a fluorophore-bearing polymerase and $\gamma$-phosphate-labeled nucleotides as described, for example, in U.S. Patent No. 7,329,492 and U.S. Patent No. 7,211,414 or nucleotide incorporations can be detected with zero-mode waveguides as described, for example, in U.S. Patent No. 7,315,019 and using fluorescent nucleotide analogs and engineered polymerases as described, for example, in U.S. Patent No. 7,405,281 and U.S. Patent Application Publication No. 2008/0108082 The illumination can be restricted to a zeptoliter-scale volume around a surface-tethered polymerase such that incorporation of fluorescently labeled nucleotides can be observed with low background (Levene, M.J. et al. "Zero-mode waveguides for single-molecule analysis at high concentrations." Science 299, 682-686 (2003); Lundquist, P.M. et al. "Parallel confocal detection of single molecules in real time." Opt. Lett. 33, 1026-1028 (2008); Korlach, J. et al. "Selective aluminum passivation for targeted immobilization of single DNA polymerase molecules in zero-mode waveguide nanostructures." Proc. Natl. Acad. Sci. USA 105, 1176-1181 (2008)). In one example single molecule, real-time (SMRT) DNA sequencing technology provided by Pacific Biosciences Inc can be utilized with the methods described herein. In some embodiments, a SMRT chip or the like may be utilized (U.S. Patent Nos. 7,181,122, 7,302,146, 7,313,308). A SMRT chip comprises a plurality of zero-mode waveguides (ZMW). Each ZMW comprises a cylindrical hole tens of nanometers in diameter perforating a thin metal film supported by a transparent substrate. When the ZMW is illuminated through the transparent substrate, attenuated light may penetrate the lower 20-30 nm of each ZMW creating a detection volume of about $1 \times 10^{-21}$ L. Smaller detection volumes increase the sensitivity of detecting fluorescent signals by reducing the amount of background that can be observed.

SMRT chips and similar technology can be used in association with nucleotide monomers fluorescently labeled on the terminal phosphate of the nucleotide (Korlach J. et al., "Long, processive enzymatic DNA synthesis using 100% dye-labeled terminal phosphate-linked nucleotides." Nucleosides, Nucleotides and Nucleic Acids, 27:1072-1083, 2008). The label is cleaved from the nucleotide monomer on incorporation of the nucleotide into the polynucleotide. Accordingly, the label is not incorporated into the polynucleotide, increasing the signal: background ratio. Moreover, the need for conditions to cleave a label from labeled nucleotide monomers is reduced.

An additional example of a sequencing platform that may be used in association with some of the embodiments described herein is provided by Helicos Biosciences Corp. In some embodiments, TRUE SINGLE MOLECULE SEQUENCING can be utilized (Harris T.D. et al., "Single Molecule DNA Sequencing of a viral Genome" Science 320:106-109 (2008)). In one embodiment, a library of target nucleic acids can be prepared by the addition of a 3' poly(A) tail to each target nucleic acid. The poly(A) tail hybridizes to poly(T) oligonucleotides anchored on a glass cover slip. The poly(T) oligonucleotide can be used as a primer for the extension of a polynucleotide complementary to the target nucleic acid. In one embodiment, fluorescently-labeled nucleotide monomer, namely, A, C, G, or T, are delivered one at a time to the target nucleic acid in the presence DNA polymerase. Incorporation of a labeled nucleotide into the polynucleotide complementary to the target nucleic acid is detected, and the position of the fluorescent signal on the glass cover slip indicates the molecule that has been extended. The fluorescent label is removed before the next nucleotide is added to continue the sequencing cycle. Tracking nucleotide incorporation in each polynucleotide strand can provide sequence information for each individual target nucleic acid.

An additional example of a sequencing platform that can be used in association with the methods described herein is provided by Complete Genomics Inc. Libraries of target nucleic acids can be prepared where target nucleic acid sequences are interspersed approximately every 20 bp with adaptor sequences. The target nucleic acids can be amplified using rolling circle replication, and the amplified target nucleic acids can be used to prepare an array of target nucleic acids. Methods of sequencing such arrays include sequencing by ligation, in particular, sequencing by combinatorial probe-anchor ligation (cPAL).

In some embodiments using cPAL, about 10 contiguous bases adjacent to an adaptor may be determined. A pool of probes that includes four distinct labels for each base (A, C, T, G) is used to read the positions adjacent to each adaptor. A separate pool is used to read each position. A pool of probes and an anchor specific to a particular adaptor is delivered to the target nucleic acid in the presence of ligase. The anchor hybridizes to the adaptor, and a probe hybridizes to the target nucleic acid adjacent to the adaptor. The anchor and probe are ligated to one another. The hybridization is detected and the anchorprobe complex is removed. A different anchor and pool of probes is delivered to the target nucleic acid in the presence of ligase.

The sequencing methods described herein can be advantageously carried out in multiplex formats such that multiple different target nucleic acids are manipulated simultaneously. In particular embodiments, different target nucleic acids can be treated in a common reaction vessel or on a surface of a particular substrate. This allows convenient delivery of sequencing reagents, removal of unreacted reagents and detection of incorporation events in a multiplex manner. In embodiments using surface-bound target nucleic acids, the target nucleic acids can be in an array format. In an array format, the target nucleic acids can be typically coupled to a surface in a spatially distinguishable manner. For example, the target nucleic acids can be bound by direct covalent attachment, attachment to a bead or other particle or associated with a polymerase or other molecule that is attached to the surface. The array can include a single copy of a target nucleic acid at each site (also referred to as a feature) or multiple copies having the same sequence can be present at each site or feature. Multiple copies can be produced by amplification methods such as, bridge amplification or emulsion PCR as described in further detail herein.

The methods set forth herein can use arrays having features at any of a variety of densities including, for example, at least about 10 features/cm$^2$, 100 features/cm$^2$, 500 features/cm$^2$, 1,000 features/cm$^2$, 5,000 features/cm$^2$, 10,000 features/cm$^2$, 50,000 features/cm$^2$, 100,000 features/cm$^2$, 1,000,000 features/cm$^2$, 5,000,000 features/cm$^2$, $10^7$ features/cm$^2$, 5x $10^7$ features/cm$^2$, $10^8$ features/cm$^2$, 5x $10^8$ features/cm$^2$, $10^9$ features/cm$^2$, 5x $10^9$ features/cm$^2$, or higher.

### Surfaces

In some embodiments, the nucleic acid template provided herein can be attached to a solid support ("substrate"). Substrates can be two-or three-dimensional and can comprise a planar surface (*e.g.*, a glass slide) or can be shaped. A substrate can include glass (*e.g.*, controlled pore glass (CPG)), quartz, plastic (such as polystyrene (low cross-linked and high cross-linked polystyrene), polycarbonate, polypropylene and poly(methylmethacrylate)), acrylic copolymer, polyamide, silicon, metal (*e.g.*, alkanethiolate-derivatized gold), cellulose, nylon, latex, dextran, gel matrix (*e.g.*, silica gel), polyacrolein, or composites.

Suitable three-dimensional substrates include, for example, spheres, microparticles, beads, membranes, slides, plates, micromachined chips, tubes (*e.g.*, capillary tubes), microwells, microfluidic devices, channels, filters, or any other structure suitable for anchoring a nucleic acid. Substrates can include planar arrays or matrices capable of having regions that include populations of template nucleic acids or primers. Examples include nucleoside-derivatized CPG and polystyrene slides; derivatized magnetic slides; polystyrene grafted with polyethylene glycol, and the like.

Various methods can be used to attach, anchor or immobilize nucleic acids to the surface of the substrate. The immobilization can be achieved through direct or indirect bonding to the surface. The bonding can be by covalent linkage. See Joos et al. (1997) Analytical Biochemistry, 247:96-101; Oroskar et al. (1996) Clin. Chem., 42: 1547-1555; and Khandjian (1986) Mol. Bio. Rep., 11 : 107-11. A preferred attachment is direct amine bonding of a terminal nucleotide of the template or the primer to an epoxide integrated on the surface. The bonding also can be through non-covalent linkage. For example, biotin- streptavidin (Taylor et al. (1991) J. Phys. D: Appl. Phys., 24: 1443,) and digoxigenin with anti-digoxigenin (Smith et al, Science, 253: 1122 (1992)), are common tools for anchoring nucleic acids to surfaces and parallels. Alternatively, the attachment can be achieved by anchoring a hydrophobic chain into a lipid monolayer or bilayer. Other methods known in the art for attaching nucleic acid molecules to substrates can also be used.

The following Examples provide illustrative embodiments of the inventions provided herein.

EXAMPLES

**Example 1 - Whole genome amplification using transposon sequences**

This example illustrates a method for uniform amplification of genomic DNA with random insertion therein of specific primer sites. Transposon sequences are prepared, each comprising a first transposase recognition site, a second transposase recognition site having a sequencing adaptor disposed therebetween, in which the sequencing adaptor comprises a first primer site and second primer site. The transposon sequences are contacted with genomic DNA in the presence of MuA transposase under conditions sufficient for the transposon sequences to integrate into the genomic DNA. The genomic DNA is amplified using primers that hybridize to the first primer site or second primer site.

**Example 2-Landmark sequencing methods using genomes with increased complexity**

This example illustrates an embodiment for providing additional markers in a genome. Additional markers can be useful in genomes that include repetitive sequences during subsequent assembly steps to generate a sequence representation of the genome. Transposon sequences are prepared, each comprising a different barcode. The transposon sequences are integrated into genomic DNA in a transposition reaction. The genomic DNA comprising the integrated transposon is amplified by whole genome amplification. A sequencing library is prepared from the amplified template nucleic acids. Sequencing data is obtained from the sequencing library. Sequencing reads can include representations of one or more nucleic acids with the same barcode on each nucleic acid. Such nucleic acids are identified as containing sequences that overlap in a sequence representation of the genomic DNA. The sequencing reads can be assembled by identifying barcodes on overlapping sequences.

**Example 3-Predicted average coverage using linked read sequencing strategy**

Useable fragment lengths are modeled as a truncated exponential distribution so that the mean useable fragment length can be obtained by setting k = b/d, where d is the mean of the non-truncated exponential (the total fragment distribution) and b is the value for truncation (either 180 or 280 for 100 nucleotides and 150 nucleotide paired-end reads, respectively) and then calculating the mean of the truncated exponential as

$$E(f) = d \, (1-(k+1)e^{-k}) \, / \, (1 - e^{-k})$$

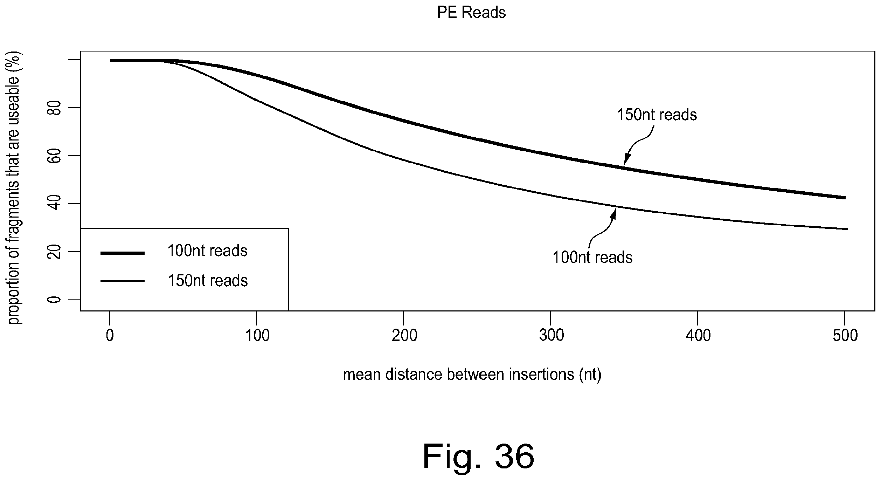

The proportion of useable reads is p = C(b) x (1 - D(0,T)) where C is the exponential cumulative distribution function, T is the average repetitions of observing each fragment (num clusters) / complexity, complexity is the genome size times the number of genome copies diluted to divided by d, and D is the Poisson cumulative distribution function
Expected length of linked read is then (E(f) - 9) x 1 / (1 - p) + 9 where p is proportion of useable reads: 9 is subtracted from each read because of the reused 9 nucleotides sequence in neighboring fragments; and 9 nucleotides is added back to the linked read to account for the end read -one of its 9 nucleotides segments is not shared with another read within the linked read.
The distribution of linked read lengths is also exponential with the above expected value. Very long linked reads can be observed, and shown in **FIG. 36.**
Table 1 sets out predicted average coverage for a bacterial genome, human PCR product, and a single copy of a human diploid genome using a linked read strategy.

TABLE 1

| Target nucleic acid | Genome size | Read length | Diluted copies | Number of clusters | Average insert distance | Average coverage | Mean linked read length |
|---|---|---|---|---|---|---|---|
| Bacterial genome | 5.0e6 | 150 | 10 | 35,000,000 | 50 nt | 9.96 X | 10,815 nt |
| Human PCR product | 20 kb | 150 | 10,000 | 40,000,000 | 50 nt | 9.96 X | 10,811 nt |
| Single cell, human diploid genome | 3e9 | 150 | 2 | 3000,000,000 | 50 nt | 9.66 X | 1,191 nt |

**Example 4-*De novo* sequencing target DNA**

This example illustrates an embodiment of assembling sequencing data obtained from a library of template nucleic acids prepared from a target DNA.

A plurality of transposon sequences are integrated into the target DNA in a transposition reaction. Each transposon sequence includes a barcode which comprises a first barcode sequence and a second barcode sequence. The first barcode sequence is the reverse complement of the second barcode sequence. There are more than $10^{18}$ different barcodes in the plurality of transposon sequences, such that the code space is large and out-competes the complexity of the target DNA. Accordingly, each integrated barcode is likely to be unique. Optionally, the target DNA comprising the integrated transposon sequences is sub-sampled. The sub-sampled target DNA comprising the integrated transposon sequences is amplified by methods of whole genome amplification. A sequencing library is prepared from the amplified nucleic acids. Sequencing data is obtained from the sequencing library. The sequencing data comprises sequencing reads for each amplified nucleic acid.

Sequencing reads that include a barcode sequence that can be paired with a barcode sequence of another sequencing read are aligned. Shorter alignments of sequencing reads are aligned with other short alignments by identifying paired barcode sequences to produce longer alignments. A sequence representation of the target DNA is generated.

**Example 5-Preparing a linked library with blunt-end barcodes**

This example illustrates an embodiment of preparing a linked library that includes identical barcodes on template nucleic acids that include adjacent sequences in a sequence representation of the target nucleic acid.

Transposon sequences are prepared comprising a first fragmentation site and a second fragmentation site, having a barcode disposed therebetween. Each fragmentation site comprises a site that can be nicked to produce a single-stranded sticky end, *e.g.*, a restriction endonuclease site that produces a single-strand sticky end. The transposon sequences are integrated into the target DNA by a transposition reaction. Optionally, the target DNA comprising the integrated transposon sequences is sub-sampled. The sub-sampled target DNA comprising the integrated transposon sequences is amplified by methods of whole genome amplification. The amplified nucleic acids are fragmented at the first and second fragmentation sites at each integrated transposon sequence to generate nucleic acids comprising sticky ends. The sticky ends are filled-in so that each end comprises a barcode. Adaptors are ligated to the blunt ends of each nucleic acid. The nucleic acids are amplified using primer sites of the adaptors.

A sequencing library is prepared from the amplified nucleic acids. Sequencing data is obtained from the sequencing library. The sequencing data comprises sequencing reads for each amplified nucleic acid. Sequencing reads that include a barcode sequence that can be paired with a barcode sequence of another sequencing read are aligned. Shorter alignments of sequencing reads are aligned with other short alignments by identifying paired barcode sequences to produce longer alignments. A sequence representation of the target DNA is generated.

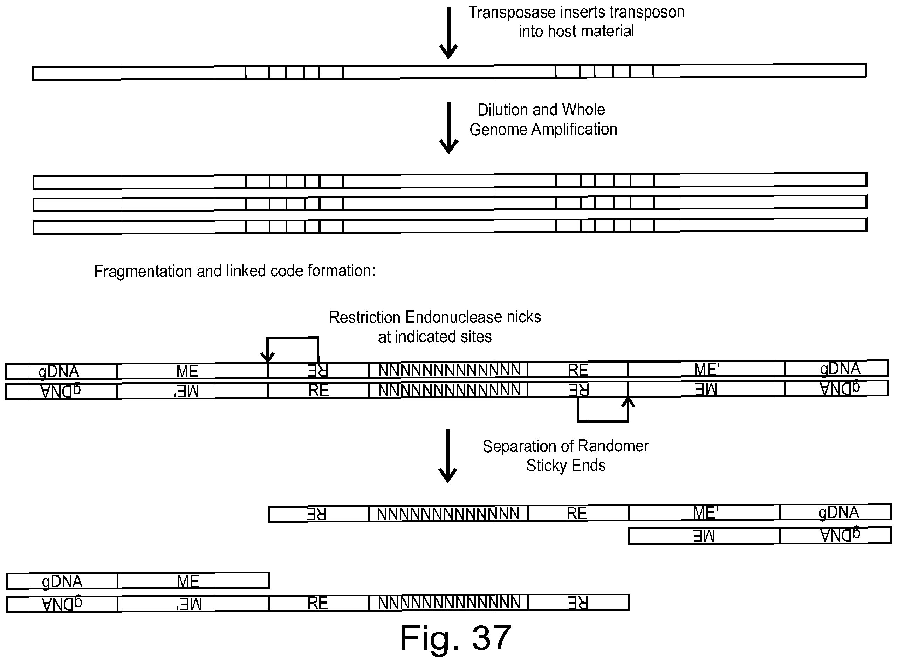

FIG. 37 depicts the insertion of the transposon sequence into the target nucleic acid (host material), dilution of the template nucleic acids, and subsequent whole genome amplification of the template nucleic acids. The amplified template nucleic acids are fragmented in the presence of a restriction endonuclease that nicks the transposon sequences at the first restriction endonuclease site and the second restriction endonuclease site to yield two sticky ends.

FIG. 15 depicts a fill-in reaction of the sticky ends, followed by A-tailing the ends and adding adaptors to the tailed ends. The adaptors can be used in subsequent amplification, library preparation, and methods to obtain sequence data. In other embodiments, the fill-in reaction itself can be used as part of a detection assay, such as by incorporating detectably labeled nucleotides in a variety of assay formats. Advantageously, methods that utilize a nicking endonuclease may be carried out with a relatively small number of target nucleic acids, or a relatively dilute concentration of the target nucleic acid.

The term "comprising" as used herein is synonymous with "including," "containing," or "characterized by," and is inclusive or open-ended and does not exclude additional, unrecited elements or method steps.

**Claims**

1.   An artificial transposon, comprising:

      a first transposase recognition site,
      a second transposase recognition site,
      a barcode disposed therebetween, wherein the barcode comprises a double-stranded nucleic acid sequence comprising

a first strand barcode and
a second strand barcode, and

a linker disposed between the first and the second barcode sequences,

wherein the linker comprises a first primer site, a second primer site and a non-amplifiable site disposed between the first and second primer sites.

2. The transposon of claim 1, wherein the transposase is a hyperactive Tn5 transposase.

3. The transposon of claim 1, wherein the transposase is a Mu transposase.

4. The transposon of claim 1, wherein the transposase is an IS5 or an IS911 transposase.

5. The transposon of claim 1, wherein the transposon is associated with an affinity tag.

6. The transposon of claim 5, wherein the affinity tag is associated with a protein that targets specific nucleic acid sequences.

7. The transposon of claim 1, wherein the first strand barcode and the second strand barcode comprise complementary sequences.

8. The transposon of claim 1, wherein the first strand barcode and the second strand barcode comprise non-complementary sequences.

9. An isolated template nucleic acid, comprising at least a portion of a target nucleic acid or copy thereof and at least first and second transposons of claim 1, wherein the barcode of the first transposon is different from the barcode of the second transposon.

10. A method for preparing a library of template nucleic acids comprising:

(a) contacting a target nucleic acid with a plurality of transposons of claim 1 under conditions such that a portion of said transposons are inserted into the target nucleic acid; and
(b) fragmenting said target nucleic acid.

11. The method of claim 10, wherein the target nucleic acid comprises cDNAs from a single cell.

12. The method of claim 10, wherein the target nucleic acid comprises nucleic acids from multiple species.

13. The method of claim 10, wherein the target nucleic acid comprises nucleic acids from multiple haplotypes.

14. The method of claim 10, wherein the fragmentation comprises contacting the target nucleic acid with a polymerase.

15. The method of claim 14, wherein the polymerase has 3' to 5' exonuclease activity.

16. A method for preparing a library of template nucleic acids, comprising:

(a) contacting a target nucleic acid with a plurality of transposons of claim 1, under conditions such that a portion of said transposons are inserted into the target nucleic acid;
(b) hybridizing a primer to the first primer site; and
(c) amplifying a portion of the transposon sequence.

17. A method of analyzing a target nucleic acid comprising:

(a) performing the method of claim 16;
(b) obtaining sequence data from said templates; and
(c) assembling a representation of a portion of said target nucleic acid from said sequence data.

**18.** The method of claim 17, wherein assembling step (c) comprises identifying the presence of a first barcode in one sequencing read and the presence of a corresponding barcode in another sequencing read, thereby indicating proximity between the two sequencing reads in the target nucleic acid.

**19.** The method of claim 17, wherein step (b) is terminated after the barcode is identified.

**Patentansprüche**

**1.** Künstliches Transposon, das Folgendes umfasst:

eine erste Transposaseerkennungsstelle,
eine zweite Transposaseerkennungsstelle,
einen dazwischen befindlichen Barcode, wobei der Barcode eine doppelsträngige Nukleinsäuresequenz umfasst, die Folgendes umfasst:

einen Barcode des ersten Strangs, und
einen Barcode des zweiten Strangs, und

einen Linker, der zwischen der ersten und der zweiten

Barcode-Sequenz angeordnet ist,
wobei der Linker eine erste Primer-Stelle, eine zweite Primer-Stelle und eine nicht amplifizierbare Stelle umfasst, die zwischen der ersten und der zweiten Primer-Stelle angeordnet ist.

**2.** Transposon nach Anspruch 1, wobei die Transposase eine hyperaktive Tn5-Transposase ist.

**3.** Transposon nach Anspruch 1, wobei die Transposase eine Mu-Transposase ist.

**4.** Transposon nach Anspruch 1, wobei die Transposase eine IS5- oder eine IS911-Transposase ist.

**5.** Transposon nach Anspruch 1, wobei das Transposon mit einem Affinitäts-Tag assoziiert ist.

**6.** Transposon nach Anspruch 5, wobei das Affinitäts-Tag mit einem Protein assoziiert ist, das auf spezifische Nukleinsäuresequenzen gerichtet ist.

**7.** Transposon nach Anspruch 1, wobei der Barcode des ersten Strangs und der Barcode des zweiten Strangs komplementäre Sequenzen umfassen.

**8.** Transposon nach Anspruch 1, wobei der Barcode des ersten Strangs und der Barcode des zweiten Strangs nicht-komplementäre Sequenzen umfassen.

**9.** Isolierte Template-Nukleinsäure, die wenigstens einen Teil einer Zielnukleinsäure oder eine Kopie davon und wenigstens ein erstes und zweites Transposon nach Anspruch 1 umfasst, wobei sich der Barcode des ersten Transposons vom Barcode des zweiten Transposons unterscheidet.

**10.** Verfahren zur Herstellung einer Bibliothek von Template-Nukleinsäuren, das Folgendes beinhaltet:

(a) Inkontaktbringen einer Zielnukleinsäure mit mehreren Transposonen nach Anspruch 1 unter solchen Bedingungen, dass ein Teil der genannten Transposone in die Zielnukleinsäure eingefügt wird; und
(b) Fragmentieren der genannten Zielnukleinsäure.

**11.** Verfahren nach Anspruch 10, wobei die Zielnukleinsäure cDNAs von einer einzigen Zelle umfasst.

**12.** Verfahren nach Anspruch 10, wobei die Zielnukleinsäure Nukleinsäuren von mehreren Spezies umfasst.

**13.** Verfahren nach Anspruch 10, wobei die Zielnukleinsäure Nukleinsäuren von mehreren Haplotypen umfasst.

**14.** Verfahren nach Anspruch 10, wobei die Fragmentierung das Inkontaktbringen der Zielnukleinsäure mit einer Polymerase umfasst.

**15.** Verfahren nach Anspruch 14, wobei die Polymerase 3' bis 5' Exonuklease-Aktivität hat.

**16.** Verfahren zur Herstellung einer Bibliothek von Template-Nukleinsäuren, das Folgendes beinhaltet:

(a) Inkontaktbringen einer Zielnukleinsäure mit mehreren Transposonen nach Anspruch 1 unter solchen Bedingungen, dass ein Teil der genannten Transposone in die Zielnukleinsäure eingefügt wird;
(b) Hybridisieren eines Primers mit der ersten Primer-Stelle; und
(c) Amplifizieren eines Abschnitts der Transposon-Sequenz.

**17.** Verfahren zum Analysieren einer Zielnukleinsäure, das Folgendes beinhaltet:

(a) Ausführen des Verfahrens nach Anspruch 16;
(b) Einholen von Sequenzdaten von den genannten Templates; und
(c) Assemblieren einer Repräsentation eines Abschnitts der genannten Zielnukleinsäure von den genannten Sequenzdaten.

**18.** Verfahren nach Anspruch 17, wobei der Assemblierungsschritt (c) das Identifizieren der Anwesenheit eines ersten Barcode in einem Sequenzierungslesestück und der Anwesenheit eines entsprechenden Barcode in einem anderen Sequenzierungslesestück beinhaltet, um dadurch Nähe zwischen den beiden Sequenzierungslesestücken in der Zielnukleinsäure anzuzeigen.

**19.** Verfahren nach Anspruch 17, wobei Schritt (b) nach dem Identifizieren des Barcode terminiert wird.

**Revendications**

**1.** Transposon artificiel, comprenant :

un premier site de reconnaissance de transposase,
un second site de reconnaissance de transposase,
un code barre disposé entre eux, le code barre comprenant une séquence d'acide nucléique double brin comprenant

un code barre de premier brin et
un code barre de second brin, et

un lieur disposé entre les première et seconde séquences de codes barres, le lieur comprenant un premier site d'amorce, un second site d'amorce et un site non amplifiable disposé entre les premier et second sites d'amorce.

**2.** Transposon selon la revendication 1, dans lequel la transposase est une transposase hyperactive Tn5.

**3.** Transposon selon la revendication 1, dans lequel la transposase est une transposase Mu.

**4.** Transposon selon la revendication 1, dans lequel la transposase est une transposase IS5 ou IS911.

**5.** Transposon selon la revendication 1, le transposon étant associé à un marqueur d'affinité.

**6.** Transposon selon la revendication 5, dans lequel le marqueur d'affinité est associé à une protéine qui cible des séquences d'acide nucléique spécifiques.

**7.** Transposon selon la revendication 1, dans lequel le code barre de premier brin et le code barre de second brin comprennent des séquences complémentaires.

**8.** Transposon selon la revendication 1, dans lequel le code barre de premier brin et le code barre de second brin comprennent des séquences non complémentaires.

9.  Acide nucléique matrice isolé, comprenant au moins une partie d'un acide nucléique cible ou une copie de celui-ci et au moins des premier et second transposons selon la revendication 1, dans lequel le code barre du premier transposon est différent du code barre du second transposon.

10. Procédé de préparation d'une bibliothèque d'acides nucléique matrices comprenant :

    (a) la mise en contact d'un acide nucléique cible avec une pluralité de transposons selon la revendication 1 dans des conditions telles qu'une partie desdits transposons soit insérée dans l'acide nucléique cible ; et
    (b) la fragmentation dudit acide nucléique cible.

11. Procédé selon la revendication 10, dans lequel l'acide nucléique cible comprend des ADNc d'une seule cellule.

12. Procédé selon la revendication 10, dans lequel l'acide nucléique cible comprend des acides nucléiques de multiples espèces.

13. Procédé selon la revendication 10, dans lequel l'acide nucléique cible comprend des acides nucléiques de multiples haplotypes.

14. Procédé selon la revendication 10, dans lequel la fragmentation comprend la mise en contact de l'acide nucléique cible avec une polymérase.

15. Procédé selon la revendication 14, dans lequel la polymérase a une activité exonucléase 3' → 5'.

16. Procédé de préparation d'une bibliothèque d'acides nucléiques matrices, comprenant :

    (a) la mise en contact d'un acide nucléique cible avec une pluralité de transposons selon la revendication 1 dans des conditions telles qu'une partie desdits transposons soit insérée dans l'acide nucléique cible ;
    (b) l'hybridation d'une amorce avec le premier site d'amorce ; et
    (c) l'amplification d'une partie de la séquence de transposon.

17. Procédé d'analyse d'un acide nucléique cible comprenant :

    (a) l'exécution du procédé selon la revendication 16 ;
    (b) l'obtention de données de séquence à partir desdites matrices ; et
    (c) l'assemblage d'une représentation d'une partie dudit acide nucléique cible provenant desdites données de séquence.

18. Procédé selon la revendication 17, dans lequel l'étape d'assemblage (c) comprend l'identification de la présence d'un premier code barre dans une lecture de séquençage et de la présence d'un code barre correspondant dans une autre lecture de séquençage, indiquant ainsi une proximité entre les deux lectures de séquençage dans l'acide nucléique cible.

19. Procédé selon la revendication 17, dans lequel l'étape (b) prend fin après l'identification du code barre.

transposon

barcode

| M | C1 | linker | C1' | M' |

first
transposase
recognition
sequence

first
barcode
sequence

second
barcode
sequence

second
transposase
recognition
sequence

Exemplary linkers:

| | N1 | | N2 | |

nickase
recognition
sites

A'

| | A | |

| | RE | |

restriction endonuclease
recognition sequence

non-amplifiable
site

A'

| | A | | nA | | B | |

B'

fragmentation sites

primer sites

# Fig. 1

Fig. 2

cME - UhpU -ME - code1 - P1 - frag - P1' - code2 - ME' - UhpU -cME'

UniCode

Fig. 3

EP 2 635 679 B1

Fig. 4

Transposon-Transposase Complex Formation

One transposon-two transposase complexes

Transposase

+

Fig. 5

Transposition of "single-stranded" Transposon

EP 2 635 679 B1

Fig. 6

Fig. 7

Fragmentation site

Transposon

Fragmentation of transposon

Transposase Units
"looped" Complexes 1

Code N

Code N

Transposition
into target DNA

Code N

Code N

Complexes 2

Fig. 8

target DNA

nick

g-code regions:

strand-displacing
amplification

library
for sequencing

# Fig. 9

Fig. 10

EP 2 635 679 B1

```
Make Transposons with bi-codes
            │
            ▼
Sequence bi-code combinations
            │
            ▼
Transposons Transposition
            │
            ▼
Make code-tagged DNA
Fragments
            │
            ▼
Make sequencing libraries
            │
            ▼
Sequence library
            │
            ▼
Create Key or Lookup Table
```

$A_i$ code                         $B_j$ code

ME-NNNNNNNNNN-P1-Fragmentation site-P1'-NNNNNNNNNN-ME

$B_j$ code

$A_i$ code

Mapped via in situ synthesis
or sequencing

[:::] Cleavage site

## Fig. 11

Fig. 12

Fig. 13

Fig. 14

Library Preparation:

Fig. 15

Step 1: synthesize randomer with hairpin

5' NNNNNNNNN
"code oligo" |||||||||
3'
Fragmentation site(s)

Step 2: extend and copy code

NNNNNNNN
|||||||||||||||||||
NNNNNNNN

Hairpin oligo allows copying of initial randomer sequence

Ensures randomer and copy are:
-Each others' reverse complement
-Linked on the same molecule

Step 3: A-tailing

5' NNNNNNNN
||||||||||||||||||
3' NNNNNNNN

Step 4: Adapter Ligation

5'
5' Primer 2 Adapter-1 3'
ME T NNNNNNNN
|||||||||||||||||
3'
ME A NNNNNNNN
5' Primer 1
Adapter-2
3'

ME's sites:
-recognition sequence to insert Transposon
-Reverse complement introduced using adapter stem sequence

Step 5: PCR amplification

| P2 | ME | Code | | | | Code' | ME | P1' |
|----|-----|------|---|---|---|-------|-----|-----|
| P2' | ME' | Code' | | | | Code | ME' | P1 |

# Fig. 16

= Fragmentation site

P4

Code Y

Code X

P3

P4'

ME

P3'

ME

Fig. 17

Fig. 18

ME'

PCR1:
Single Primer PCR to
increase template copy number

ME'

PCR2:
Introduce sequencing adaptors

"PCR suppression"

Fig. 19

Fig. 20

e.g.
fragment
or
amplify with A', optionally B' primers

library
of
template
nucleic
acids

M | C1

C1' | M' | M | C2

C2' | M' | M | C3

C3' | M

Fig. 21

Fig. 22

Amplification

Tag 5
Tag 13
Tag 1
Tag n

Molecule 1
Molecule 2
Molecule 3
Molecule n

Tag n

Molecule 1
Molecule 2
Molecule 3
Molecule n

Tag 5
Tag 5

Molecule 1
Molecule 1

Tag 13
Tag 13
Tag 13

Molecule 2
Molecule 2
Molecule 2

Tag 1
Tag n

Molecule 3
Molecule n

Duplicate
removal
Normalization

Tag 5
Tag 13
Tag 1
Tag n

Molecule 1
Molecule 2
Molecule 3
Molecule n

Determine original pool representation

Fig. 23

Fig. 24

Fig. 25

dsDNA

(1) Strand Invade/Triplex

(2a) Locally Tagment

(2b) Tagmentation fragments

ME

(4) Strand displace finish

ROI

Fig. 26

5'-tagging & fragmentation of double-stranded target DNA by a transposase and a mutant transposon end composition carrying a 3'-end overhang and affinity tag. Mutant end compositions result in HMW fragments.

Enrich for 3kb fragments

Circularize dsDNA fragments via complementary 3'-overhang

Tagged circular dsDNA Fragments

Purify and immobilize circular dsDNA fragments via affinity tag.

5'-tagging & fragmentation of immobilized target DNA by a transposase and a transposon end composition. Steric hindrance by the affinity bead precludes transposase fragmentation near the affinity tag (mate-pair junction).

Wash away untagged fragments to enrich the immobilized mate-pair fragments. PCR is used to complete di-tagged sequencing library.

Fig. 27

Code-pairing: short read assembly of genomes

Code 1 → Tpn-Code 1  Code 2 → Tpn-Code 2  Code 5 → Tpn-code 5

① gDNA or Reduced Representation

+

② Transposon Insertion of code-pairing system

③ Fragmentation + gap fill

④ Library Preparation

⑤ Sequence codes and inserts

⑥ Assembly algorithms - code-pair assembly for contigs

Fragment w/code 5

Fig. 28

Host DNA

N'N'N'N'N'N'N'N'

ME'

Code'

Linker

Code

ME

NNNNNNNN

Host DNA

5'

3'

Inverted repeats

Linked Codes

Target site
duplication
(gap fill optional)

Fragmentation at Linker

Host DNA

N'N'N'N'N'N'N'N'

Code'  ME'

Code  ME

NNNNNNNN

Host DNA

5'

3'

Fig. 29

Fig. 30

Assembly of reads through code-pairing

Fig. 31

EP 2 635 679 B1

Fig. 32

De Novo Assembly of Genome

Fig. 33

Fig. 34

Fig. 35

Fig. 36

Transposase inserts transposon
into host material

Dilution and Whole
Genome Amplification

Fragmentation and linked code formation:

Restriction Endonuclease nicks
at indicated sites

| gDNA | ME | | Ǝᴚ | NNNNNNNNNNNNN | RE | ME' | gDNA |
| ∀NⒺᵍ | ,ƎW | RE | NNNNNNNNNNNNN | Ǝᴚ | ƎW | ∀Nᗡᵍ |

Separation of Randomer
Sticky Ends

| | | Ǝᴚ | NNNNNNNNNNNNN | RE | ME' | gDNA |
| | | | | | ƎW | ∀Nᗡᵍ |

| gDNA | ME |
| ∀Nᗡᵍ | ,ƎW | RE | NNNNNNNNNNNNN | Ǝᴚ |

# Fig. 37

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010048605 A
- US 5644048 A
- US 5386023 A
- US 5637684 A
- US 5602240 A
- US 5216141 A
- US 4469863 A
- US 5235033 A
- US 5034506 A
- US 5432272 A
- WO 9523875 A
- US 20100022403 A
- US 20110014657 A
- US 7741463 B
- US 6544732 B
- US 7582420 B
- US 7955794 B
- US 8003354 B
- WO 2010062913 A
- US 20100323348 A
- US 7574305 B
- US 6654696 B
- US 6210891 B
- US 6258568 B
- US 6274320 B
- US 742767 A
- US 74141163 B
- US 7057026 B
- WO 9106678 A
- WO 07123744 A
- US 12295337 B
- US 20070166705 A
- US 20060188901 A
- US 20060240439 A
- US 20060281109 A
- WO 05065814 PCT
- US 20050100900 A
- WO 06064199 PCT
- WO 07010251 PCT
- US 6969488 B
- US 6172218 B
- US 6306597 B
- US 7001792 B
- US 7329492 B
- US 7211414 B
- US 7315019 B
- US 7405281 B
- US 20080108082 A
- US 7181122 B
- US 7302146 B
- US 7313308 B

**Non-patent literature cited in the description**

- **SYED F et al.** *Nat Methods,* 2009, vol. 6 (10), I-II
- **BIMBER B. et al.** *J Virol,* 2010, vol. 84 (22), 12087-12092
- **POBIGAYLO N. et al.** *Appl Environ Microb,* 2006, vol. 72 (6), 4329-4337
- **ZHOU M. et al.** *J Mol Biol,* 1998, vol. 276 (8), 913-925
- **BEAUCAGE et al.** *Tetrahedron,* 1993, vol. 49, 1925
- **LETSINGER.** *J. Org. Chem.,* 1970, vol. 35, 3800
- **SPRINZL et al.** *Eur. J. Biochem.,* 1977, vol. 81, 579
- **LETSINGER et al.** *Nucl. Acids Res.,* 1986, vol. 14, 3487
- **SAWAI et al.** *Chem. Lett.,* 1984, vol. 805
- **LETSINGER et al.** *J. Am. Chem. Soc.,* 1988, vol. 110, 4470
- **PAUWELS et al.** *Chemica Scripta,* 1986, vol. 26, 141
- **MAG et al.** *Nucleic Acids Res.,* 1991, vol. 19, 1437
- **BRIU et al.** *J. Am. Chem. Soc.,* 1989, vol. 111, 2321
- **ECKSTEIN.** Oligonucleotides and Analogues: A Practical Approach. Oxford University Press
- **EGHOLM.** *J. Am. Chem. Soc.,* 1992, vol. 114, 1895
- **MEIER et al.** *Chem. Int. Ed. Engl.,* 1992, vol. 31, 1008
- **NIELSEN.** *Nature,* 1993, vol. 365, 566
- **CARLSSON et al.** *Nature,* 1996, vol. 380, 207
- **DENPCY et al.** *Proc. Natl. Acad. Sci. USA,* 1995, vol. 92, 6097
- **KIEDROWSHI et al.** *Angew. Chem. Intl. Ed. English,* 1991, vol. 30, 423

- **LETSINGER et al.** *Nucleosides & Nucleotides,* 1994, vol. 13, 1597
- Carbohydrate Modifications in Antisense Research. **S. SANGHUI ; P. DAN COOK.** ASC Symposium Series 580
- **MESMAEKER et al.** *Bioorganic & Medicinal Chem. Lett.,* 1994, vol. 4, 395
- **JEFFS et al.** *J. Biomolecular NMR,* 1994, vol. 34, 17
- *Tetrahedron Lett.,* 1996, vol. 37, 743
- Carbohydrate Modifications in Antisense Research. ASC Symposium Series 580
- **JENKINS et al.** *Chem. Soc. Rev.,* 1995, 169-176
- **LOAKES et al.** *Nucleic Acid Res,* 1994, vol. 22, 4039
- **VAN AERSCHOT et al.** *Nucleic Acid Res.,* 1995, vol. 23, 4363
- **NICHOLS et al.** *Nature,* 1994, vol. 369, 492
- **BERGSTROM et al.** *Nucleic Acid Res,* 1997, vol. 25, 1935
- **LOAKES et al.** *Nucleic Acid Res.,* 1995, vol. 23, 2361
- **LOAKES et al.** *J. Mol. Biol,* 1997, vol. 270, 426
- **FOTIN et al.** *Nucleic Acid Res.,* 1998, vol. 26, 1515
- **SCHEIT.** Nucleotide Analogs. John Wiley, 1980
- **ENGLISCH.** *Angew. Chem. Int. Ed. Engl.,* 1991, vol. 30, 613-29
- **AGARWAL.** Protocols for Polynucleotides and Analogs. Humana Press, 1994
- **S. VERMA ; F. ECKSTEIN.** *Ann. Rev. Biochem.,* 1998, vol. 67, 99-134
- *The Glen Report,* 2003, vol. 16 (2), 5
- **KOSHKIN et al.** *Tetrahedron,* 1998, vol. 54, 3607-30
- **STERCHAK, E. P. et al.** *Organic Chem.,* 1987, vol. 52, 4202
- **NIELSEN et al.** *Science,* 1991, vol. 254, 1497-1500
- **EGHOLM et al.** *J. Am. Chem. Soc.,* 1992, vol. 114, 1895-1897
- **DEMIDOV et al.** *Proc. Natl. Acad. Sci.,* 2002, vol. 99, 5953-58
- Peptide Nucleic Acids: Protocols and Applications. Horizon Bioscience, 2004
- **GORYSHIN ; REZNIKOFF.** *J. Biol. Chem.,* 1998, vol. 273, 7367
- **MIZUUCHI, K.** *Cell,* 1983, vol. 35, 785
- **SAVILAHTI, H et al.** *EMBO J.,* 1995, vol. 14, 4893
- **COLEGIO et al.** *J. Bacteriol.,* 2001, vol. 183, 2384-8
- **KIRBY C et al.** *Mol. Microbiol.,* 2002, vol. 43, 173-86
- **DEVINE ; BOEKE.** *Nucleic Acids Res.,* 1994, vol. 22, 3765-72
- **CRAIG, N L.** *Science,* 1996, vol. 271, 1512
- **CRAIG, N L.** *Curr Top Microbiol Immunol.,* 1996, vol. 204, 27-48
- **KLECKNER N et al.** *Curr Top Microbiol Immunol.,* 1996, vol. 204, 49-82
- **LAMPE D J et al.** *EMBO J.,* 1996, vol. 15, 5470-9
- **PLASTERK R H.** *Curr. Topics Microbiol. Immunol.,* 1996, vol. 204, 125-43
- **GLOOR, G B.** *Methods Mol. Biol.,* 2004, vol. 260, 97-114
- **ICHIKAWA ; OHTSUBO.** *J Biol. Chem.,* 1990, vol. 265, 18829-32
- **OHTSUBO ; SEKINE.** *Curr. Top. Microbiol. Immunol.,* 1996, vol. 204, 1-26
- **BROWN et al.** *Proc Natl Acad Sci USA,* 1989, vol. 86, 2525-9
- **BOEKE ; CORCES.** *Annu Rev Microbiol.,* 1989, vol. 43, 403-34
- **ZHANG et al.** *PLoS Genet.,* 2009, vol. 5, e1000689
- **WILSON C. et al.** *J. Microbiol. Methods,* 2007, vol. 71, 332-5
- **CHEN et al.** *Biotechniques,* 2002, vol. 32, 518-520
- **KOMIYAMA et al.** *Chem. Commun.,* 1999, 1443-1451
- **RICHARD P. HAUGLAND.** Molecular Probes Handbook
- **RONAGHI et al.** Real-time DNA sequencing using detection of pyrophosphate release. *Analytical Biochemistry,* 1996, vol. 242 (1), 84-9
- **RONAGHI, M.** Pyrosequencing sheds light on DNA sequencing. *Genome Res.,* 2001, vol. 11 (1), 3-11
- **RONAGHI et al.** A sequencing method based on real-time pyrophosphate. *Science,* 1998, vol. 281 (5375), 363
- **DEAMER, D.W. ; AKESON, M.** Nanopores and nucleic acids: prospects for ultrarapid sequencing. *Trends Biotechnol.,* 2000, vol. 18, 147-151
- **DEAMER, D. ; D. BRANTON.** Characterization of nucleic acids by nanopore analysis. *Acc. Chem. Res.,* 2002, vol. 35, 817-825
- **LI et al.** DNA molecules and configurations in a solid-state nanopore microscope. *Nat. Mater.,* 2003, vol. 2, 611-615
- **SONI ; MELLER.** A. Progress toward ultrafast DNA sequencing using solid-state nanopores. *Clin. Chem.,* 2007, vol. 53, 1996-2001
- **HEALY, K.** Nanopore-based single-molecule DNA analysis. *Nanomed.,* 2007, vol. 2, 459-481
- **COCKROFT et al.** A single-molecule nanopore device detects DNA polymerase activity with single-nucleotide resolution. *J. Am. Chem. Soc.,* 2008, vol. 130, 818-820

- **LEVENE, M.J. et al.** Zero-mode waveguides for single-molecule analysis at high concentrations. *Science,* 2003, vol. 299, 682-686
- **LUNDQUIST, P.M. et al.** Parallel confocal detection of single molecules in real time. *Opt. Lett.,* 2008, vol. 33, 1026-1028
- **KORLACH, J. et al.** Selective aluminum passivation for targeted immobilization of single DNA polymerase molecules in zero-mode waveguide nanostructures. *Proc. Natl. Acad. Sci. USA,* 2008, vol. 105, 1176-1181
- **KORLACH J. et al.** Long, processive enzymatic DNA synthesis using 100% dye-labeled terminal phosphate-linked nucleotides. *Nucleosides, Nucleotides and Nucleic Acids,* 2008, vol. 27, 1072-1083
- **HARRIS T.D. et al.** Single Molecule DNA Sequencing of a viral Genome. *Science,* 2008, vol. 320, 106-109
- **JOOS et al.** *Analytical Biochemistry,* 1997, vol. 247, 96-101
- **OROSKAR et al.** *Clin. Chem.,* 1996, vol. 42, 1547-1555
- **KHANDJIAN.** *Mol. Bio. Rep.,* 1986, vol. 11, 107-11
- **TAYLOR et al.** *J. Phys. D: Appl. Phys.,* 1991, vol. 24, 1443
- **SMITH et al.** *Science,* 1992, vol. 253, 1122